(19) Europäisches Patentamt
European Patent Office
Office européen des brevets

(11) EP 1 470 113 B1

(12) EUROPEAN PATENT SPECIFICATION

(45) Date of publication and mention of the grant of the patent:
**07.01.2009 Bulletin 2009/02**

(21) Application number: **03700366.2**

(22) Date of filing: **15.01.2003**

(51) Int Cl.:
*C07D 239/54* (2006.01) *C07D 409/04* (2006.01)
*C07D 417/04* (2006.01) *A61K 31/505* (2006.01)
*A61P 31/12* (2006.01)

(86) International application number:
**PCT/GB2003/000124**

(87) International publication number:
**WO 2003/062211 (31.07.2003 Gazette 2003/31)**

(54) **PYRIMIDINONE VIRAL POLYMERASE INHIBITORS**

PYRIMIDINONEN ALS VIRALE POLYMERASE INHIBITOREN

INHIBITEURS PYRIMIDONE DE POLYMERASES VIRALES

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **18.01.2002 GB 0201179**

(43) Date of publication of application:
**27.10.2004 Bulletin 2004/44**

(73) Proprietor: **ISTITUTO DI RICERCHE DI BIOLOGIA MOLECOLARE P. ANGELETTI S.P.A.**
**00040 Pomezia (IT)**

(72) Inventors:
- **AVOLIO, Salvatore**
**IRBM**
**I-00040 Pomezia (IT)**
- **COLARUSSO, Stefania**
**IRBM**
**I-00040 Pomezia (IT)**
- **CONTE, Immacolata**
**IRBM**
**I-00040 Pomezia (IT)**
- **HARPER, Steven**
**IRBM**
**I-00040 Pomezia (IT)**
- **KOCH, Uwe**
**IRBM**
**I-00040 Pomezia (IT)**
- **MALANCONA, Savina**
**IRBM**
**I-00040 Pomezia (IT)**
- **MATASSA, Victor, Giulio**
**IRBM**
**I-00040 Pomezia (IT)**
- **NARJES, Frank**
**IRBM**
**I-00040 Pomezia (IT)**
- **PETROCCHI, Alessia**
**IRBM**
**I-00040 Pomezia (IT)**
- **SUMMA, Vincenzo**
**IRBM**
**I-00040 Pomezia (IT)**

(74) Representative: **Horgan, James Michael Frederic et al**
**Merck & Co., Inc.**
**European Patent Department**
**Merck Sharp & Dohme Limited,**
**Hertford Road,**
**Hoddesdon**
**Hertfordshire, EN11 9BU (GB)**

(56) References cited:
**WO-A-00/13708** **WO-A-02/06246**

- **CULBERSTON: "SYNTHESIS OF 5,6-DIHYDROXY-2-PHENYL-4-PYRIMIDINECARBOXY LIC ACID,METHYL ESTER" JOURNAL OF HETEROCYCLIC CHEMISTRY., vol. 16, no. 7, November 1979 (1979-11), pages 1423-4, XP001029094 HETEROCORPORATION. PROVO., US ISSN: 0022-152X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 470 113 B1

## Description

This invention relates to compounds which can act as inhibitors of viral polymerases, especially the hepatitis C virus (HCV) polymerase, to uses of such compounds and to their preparation.

The hepatitis C virus (HCV) is the major causative agent of parenterally-transmitted and sporadic non-A, non-B hepatitis (NANB-H). Some 1% of the human population of the planet is believed to be affected. Infection by the virus can result in chronic hepatitis and cirrhosis of the liver, and may lead to hepatocellular carcinoma. Currently no vaccine nor established therapy exists, although partial success has been achieved in a minority of cases by treatment with recombinant interferon-$\alpha$ either alone or in combination with ribavirin. There is therefore a pressing need for new and broadly-effective therapeutics.

Several virally-encoded enzymes are putative targets for therapeutic intervention, including a metalloprotease (NS2-3), a serine protease (NS3), a helicase (NS3), and an RNA-dependent RNA polymerase (NS5B). Of these, the polymerase plays an essential role in replication of the virus and is therefore an important target in the fight against hepatitis C.

In J. Heterocyclic Chemistry, vol. 16, no. 7, November 1979 (1979-11), pages 1423-4, HeteroCorporation, Culberston describes the synthesis of 5,6-dihydroxy-2-phenyl-4-pyrimidinecarboxylic acid, methyl ester, no biological activity of this compound is reported.

PCT application WO U0/13708 (Viropharma Inc.) provides a method for treating viral infections and diseases by administering to a host a therapeutic amount of a substituted pyrimidine-4, 6-dione.

PCT application WO 02/06246 provides 2-aryl-4, 5-dihydroxy-6-Carboxypyrimidines as inhibitors of hepatitis C virus (HCV) polymerase enzyme.

It has now been found that certain pyrimidinone derivatives act as inhibitors of hepatitis C virus (HCV) polymerase enzyme.

The present invention provides a compound of formula (I) below, or a pharmaceutically acceptable salt thereof:

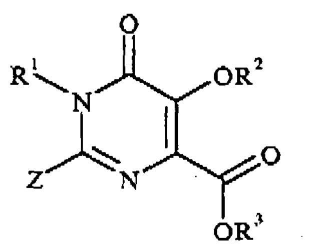

(I)

wherein

Z represents $C_{2-6}$ alkynyl, aryl or heteroaryl, any of which groups may be optionally substituted;
$R^1$ represents $C_{1-6}$ alkyl or aryl($C_{1-6}$)alkyl, either of which groups may be optionally substituted;
$R^2$ represents hydrogen; or $C_{1-6}$ alkyl, $C_{2-6}$ alkylcarbonyl, aryl, arylcarbonyl, heteroaryl, aryl($C_{1-6}$)alkyl or heteroaryl ($C_{1-6}$)alkyl, any of which groups may be optionally substituted; and
$R^3$ represents hydrogen, $C_{1-6}$ alkyl, $C_{3-7}$ heterocyloalkyl($C_{1-6}$)alkyl, di($C_{1-6}$)alkylamino($C_{1-6}$)alkyl, $C_{2-6}$ alkylcarbonyloxy($C_{1-6}$)alkyl or $C_{3-7}$ cycloalkoxycarbonyloxy($C_{1-6}$)alkyl;

for use in therapy, especially for pharmaceutical use in humans.

An inconclusive synthetic route, which may have given rise to the compound of formula (I) as depicted above, wherein Z is unsubstituted phenyl and $R^1$, $R^2$ and $R^3$ are all methyl, is described in *J. Heterocycl. Chem.,* 1979, 16, 1423. No pharmacological activity is, however, ascribed therein to that specific compound.

The present invention also provides a compound of formula (I) as defined above, or a pharmaceutically acceptable salt thereof; provided that, when Z is unsubstituted phenyl, then $R^1$, $R^2$ and $R^3$ do not each simultaneously represent methyl.

Typical examples of $C_{1-6}$ alkyl groups include methyl and ethyl groups, and straight-chained or branched propyl, butyl, pentyl and hexyl groups. Particular alkyl groups are methyl, ethyl, *n*-propyl, isopropyl, *tert*-butyl and 1,1-dimethylpropyl. Derived expressions such as "$C_{1-6}$ alkoxy" are to be construed accordingly.

Typical examples of $C_{2-6}$ alkenyl groups include vinyl, allyl and dimethylallyl groups.

Typical examples of $C_{2-6}$ alkynyl groups include ethynyl and propargyl groups.

Typical $C_{3-7}$ cycloalkyl groups include cyclopropyl, cyclobutyl, cyclopentyl and cyclohexyl.

Suitable $C_{3-7}$ heterocycloalkyl groups include azetidinyl, pyrrolidinyl, piperidinyl, piperazinyl, morpholinyl and thiomorpholinyl groups.

Suitable aryl groups include phenyl and naphthyl, especially phenyl.

Suitable heteroaryl groups include pyridinyl, quinolinyl, isoquinolinyl, pyridazinyl, pyrimidinyl, pyrazinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl and tetrazolyl groups.

Typical aryl($C_{1-6}$)alkyl groups include benzyl, phenylethyl, phenylpropyl, phenylbutyl and naphthylmethyl.

Typical heteroaryl($C_{1-6}$)alkyl groups include furylmethyl, furylethyl, thienylmethyl, thienylethyl, oxazolylmethyl, oxazolylethyl, thiazolylmethyl, thiazolylethyl, imidazolylmethyl, imidazolylethyl, oxadiazolylmethyl, oxadiazolylethyl, thiadiazolylmethyl, thiadiazolylethyl, triazolylmethyl, triazolylethyl, tetrazolylmethyl, tetrazolylethyl, pyridinylmethyl, pyridinylethyl, pyrimidinylmethyl, pyrazinylmethyl, quinolinylmethyl and isoquinolinylmethyl.

Where a compound or group is described as "optionally substituted" one or more substituents may be present. Optional substituents are not particularly limited and may, for instance, be selected from $C_{1-6}$ alkyl, $C_{2-6}$ alkenyl, $C_{3-7}$ cycloalkyl, $C_{3-7}$ heterocycloalkyl, aryl, aryl($C_{1-6}$)alkyl, heteroaryl, heteroaryl($C_{1-6}$)alkyl, $C_{1-6}$ alkoxy, aryloxy, aryl($C_{1-6}$) alkoxy, heteroaryloxy, heteroaryl($C_{1-6}$)alkoxy, amino, nitro, halo, hydroxy, carboxy, formyl, cyano and trihalomethyl groups. Furthermore, optional substituents may be attached to the compounds or groups which they substitute in a variety of ways, either directly or through a connecting group of which the following are examples: amine, amide, ester, ether, thioether, sulphonamide, sulphamide, sulphoxide, urea, thiourea and urethane. As appropriate an optional substituent may itself be substituted by another substituent, the latter being connected directly to the former or through a connecting group such as those exemplified above.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

Where the moiety Z in the compounds of formula (I) above represents optionally substituted $C_{2-6}$ alkynyl, this is suitably an optionally substituted ethynyl group. A typical substituent on the $C_{2-6}$ alkynyl group is tri($C_{1-6}$)alkylsilyl, especially trimethylsilyl. In this context, a typical value for the moiety Z is trimethylsilylethynyl.

Where Z represents an optionally substituted aryl or heteroaryl moiety, it may suitably be selected from phenyl, thienyl, oxazolyl, thiazolyl, furyl, isoquinolinyl, indolyl, isoxazolyl, pyrazolopyrimidinyl and pyrazinyl, any of which groups may be optionally substituted. Particular values of Z include phenyl, thienyl, thiazolyl and furyl, any of which groups may be optionally substituted. These groups may be joined to the 2-position of the pyrimidinone nucleus at any available position of the aryl or heteroaryl ring. However, connection at certain positions may be preferred and this is considered in some more detail below.

Preferred optional substituents on the aryl or heteroaryl group Z may be selected from a wide variety of groups. For instance, they may be simple, relatively small groups such as halogen (especially fluorine, chlorine and bromine), hydroxy, $-NO_2$, $-NH_2$, formyl, $C_{2-6}$ alkylcarbonyl, $-CO_2H$ $C_{2-6}$ alkoxycarbonyl, $C_{1-6}$ alkyl (especially methyl), $C_{2-6}$ alkenyl, $C_{2-6}$ alkynyl, -CN, $C_{1-6}$alkoxy (especially methoxy), $C_{1-6}$alkylthio (especially methylthio), $C_{1-6}$ alkylsulfinyl (especially methylsulfinyl) or $C_{1-6}$ alkylsulfonyl (especially methylsulfonyl). As appropriate any of these substituents may be substituted by one or more of the others. However, in general at least one substituent is a group of formula (II):

$$-X-R^4 \qquad (II)$$

where $R^4$ is a generally hydrophobic moiety containing one or more, but generally at least 3, preferably 4 to 20, particularly 4 to 14, carbon atoms. Preferably, $R^4$ includes one or more of the following groups, any of which may, optionally, be substituted: aryl, aryl($C_{1-6}$)alkyl, $C_{3-7}$ cycloalkyl, $C_{1-6}$ alkyl (especially branched $C_{1-6}$ alkyl), heteroaryl($C_{1-6}$)alkyl, $C_{3-7}$ heterocycloalkyl and $C_{2-6}$ alkenyl. The group X is preferably selected from $-NH-SO_2$ $-NH-SO_2-NH-$ $-CH_2-SO_2-$, $-SO_2-NH-$ -NH-CO-NH-, -NH-CS-NH-, -NH-CO-O-, -NH-CO-, -CO-NH-, $-NH-CO-NH-SO_2-$, -NH-CO-NH-CO-, -O-, -S-, -SO-, $-SO_2-$ -NH-, $-CH_2-$, $-CH_2O-$ and $-CH_2S-$.

The hydrogen atom of any NH group may, optionally, be replaced by a $C_{1-6}$ alkyl group.

Where $R^1$ represents optionally substituted aryl($C_{1-6}$)alkyl, this may be benzyl or phenylethyl, either of which groups may be optionally substituted. A particular substituent in this regard is halogen, especially chloro.

Specific values of $R^1$ include methyl, ethyl, trifluoroethyl (particularly 2,2,2-trifluoroethyl), benzyl, carboxybenzyl and chlorophenylethyl, especially methyl.

Typical values of $R^2$ include hydrogen; and $C_{1-6}$ alkyl, $C_{2-6}$ alkylcarbonyl, arylcarbonyl or aryl($C_{1-6}$)alkyl, any of which groups may be optionally substituted. Particular values of $R^2$ include hydrogen; and $C_{1-6}$ alkyl, $C_{2-6}$ alkylcarbonyl or arylcarbonyl, any of which groups may be optionally substituted. Examples of suitable substituents on $R^2$ include halogen and $C_{1-6}$ alkoxy, especially fluoro or methoxy.

Particular values of $R^2$ include hydrogen, methyl, ethyl, tert-butyl, acetyl, pivaloyl, benzoyl, benzyl, difluorobenzyl

and methoxybenzyl.

Specific values of $R^2$ include hydrogen, methyl, ethyl, tert-butyl, acetyl, pivaloyl or benzyl.

In one embodiment, $R^2$ represents hydrogen.

Particular values of $R^3$ include hydrogen, methyl, ethyl, morpholinylethyl, dimethylaminoethyl, acetoxymethyl, pivaloyloxymethyl and 1-(cyclohexyloxycarbonyloxy)ethyl.

Specific values of $R^3$ include hydrogen, methyl and ethyl.

In one embodiment, $R^3$ represents hydrogen.

One illustrative sub-class of compounds in accordance with the invention is represented by formula (III) below:

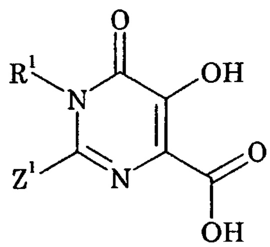

(III)

wherein

$Z^1$ represents optionally substituted aryl; and
$R^1$ is as defined above.

For instance, examples of compounds within this class are those of formula (IV):

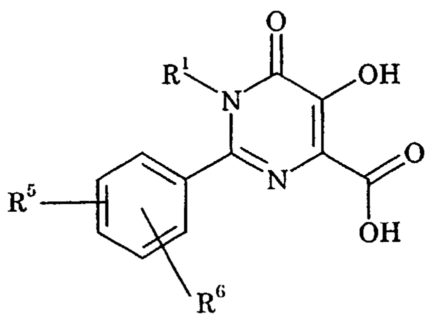

(IV)

wherein

$R^1$ is as defined above; and
each of $R^5$ and $R^6$ may independently be selected from H or a substituent group.

Preferably, one of $R^5$ and $R^6$ is hydrogen, while the other is a substituent. Where a substituent is present it may be at any of the 2-, 3-or 4-positions - i.e. *ortho*, *meta* or *para* to the pyrimidinone nucleus. However, where a single substituent is present, substitution at the *ortho* or *meta* positions is preferred.

The substituents $R^5$ and $R^6$ may be selected from a wide variety of groups. For instance, they may be simple, relatively small groups such as halogen (especially fluorine, chlorine and bromine), hydroxy, $-NO_2$ $-NH_2$, formyl, $C_{2-6}$ alkylcarbonyl, $-CO_2H$, $C_{2-6}$ alkoxycarbonyl, $C_{1-6}$ alkyl (especially methyl), $C_{2-6}$ alkenyl, $C_{2-6}$ alkynyl, -CN, $C_{1-6}$ alkoxy (especially methoxy), $C_{1-6}$ alkylthio (especially methylthio), $C_{1-6}$ alkylsulfinyl (especially methylsulfinyl) or $C_{1-6}$ alkylsulfonyl (especially methylsulfonyl). As appropriate any of these substituents may be substituted by one or more of the others.

Although some such compounds are of high activity, it is generally preferable that substituent $R^5$ and/or $R^6$ include a relatively hydrophobic group $R^4$ which is bonded to the phenyl group through a linkage X. In this case the substituents $R^5$ and/or $R^6$ may be represented by the formula (II):

$$-X-R^4 \quad \text{(II)}$$

where $R^4$ and X are as defined above.

For instance, examples of preferred classes of compound are those in which a single *ortho* or *metal* substituent is present, and that substituent is selected from the following formulae (V), (VI), (VII), (VIII) and (IX):

$$-X-(CH_2)_n-R^7 \qquad (V)$$

$$-X-CH=CH-R^7 \qquad (VI)$$

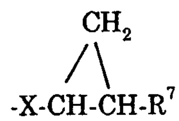

(VII)

$$-X-(CHR^8)_p-(CH_2)_m-(CHR^8)_q-R^7 \qquad (VIII)$$

$$-X-(CH_2)_r-Y-R^7 \qquad (IX)$$

wherein

n is zero or an integer from 1 to 6, and preferably is from zero to 3, especially 0 or 1;
m is zero or an integer from 1 to 6, but preferably is 0 or 1;
each of p and q is independently 0 or 1, but preferably they are not simultaneously 1;
r is an integer from 1 to 6, preferably 1;
$R^7$ is an optionally substituted aryl, heteroaryl, $C_{3-7}$ cycloalkyl, $C_{3-7}$ heterocycloalkyl or branched $C_{1-6}$ alkyl group;
each $R^8$ is independently a $C_{1-6}$ alkyl group (especially methyl), a $C_{3-7}$ cycloalkyl group, an optionally substituted aryl group (especially phenyl), hydroxy or hydroxy($C_{1-6}$)alkyl (especially hydroxymethyl), any of which may be optionally etherified, or $-NH_2$, optionally protonated, alkylated or derivatised as a urethane group; and
Y is selected from -O-, -S- and -NH-.

In each of the formulae (V) to (IX) the linkage X may be any of the X groups specified above.

Among the groups X, the sulfonamide ($-NH-SO_2-$) urea (-NH-CO-NH-), urethane (-NH-CO-O-) and amide (-NH-CO-) groups are favoured. A particular value of X is $-NH-CO-NH-SO_2-$.

The group $R^7$ is preferably an aryl or heteroaryl group, of which optionally substituted phenyl, naphthyl, thienyl, benzothienyl, pyridyl, quinolyl and thiazolyl are particularly preferred examples. Each of these may, optionally, be substituted by another optionally substituted aryl or heteroaryl group of the same or different type.

Typical compounds of formula (II) are specifically exemplified herein as Examples 24-26, 36 and 37. All those compounds have $IC_{50}$ values below 100 $\mu$M when measured in the assay described below.

Another illustrative sub-class of compounds in accordance with the invention is represented by formula (X) below:

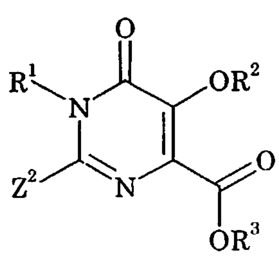

(X)

wherein

$Z^2$ represents optionally substituted heteroaryl; and

$R^1$, $R^2$ and $R^3$ are as defined above.

Particular values of $Z^2$ include thienyl, thiazolyl and furyl, especially thienyl, any of which groups may be optionally substituted.

Preferred compounds in this sub-class are those in which the heteroaryl group $Z^2$ is unsubstituted, or carries a single substituent $R^9$, as defined *infra.*

A favoured subset of the compounds of formula (X) is represented by formula (XI) below:

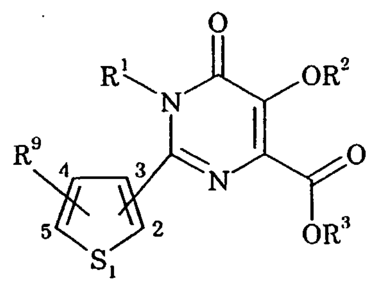

(XI)

wherein

$R^1$, $R^2$ and $R^3$ are as defined above; and
$R^9$ is as defined *infra*.

The pyrimidinone nucleus and the $R^9$ substituent may be at any position on the thiophene ring. However, it is preferred that when the pyrimidinone is at position 2 on the thiophene ring, then substituent $R^9$ is at the 3-position, substitution at the 4- or 5-positions being less preferred. When the pyrimidinone group is at the 3-position of the thiophene ring, then $R^9$ is preferably at the 2- or 4-position of the thiophene ring, more preferably at the 4-position. In summary, favoured compounds in accordance with the present invention are represented by formula (XII) and (XIII) below:

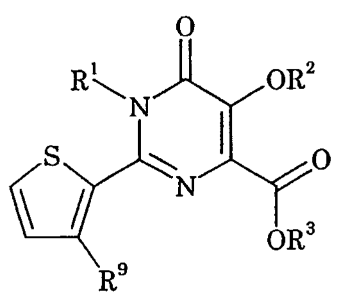

(XII)

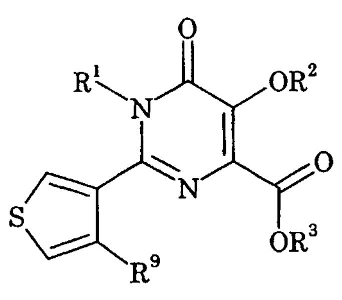

(XIII)

wherein

$R^1$, $R^2$ and $R^3$ are as defined above; and
$R^9$ is as defined *infra.*

Substituent $R^9$ may be selected from a wide variety of groups. For instance, like substituents $R^5$ and $R^6$ discussed above it may be a simple, relatively small group such as halogen (especially fluorine, chlorine and bromine), hydroxy, $-NO_2$ $-NH_2$, formyl, $C_{2-6}$ alkylcarbonyl, $-CO_2H$ $C_{2-6}$ alkoxycarbonyl, $C_{1-6}$ alkyl (especially methyl), $C_{1-6}$ alkenyl, $C_{2-6}$ alkynyl, -CN, $C_{1-6}$ alkoxy (especially methoxy), $C_{1-6}$ alkylthio (especially methylthio), $C_{1-6}$ alkylsulfinyl (especially methylsulfinyl) or $C_{1-6}$ alkylsulfonyl (especially methylsulfonyl). As appropriate any of these substituents may be substituted by one or more of the others.

More preferably, however, $R^9$ includes a relatively hydrophobic group which is bonded to the thienyl group through a linkage X. In this case, the group $R^9$ may be represented by the formula (II):

$$-X-R^4 \qquad (II)$$

where X and $R^4$ are as defined above.

Preferred X groups are amide, sulphonamide, urea and urethane linkages. A particularly preferred X group is $-NH-CO-NH-SO_2-$. Preferred $R^4$ groups are those shown in formulae (V) to (IX) already discussed above, and which include a group $R^7$. Advantageously, $R^4$ is naphthyl.

Preferred $R^7$ groups are aromatic groups, especially phenyl, naphthyl, thienyl, pyridyl, benzothienyl, indolyl, benzimidazolyl and oxazolyl groups. When $R^7$ comprises fused aromatic rings, the connection to the remainder of the $R^4$ group may be through any ring.

Preferred optional substituents on $R^7$, especially in the case where $R^7$ is an aryl group, include halogen (e.g. fluorine, chlorine and/or bromine), nitro ($-NO_2$), $C_{1-6}$ alkyl (especially methyl), $C_{1-6}$ alkoxy (especially methoxy), trifluoromethyl and aryl (especially phenyl).

Suitably, n is zero.

Suitably, $R^7$ is naphthyl.

Typical compounds of formula (X) are specifically exemplified herein as Examples 1-23, 27-35, 38-40 and 42-49. Those compounds all have $IC_{50}$ values of less than 100 $\mu$M as measured in the assay described *infra.* In fact, most have an $IC_{50}$ of less than 25 $\mu$M, frequently less than 10 $\mu$M. Certain of the compounds have submicromolar $IC_{50}$ values.

In another aspect, the invention provides the use of a compound of formula (I) as defined above, or a pharmaceutically acceptable salt thereof, for the manufacture of a medicament for treatment or prevention of infection by hepatitis C virus in a human or animal.

A further aspect of the invention provides a pharmaceutical composition comprising a compound of formula (I) as defined above, or a pharmaceutically acceptable salt thereof, in association with a pharmaceutically acceptable carrier. The composition may be in any suitable form, depending on the intended method of administration. It may for example be in the form of a tablet, capsule or liquid for oral administration, or of a solution or suspension for administration parenterally.

The pharmaceutical compositions optionally also include one or more other agents for the treatment of viral infections such as an antiviral agent, or an immunomodulatory agent such as $\alpha$-, $\beta$- or $\gamma$-interferon.

The compounds of the invention can be used in a a method of inhibiting hepatitis C virus polymerase and/or of treating or preventing an illness due to hepatitis C virus, the method involving administering to a human or animal (preferably mammalian) subject suffering from the condition a therapeutically or prophylactically effective amount of the pharmaceutical composition described above or of a compound of formula (I) as defined above, or a pharmaceutically acceptable salt thereof. "Effective amount" means an amount sufficient to cause a benefit to the subject or at least to cause a change in the subject's condition.

The dosage rate at which the compound is administered will depend on a variety of factors including the activity of the specific compound employed, the metabolic stability and length of action of that compound, the age of the patient, body weight, general health, sex, diet, mode and time of administration, rate of excretion, drug combination, the severity of the particular condition and the host undergoing therapy. Suitable dosage levels may be of the order of 0.02 to 5 or 10 g per day, with oral dosages two to five times higher. For instance, administration of from 10 to 50 mg of the compound per kg of body weight from one to three times per day may be in order. Appropriate values are selectable by routine testing. The compound may be administered alone or in combination with other treatments, either simultaneously or sequentially. For instance, it may be administered in combination with effective amounts of antiviral agents, immunomodulators, anti-infectives or vaccines known to those of ordinary skill in the art. It may be administered by any suitable route, including orally, intravenously, cutaneously and subcutaneously. It may be administered directly to a suitable site or in a manner in which it targets a particular site, such as a certain type of cell. Suitable targeting methods are already known.

An additional aspect of the invention provides a method of preparation of a pharmaceutical composition, involving admixing at least one compound of formula (I) as defined above, or a pharmaceutically acceptable salt thereof, with one or more pharmaceutically acceptable adjuvants, diluents or carriers and/or with one or more other therapeutically

or prophylactically active agents.

The compounds according to the present invention may be prepared by a process which comprises reacting a compound of formula (XIV) with a compound of formula (XV):

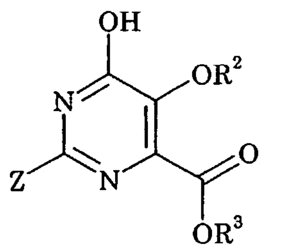

$R^1—L^1$

(XIV) (XV)

wherein Z, $R^1$, $R^2$ and $R^3$ are as defined above, and $L^1$ represents a suitable leaving group.

The leaving group $L^1$ is typically a halogen atom, e.g. iodo; or a sulphate moiety, e.g. methoxysulphonyloxy. The reaction is conveniently effected in the presence of a base, e.g. caesium carbonate or lithium hydride; in a suitable solvent such as tetrahydrofuran or 1,4-dioxane.

The intermediates of formula (XIV) wherein $R^2$ is hydrogen may be prepared by cyclization of amidoximes of formula (XVI):

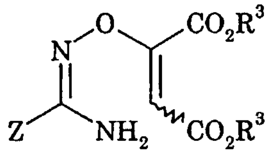

(XVI)

wherein Z and $R^3$ are as defined above; typically by heating at reflux in xylene.

Amidoximes of formula (XVI) may be prepared by reacting a compound of formula (XVII) with a compound of formula (XVIII):

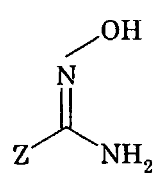

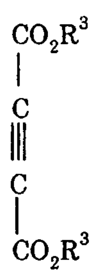

(XVII) (XVIII)

wherein Z and $R^3$ are as defined above; optionally in the presence of an organic base such as triethylamine; in a suitable solvent, which will typically be a chlorinated solvent such as chloroform or dichloromethane.

Amidoximes of formula (XVII) may be prepared by reacting hydroxylamine with a nitrile of formula Z-CN; typically in the presence of a base such as sodium carbonate, potassium carbonate or triethylamine.

In another procedure, the compounds according to the invention may be prepared by a process which comprises reacting a compound of formula (XVIII) as defined above with a compound of formula (XIX):

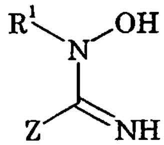

(XIX)

wherein Z and $R^1$ are as defined above; followed by cyclisation of the intermediate thereby obtained.

The reaction between compounds (XVIII) and (XIX) is conveniently accomplished in a suitable solvent, which will typically be a chlorinated solvent such as chloroform or dichloromethane. Cyclisation of the ensuing intermediate is suitably effected by heating in xylene.

The intermediates of formula (XIX) may be prepared by reacting a compound of formula $R^1$-NHOH with a nitrile of formula Z-CN; typically in the presence of a base such as sodium carbonate.

Nitriles of formula Z-CN may be obtained from commercial sources or may be prepared from the corresponding primary amides of formula Z-$CONH_2$ using established methods known to those skilled in the art.

Primary amides of formula Z-$CONH_2$ may be obtained from commercial sources or may be prepared from the corresponding esters or carboxylic acids using established methods known to those skilled in the art.

In a further procedure, the compounds according to the invention may be prepared by a process which comprises reacting a compound of formula (XVIII) as defined above with a compound of formula (XX):

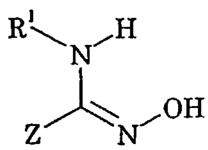

(XX)

wherein Z and $R^1$ are as defined above; followed by cyclisation of the intermediate thereby obtained.

The reaction between compounds (XVIII) and (XX) is conveniently accomplished in a solvent such as chloroform. Cyclisation of the ensuing intermediate is suitably effected by heating in xylene.

The intermediates of formula (XX) may be prepared by reacting a compound of formula $R^1$-$NH_2$ with a compound of formula (XXI):

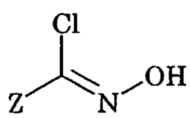

(XXI)

wherein Z and $R^1$ are as defined above.

The intermediates of formula (XXI) may be prepared by reacting hydroxylamine with a compound of formula Z-CHO; followed by treating the oxime thereby obtained with *N*-chlorosuccinimide.

In an additional procedure, the compounds according to the invention may be prepared by reacting a compound of formula Z-$B(OH)_2$ with a compound of formula (XXII):

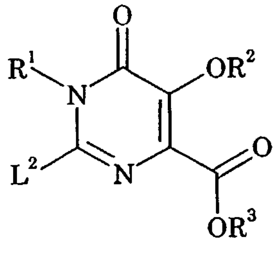

(XXII)

wherein Z, $R^1$, $R^2$ and $R^3$ are as defined above, and $L^2$ represents a suitable leaving group; in the presence of a transition

metal catalyst.

The leaving group $L^2$ is typically a halogen atom, e.g. chloro.

The transition metal catalyst of use in the foregoing reaction is suitably bis(tri-*tert*-butylphosphine)palladium (0) or tetrakis-(triphenylphosphine)palladium(0).

Where $L^2$ in the intermediates of formula (XXII) above is chloro, such compounds may be prepared by reacting a compound of formula (XXIII):

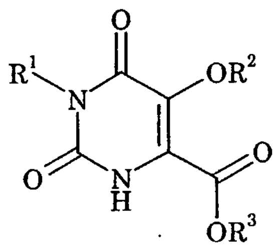

(XXIII)

wherein $R^1$, $R^2$ and $R^3$ are as defined above; with phosphorus oxychloride.

The intermediates of formula (XXIII) may be prepared as depicted in the following reaction scheme:

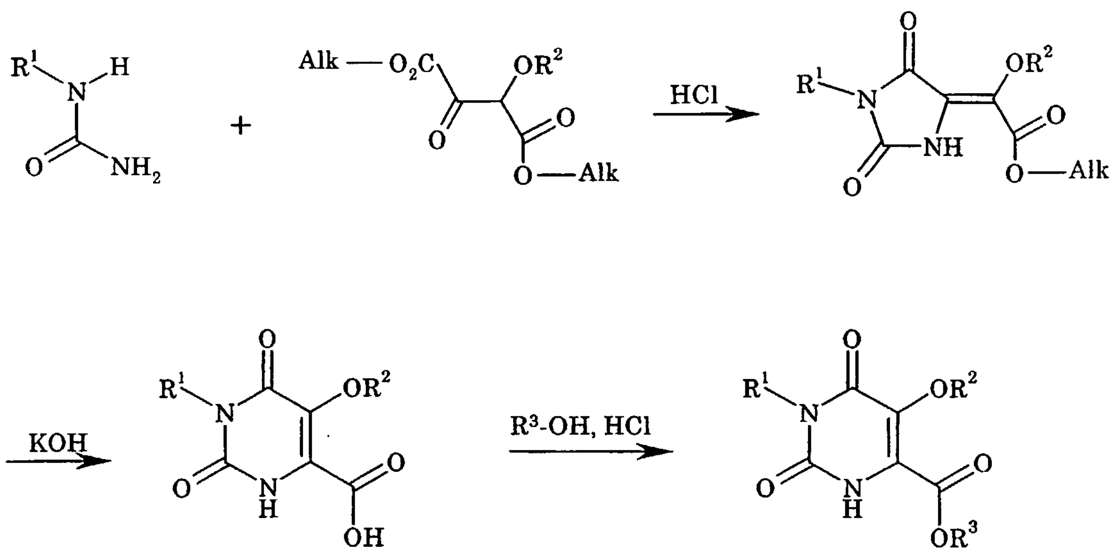


(XXIII)

wherein $R^1$, $R^2$ and $R^3$ are as defined above, and Alk represents $C_{1-6}$ alkyl, e.g. methyl or ethyl, especially ethyl.

It will be understood that any compound of formula (I) initially obtained from any of the above processes may, where appropriate, subsequently be elaborated into a further compound of formula (I) by techniques known from the art. For example, a compound of formula (I) wherein the moiety Z is substituted by a simple, relatively small group as specified *supra* may be converted into the corresponding compound wherein Z is substituted by a group of formula (II) as defined above by means of procedures analogous to those described in many of the accompanying Examples. A compound of formula (I) wherein $R^2$ represents hydrogen may be converted into the corresponding compound wherein $R^2$ is other than hydrogen by means of conventional etherification or esterification procedures. A compound of formula (I) wherein $R^2$ represents $C_{1-6}$ alkyl, e.g. ethyl, may be converted into the corresponding compound wherein $R^2$ represents hydrogen by treatment with boron tribromide. Moreover, a compound of formula (I) wherein $R^2$ represents *tert*-butyl may be converted into the corresponding compound wherein $R^2$ represents hydrogen by treatment with trifluoroacetic acid. A compound of formula (I) wherein $R^2$ represents $C_{2-6}$ alkylcarbonyl or arylcarbonyl, e.g. acetyl, pivaloyl or benzoyl, may be converted into the corresponding compound wherein $R^2$ represents hydrogen by means of standard saponification techniques, e.g. by treatment with an alkaline reagent such as sodium hydroxide or lithium hydroxide. A compound of formula (I) wherein $R^3$ represents hydrogen may be converted into the corresponding compound wherein $R^3$ is other

than hydrogen by means of conventional esterification procedures, e.g. by treatment with the appropriate alcohol of formula $R^3$-OH in the presence of a mineral acid such as hydrochloric acid. A compound of formula (I) wherein $R^3$ is other than hydrogen may be converted into the corresponding compound wherein $R^3$ is hydrogen by means of standard saponification techniques, e.g. by treatment with an alkaline reagent such as sodium hydroxide or lithium hydroxide.

Where a mixture of products is obtained from any of the processes described above for the preparation of compounds according to the invention, the desired product can be separated therefrom at an appropriate stage by conventional methods such as preparative HPLC; or column chromatography utilising, for example, silica and/or alumina in conjunction with an appropriate solvent system.

Where the above-described processes for the preparation of the compounds according to the invention give rise to mixtures of stereoisomers, these isomers may be separated by conventional techniques such as preparative chromatography. The novel compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as (-)-di-*p*-toluoyl-d-tartaric acid and/or (+)-di-*p*-toluoyl-l-tartaric acid, followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 3rd edition, 1999. The protecting groups may be removed at a convenient subsequent stage using methods known from the art.

The following Examples illustrate the preparation of compounds according to the invention.

The compounds in accordance with this invention are potent inhibitors of HCV polymerase. The $IC_{50}$ values in $\mu$M of these compounds can be measured in the following way.

Test for Inhibition of Hepatitis C Virus RdRp

WO 96/37619 describes the production of recombinant HCV RdRp from insect cells infected with recombinant baculovirus encoding the enzyme. The purified enzyme was shown to possess *in vitro* RNA polymerase activity using RNA as template. The reference describes a polymerisation assay using poly(A) as a template and oligo(U) as a primer. Incorporation of tritiated UTP is quantified by measuring acid-insoluble radioactivity. The present inventors have employed this assay to screen the compounds of the accompanying Examples as inhibitors of HCV RdRp.

Incorporation of radioactive UMP was measured as follows. The standard reaction (100 $\mu$l) was carried out in a buffer containing 20 mM tris/HCl pH 7.5, 5 mM $MgCl_2$, 1 mM DTT, 50 mM NaCl, 1 mM EDTA, 2OU Rnasin (Promega), 0.05% Triton X-100, 1 $\mu$Ci [$^3$H]-UTP (40 Ci/mmol, NEN), 10 $\mu$M UTP and 10 $\mu$g/ml poly(A). Oligo$(U)_{12}$ (1 $\mu$g/ml, Genset) was added as a primer. The final NSSB enzyme concentration was 20 nM. After 1 h incubation at 22°C the reaction was stopped by adding 100 $\mu$l of 20% TCA and applying samples to DE81 filters. The filters were washed thoroughly with 5% TCA containing 1M $Na_2HPO_4/NaH_2PO_4$, pH 7.0, rinsed with water and then ethanol, air dried, and the filter-bound radioactivity was measured in the scintillation counter. By carrying out the reaction in the presence of various concentrations of each test compound it was possible to determine $IC_{50}$ values for each compound utilizing the formula:

$$\% \text{ residual activity} = 100/(1+[I]/IC_{50})^S$$

where [I] is the inhibitor concentration and "s" is the slope of the inhibition curve.

The compounds of the accompanying Examples were tested in the above assay, and all were found to possess an $IC_{50}$ value of 100 $\mu$M or less.

## EXAMPLES

The following abbreviations are used in the Examples:

Ac: acetate; anhydr.: anhydrous; app.: apparent; aqu.: aqueous; Boc: *tert*-butyloxycarbonyl; brine: saturated aqueous solution of sodium chloride; Bz: benzoyl; BOP-Cl: bis(2-oxo-3-oxazolidinyl)phosphinic chloride; bp: boiling point; Bu: butyl; DCM: dichloromethane; DIPEA: diisopropylethyl amine; 4-DMAP: *N,N*-dimethylaminopyridine; DMF: *N,N*-dimethylformamide; DMSO: dimethylsulfoxide; eq: equivalent; Et: ethyl; EtOAc: ethyl acetate; $Et_2O$ diethyl ether; h: hour(s); HATU: *O*-(7-azabenzotriazol-1-yl)-*N,N,N',N'*-tetramethyluronium hexafluorophosphate; Me: methyl; min: minutes; mp: melting point; NCS: *N*-chlorosuccinimide; PE: petrol ether (bp 40-60°C); Ph: phenyl; PMB: *para*-methoxybenzyl;

Pr: propyl; Pv: pivaloyl; PyBOP: benzotriazol-1-yloxytripyrrolidino-phosphonium hexafluorophosphate; RP-HPLC: reversed phase highpressure liquid chromatography; sat.: saturated; TFA: trifluoroacetic acid; THF: tetrahydrofuran; TLC: thin-layer chromatography.

Nuclear magnetic resonance (NMR) spectra were recorded on a Bruker AMX 300 or AMX 400 spectrometer at a temperature of 300 K unless otherwise indicated. The chemical shifts (δ) are reported in parts per million (ppm) relative to internal tetramethylsilane or the residual solvent peak. For $^1$H-NMR spectra, significant peaks are tabulated in the order number of protons, multiplicity (s, singlet; d, doublet; t, triplet; q, quartet; m, multiplet; bs, broad singlet), coupling constant(s) in Hertz. Mass spectral data were obtained on a Perkin Elmer API 100 in negative (ES-) or positive ($ES^+$) ionization mode and results are reported as the ratio of mass over charge (*m*/*z*) for the parent ion only, followed in many cases by the relative abundance of the ion.

Organic extracts were usually dried over sodium sulfate, the drying agent was removed by filtration and the solvents evaporated on a rotary evaporator under reduced pressure.

Flash chromatography was carried out on silica gel according to Still's published procedure (W.C. Still et al., J. Org. Chem., 1978, 43, 2923) or on flash chromatography systems with prepacked columns (Biotage Corporation).

Preparative RP-HPLC was carried out with a Waters Delta Prep 4000 separation module, equipped with a Waters 486 absorption detector. Compounds were eluted with gradients of water and acetonitrile both containing 0.1% TFA. If not stated otherwise, a Waters Symmetry or X-terra column (19 x 150 mm, 7 micron) was used, with flow rates between 15 and 25 ml/min.

## EXAMPLE 1

2-[3-({[(2-Chlorobenzyl)amino]carbonyl}amino)thien-2-yl]-5-hydroxy-1-methyl-6-oxo-1,6-dihydropyrimidine-4-carboxylic acid

Step 1: 3-Nitrothiophene-2-carbonitrile

A modification of the method described by Huddleston (P.R. Huddleston et al., Synthetic Communications, 1995, 25, 3729) was used, as follows: commercial methyl 3-amino-2-thiophenecarboxylate (1 eq) was suspended in water (3.8 M) and treated with concentrated hydrochloric acid (125 ml). The resulting suspension was stirred for 45 min at room temperature then cooled to minus 10˚C with an ice/salt bath (internal thermometer). A solution of sodium nitrite (1 eq) in water (6 M) was carefully added via a dropping funnel, while the internal temperature was kept between minus 5 and 0˚C. After the addition, the reaction mixture was stirred for 1 h at 0˚C, then treated in one portion with a solution of sodium tetrafluoroborate (1.5 eq) in water (4.8 M). The precipitated salt was isolated by filtration, washed with cold 5% aqueous sodium tetrafluoroborate, ethanol and diethyl ether, then dried in the air. The beige solid (81%) thus obtained was used without further purification. Activated copper bronze (3 eq; for the activation see Vogel's Textbook of Practical Organic Chemistry, 5th ed., p. 426) was added to a mechanically stirred solution of sodium nitrite (12 eq) in water (6.7 M). A suspension of the foregoing compound (1 eq) in water (1 M) was added to the vigorously stirred mixture portionwise over 1 h at room temperature. After the addition, stirring was continued for another hour. The reaction mixture was diluted with dichloromethane and filtered through diatomaceous earth (Celite). After separation of the phases, the aqueous phase was extracted with dichloromethane and the combined organic layers were dried over sodium sulfate in the presence of activated charcoal. Filtration and evaporation afforded methyl 3-nitrothiophene-2-carboxylate as a red solid (92%), which was used without further purification. $\delta_H$ (400 MHz, DMSO $d_6$) 3.90 (3H, s), 7.43 (1H, d, *J* 5), 7.48 (1H, d, *J* 5).

The foregoing ester (1 eq) was stirred for 24 h at 100˚C in a well-closed Pyrex bottle in the presence of a solution of ammonia (6.15 eq) in methanol (2 M). The volatiles were removed *in vacuo,* and the residue crystallized from hot ethyl acetate to afford 3-nitrothiophene-2-carboxamide (74%). $\delta_H$ (400 MHz, DMSO-$d_6$) 3.28 (3H, s), 7.57 (1H, d, *J* 5), 7.73 (1H, d, *J* 5), 7.91 (1H, bs), 8.21 (1H, bs). An analytical sample was obtained by further recrystallization from ethyl acetate; elemental analysis, calc. for $C_5H_9N_2O_3S$: C, 34.88; H, 2.34; N, 16.27. Found: C, 34.75; H, 2.24; N, 15.96.

The foregoing amide (1 eq) was dissolved in dichloromethane (0.12 M). Triethylamine (3 eq) was added and the solution cooled to 0˚C. After dropwise addition of neat trifluoroacetic anhydride (1.3 eq), the reaction was allowed to warm to room temperature and stirred for 1 h. Then the solution was concentrated *in vacuo*, the residue taken into ethyl acetate and washed successively with hydrochloric acid (1 N, 2x), water (1x), saturated aqueous sodium hydrogencarbonate (2x) and brine. Drying over sodium sulfate and removal of solvent gave a dark solid, which was purified by flash chromatography (PE/EtOAc 7:3 + 1% MeOH) yielding 2-cyano-3-nitrothiophene (97%). $\delta_H$ ($CDCl_3$) 7.75 (1H, d, *J* 5.5), 7.68 (1H, d, *J* 5.5). An analytical sample was obtained by recrystallization from dichloromethane/*n*-pentane, mp 87-88˚C; elemental analysis, calc. for $C_5H_2N_2O_2S$: C, 38.96; H, 1.31; N, 18.17; S, 20.80. Found: C, 38.92; H, 1.20; N, 18.03; S, 21.31.

Step 2: *N'*-Hydroxy-3-nitrothiophene-2-carboximidamide

The nitrile (1 eq) prepared as described in Example 1, Step 1, was suspended in a mixture of water and ethanol (0.21 M, 7.5:1). Sodium carbonate (1.7 eq) and hydroxylamine (3.3 eq) were added and the mixture was left at room temperature for 24 h. The orange solid was isolated by filtration, washed with a small portion of diethyl ether and dried to give the amidoxime as an orange solid (86%). $\delta_H$ (400 MHz; DMSO-$d_6$) 6.08 (2H, bs), 7.60 (1H, d, *J* 5.3), 7.68 (1H, d, *J* 5.3), 9.95 (1H, bs). *m/z* ($ES^+$) 188 $(M+H)^+$. An analytical sample was obtained by recrystallization from dichloromethane/*n*-pentane, mp 201-202˚C; elemental analysis, calc. for $C_5H_5N_3O_3S$: C, 32.09; H, 2.69; N, 22.45; S, 17.13. Found: C, 32.34; H, 2.64; N, 21.96; S, 17.47.

Step 3: Dimethyl 2-({[1-amino-1-(3-nitrothien-2-yl)methylidene]-amino}oxy)but-2-enedioate

The amidoxime (1 eq), prepared as described above, was suspended in dichloromethane (0.25 M). Triethylamine (0.5 ml) and dimethyl acetylenedicarboxylate (1.05 eq, filtered over basic alumina) were added. The mixture was refluxed for 3 h and became homogeneous during this time. Evaporation of the dichloromethane gave a red oil, which was dissolved in ethyl acetate. After washing with water and brine, the organic phase was dried over sodium sulfate and the solution was concentrated *in vacuo.* The residual oil (97%) was used without further purification. $\delta_H$ (400 MHz; $CDCl_3$, 2 diastereomers 2.5:1*) 3.68*, 3.71 (3H, s), 3.83, 3.90* (3H, s), 5.88*, 5.94 (1H, s), 5.84*, 6.10 (2H, bs), 7.33, 7.38* (1H, d, *J* 5.6, 5.6*), 7.58, 7.61* (1H, d, *J* 5.6, 5.6*). *m/z* ($ES^+$) 330 $(M+H)^+$, 352 $(M+Na)^+$.

Step 4: Methyl 5-hydroxy-2-(3-nitrothien-2-yl)-6-oxo-1,6-dihydropyrimidine-4-carboxylate

A solution of the foregoing compound in xylene (0.2 M) was heated at reflux until the disappearance of the starting material was evident by TLC. The reaction mixture was stored in a refrigerator at 4˚C overnight and the precipitate isolated by filtration. The solid was washed with ethyl acetate and petroleum ether and dried *in vacuo.* The product was obtained as a yellow powder (48%). $\delta_H$ (400 MHz; DMSO-$D_6$) 3.82 (3H, s), 7.71 (1H, d, *J* 5.4), 7.89 (1H, d, *J* 5.4), 11.8 (1H, bs), 13.3 (1H, bs). *m/z* (ES-) 296 (M-H)-.

Step 5: Methyl 5-[(2,2-dimethylpropanoyl)oxy]-6-hydroxy-2-(3-nitrothien-2-yl)pyrimidine-4-carboxylate

The foregoing compound (1 eq) was dissolved in anhydr. pyridine (0.25 M) at room temperature. A catalytic amount of 4-DMAP was added, followed by neat pivaloyl chloride (1 eq). After 3 h at room temperature, the pyridine was removed *in vacuo,* the residue dissolved in ethyl acetate and washed with hydrochloric acid (1 N, 2x). After removal of the volatiles the crude product was recrystallized from ethyl acetate to give the title compound (65%) as a light brown solid. $\delta_H$ (400 MHz; DMSO-$d_6$) 1.30 (9H, s), 3.83 (3H, s), 7.73 (1H, d, *J* 5.5), 7.96 (1H, d, *J* 5.5), 13.9 (1H, bs). *m/z* ($ES^+$) 382 $(M+H)^+$.

Step 6: Methyl 5-[(2,2-dimethylpropanoyl)oxy]-1-methyl-2-(3-nitrothien-2-yl)-6-oxo-1,6-dihydropyrimidine-4-carboxylate

The foregoing compound (1 eq) was dissolved in anhydr. THF (0.2 M). Cesium carbonate (1.5 eq) was added, and the mixture was heated to 50˚C. After 5 min neat dimethyl sulfate (2 eq) was added dropwise, and the reaction was then stirred for 1 h at this temperature. After cooling to room temperature, the reaction mixture was diluted with ethyl acetate and the organic phase washed with aqu. ammonium chloride (2 M) and brine. Flash chromatography (PE/EtOAc 3:1, then 1:1) gave the title compound as a colourless solid (50%) as the second fraction. The first fraction contains the O-methylated compound (data not shown). $\delta_H$ (400 MHz; $CDCl_3$) 1.43 (9H, s), 3.40 (3H, s), 3.92 (3H, s), 7.57 (1H, d, *J* 5.5), 7.71 (1H, d, *J* 5.5). *m/z* ($ES^+$) 396 $(M+H)^+$. An analytical sample was obtained by recrystallization from dichloromethane/*n*-pentane, mp 159-161˚C; elemental analysis, calc. for $C_{16}H_{17}N_3O_7S$: C, 48.60; H, 4.33; N, 10.63; S, 8.11. Found: C, 48.56; H, 4.33; N, 10.66; S, 7.84.

Step 7: Methyl 5-[(2,2-dimethylpropanoyl)oxy]-1-methyl-2-(3-aminothien-2-yl)-6-oxo-1,6-dihydropyrimidine-4-carboxylate

The foregoing compound was dissolved in methanol (0.02 M). Pd/C (10% Pd, 20% in weight) was added, and the reaction stirred for 4 h under an atmosphere of hydrogen. Removal of the catalyst by filtration, exhaustive washing of the catalyst and evaporation of the solvents gave the title compound (93%) as a yellow powder, which was used without further purification. $\delta_H$ (400 MHz; DMSO-$d_6$) 1.29 (9H, s), 3.67 (3H, s), 3.83 (3H, s), 6.72 (1H, d, *J* 5.5), 7.05 (2H, bs), 7.68 (1H, d, *J* 5.5). *m/z* ($ES^+$) 366 $(M+H)^+$.

Step 8: 2-[3-({[(2-Chlorobenzyl)amino]carbonyl}amino)thien-2-yl]-5-hydroxy-1-methyl-6-oxo-1,6-dihydropyrimidine-4-carboxylic acid

To a solution of the foregoing amine (1 eq) in dichloromethane (0.1 M) and pyridine (3 eq) was added neat *ortho*-chlorobenzyl isocyanate (1.1 eq) at 0˚C. The resulting solution was stirred overnight at room temperature, whereupon another 1.1 eq of isocyanate was added. The reaction was brought to reflux for 5 h, then cooled to room temperature. After dilution with ethyl acetate, the organic phase was washed with hydrochloric acid (1 N), water, sat. aqu. sodium hydrogencarbonate, and brine. The crude product, obtained after evaporation of the solvents, was directly hydrolyzed using sodium hydroxide (0.5 M, 3 eq) in methanol (0.5 M) at 85˚C for 1 h. The solution was cooled to room temperature and acidified to pH 2 with hydrochloric acid (1 N). The crude product was purified by RP-HPLC. After lyophilization 2-[3-({[(2-chlorobenzyl)amino]carbonyl}amino)thien-2-yl]-5-hydroxy-1-methyl-6-oxo-1,6-dihydropyrimidine-4-carboxylic acid was obtained as a colourless powder (38%). $\delta_H$ (400 MHz; DMSO-$d_6$) 3.32 (3H, s), 4.31 (2H, d, *J* 5.8), 6.73 (1H, t, *J* 5.8), 7.22-7.35 (3H, m), 7.42 (1H, d, *J* 6.7), 7.53 (1H, d, *J* 5.4), 7.66 (1H, d, *J* 5.4), 8.88 (1H, s). MS ($ES^-$) 433, 435 $(M-H)^-$.

**EXAMPLE 2**

5-Hydroxy-1-methyl-6-oxo-2-(thien-2-yl)-1,6-dihydropyrimidine-4-carboxylic acid

Step 1: Methyl 5,6-dihydroxy-2-(thien-2-yl)pyrimidine-4-carboxylate

To a solution (1.1 M) of 2-thiophene amidoxime (1.0 eq) in chloroform was added dropwise dimethyl acetylenedicarboxylate (1.02 eq). The mixture was stirred at room temperature for 2 h, and then concentrated *in vacuo* to give a residue, which was taken up inp-xylene. The resulting solution (1.3 M) was heated at reflux for 5 h, then cooled to room temperature. The precipitate was collected by filtration, washed with methanol and diethyl ether and crystallized from acetic acid to afford the title compound (26%). $\delta_H$ (400 MHz; DMSO-$d_6$) 3.84 (3H, s), 7.16 (1H, dd, *J* 5.0, 3.5), 7.76 (1H, d, *J* 5.0), 7.99 (1H, d, *J* 3.5), 10.48 (1H, bs), 13.20 (1H, bs); *m/z* ($ES^+$) 253 $(M+H)^+$, 85%.

Step 2: Methyl 5-(benzoyloxy)-6-hydroxy-2-(thien-2-yl)pyrimidine-4-carboxylate

A solution (0.2 M) of methyl 5,6-dihydroxy-2-(thien-2-yl)pyrimidine-4-carboxylate (1.0 eq) in dichloromethane was treated with pyridine (10.0 eq) and benzoyl chloride (1.5 eq) was added dropwise. The resulting mixture was stirred at room temperature for 0.5 h, and then concentrated to afford a residue which was treated with aqueous hydrochloric acid (1 N) and ethyl acetate. The precipitate was filtered, triturated with diethyl ether and then dried to afford the title compound (76%). $\delta_H$ (300 MHz; DMSO-$d_6$) 3.77 (3H, s), 7.25 (1H, dd, *J* 5.1, 3.9), 7.62 (2H, t, *J* 7.8), 7.78 (1H, t, *J* 7.8), 7.93 (1H, d, *J* 5.1), 8.09 (2H, d, *J* 7.8), 8.18 (1H, d, *J* 3.9), 13.73 (1H, bs); *m/z* ($ES^+$) 357 $(M+H)^+$, 70%.

Step 3: Methyl 5-(benzoyloxy)-1-methyl-6-oxo-2-(thien-2-yl)-1,6-dihydropyrimidine-4-carboxylate

A solution (0.06 M) of methyl 5-(benzoyloxy)-6-hydroxy-2-(thien-2-yl)pyrimidine-4-carboxylate (1.0 eq) in tetrahydrofuran was treated with cesium carbonate (2.0 eq) and then methyl iodide (6.0 eq) was added dropwise. The resulting mixture was stirred at 40˚C for 12 h then concentrated *in vacuo* to afford a residue which was taken up with ethyl acetate and aqueous hydrochloric acid (1 N). The organic layer was washed with brine, dried and concentrated to give a solid that was purified by flash chromatography (25% ethyl acetate in petroleum ether) to give the title compound (32%). $\delta_H$ (300 MHz; DMSO-$d_6$) 3.73 (3H, s), 3.78 (3H, s), 7.28 (1H, dd, *J* 5.0, 3.9), 7.64 (2H, t, *J* 7.8), 7.80 (1H, t, *J* 7.8 Hz), 7.88 (1H, dd, *J* 3.9, 0.9), 7.96 (1H, dd, *J* 5.0, 0.9), 8.11 (2H, d, *J* 7.8).

Step 4: 5-Hydroxy-1-methyl-6-oxo-2-(thien-2-yl)-1,6-dihydropyrimidine-4-carboxylic acid

A suspension (0.09 M) of methyl 5-(benzoyloxy)-1-methyl-6-oxo-2-(thien-2-yl)-1,6-dihydropyrimidine-4-carboxylate (1.0 eq) in aqueous sodium hydroxide (0.5.N, 3 eq) was heated at 50˚C for 24 h. The resulting mixture was cooled to room temperature and acidified with aqueous hydrochloric acid (1 N). The precipitate was collected and washed with water and diethyl ether, then dried to give the title compound (25%). $\delta_H$ (300 MHz; DMSO-$d_6$) 3.58 (3H, s), 7.17 (1H, dd, *J* 4.8, 3.0), 7.59 (1H, d, *J* 3.0), 7.76 (1H, d, *J* 4.8); δc (75 MHz; DMSO-$d_6$) 34.4, 127.2, 127.7, 130.0, 130.1, 136.4, 143.7, 145.6, 159.0, 168.6; *m/z* ($ES^+$) 253 $(M+H)^+$, 100%.

**EXAMPLE 3**

5-Hydroxy-1-methyl-6-oxo-2-(thiazol-2-yl)-1,6-dihydropyrimidine-4-carboxylic acid

Step 1: 1,3-Thiazole-2-carbonitrile

2-Bromothiazole (1 eq) was added dropwise to a stirred solution of copper(I) cyanide (2.3 eq) in anhydr. DMF (1 M) at 140˚C. The reaction was stirred for 30 min. After cooling to 0˚C in an ice bath, the reaction was poured into diethyl ether and vigorously stirred for 10 min. The ethereal layer was decanted from the oily residue and washed with water and brine. After evaporation the desired nitrile was obtained as a colourless liquid (34%) and was used without further purification. $\delta_H$ (400 MHz; $CDCl_3$) 7.20 (1H, d, *J* 3.1), 7.77 (1H, d, *J* 3.1); *m*/*z* ($ES^+$) 111 $(M+H)^+$, 100%.

Step 2: 5-Hydroxy-1-methyl-6-oxo-2-(thiazol-2-yl)-1,6-dihydropyrimidine-4-carboxylic acid

Using essentially the procedure described in Example 2, except for the formation of the benzoate derivative in Step 2, which was obtained from 5,6-dihydroxy-2-(1,3-thiazol-2-yl)pyrimidine-4-carboxylic acid (1 eq) by coupling with benzoic acid (1.1 eq) using PyBOP (1.1 eq) and triethylamine (3.0 eq) in DMF (0.1 M), gave the title compound as a colourless powder after purification by RP-HPLC and lyophilization. $\delta_H$ (400 MHz; DMSO-$d_6$) 3.91 (3H, s), 7.90-8.10 (2H, m); $\delta_c$ (100 MHz; DMSO-$d_6$) 33.2, 124.7, 126.7, 140.6, 143.5, 146.5, 158.6, 163.1, 167.8; *m*/*z* ($ES^+$) 254 $(M+H)^+$, 100%.

**EXAMPLE 4**

5-Hydroxy-1-methyl-2-(3-nitrothien-2-yl)-6-oxo-1,6-dihydropyrimidine-4-carboxylic acid

Methyl 5-[(2,2-dimethylpropanoyl)oxy]-1-methyl-2-(3-nitrothien-2-yl)-6-oxo-1,6-dihydropyrimidine-4-carboxylate (obtained as described in Example 1, Step 6) was hydrolyzed as described in Example 1, Step 8 to afford the title compound. $\delta_H$ (400 MHz; DMSO-$d_6$) 3.25 (3H, s), 7.78 (1H, d, *J* 5.5), 7.97 (1H, d, *J* 5.5); $\delta_c$ (100 MHz; DMSO-$d_6$) 32.8, 122.8, 126.7, 129.5, 135.5, 139.6, 145.0, 147.7, 158.2, 167.1; *m*/*z* ($ES^-$) 296 $(M-H)^-$, 100%.

**EXAMPLE 5**

5-Hydroxy-1-methyl-2-(3-aminothien-2-yl)-6-oxo-1,6-dihydropyrimidine-4-carboxylic acid hydrochloride salt

Methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) was hydrolyzed as described in Example 1, Step 8 to afford the title compound. The product precipitated upon acidification, and was isolated by filtration and dried *in vacuo.* $\delta_H$ (400 MHz; $D_2O$ + 15 $\mu$l 1 N NaOH) 3.34 (3H, s), 6.66 (1H, d, *J* 5.3), 7.33 (1H, d, *J* 5.3); *m*/*z* ($ES^-$) 266 $(M-H)^-$, 80%.

**EXAMPLE 6**

5-Hydroxy-1-methyl-2-(3-bromothien-2-yl)-6-oxo-1,6-dihydropyrimidine-4-carboxylic acid

The title compound was prepared following generally the procedures outlined in Example 1, Steps 2-8 starting from 3-bromothiophene-2-carbonitrile. $\delta_H$ (400 MHz; DMSO-$d_6$) 3.30 (3H, s), 7.26 (1H, d, *J* 5.3), 7.90 (1H, d, *J* 5.3); *m*/*z* ($ES^+$) 331, 333 $(M+H)^+$.

**EXAMPLE 7**

2-[3-(Acetylamino)thien-2-yl]-5-hydroxy-1-methyl-6-oxo-1,6-dihydropyrimidine-4-carboxylic acid

Treating methyl 5-[(2,2-dimethylpropanoyl)oxy]-1-methyl-2-(3-aminothien-2-yl)-6-oxo-1,6-dihydropyrimidine-4-carboxylate (1 eq) (obtained as described in Example 1, Step 7) with acetic anhydride (2 eq) in DMF gave after hydrolysis, as described in Example 1, Step 8, the title compound after purification by RP-HPLC and lyophilization. $\delta_H$ (400 MHz; DMSO-$d_6$) 2.01 (3H, s), 3.34 (3H, s), 7.57 (1H, d, *J* 5.2), 7.71 (1H, d, *J* 5.2), 10.32 (1H, s); *m*/*z* ($ES^-$) 308 $(M-H)^-$, 100%.

**EXAMPLE 8**

2-[3-(Benzoylamino)thien-2-yl]-5-hydroxy-1-methyl-6-oxo-1,6-dihydropyrimidine-4-carboxylic acid

Treating methyl 5-[(2,2-dimethylpropanoyl)oxy]-1-methyl-2-(3-aminothien-2-yl)-6-oxo-1,6-dihydropyrimidine-4-carboxylate (1 eq) (obtained as described in Example 1, Step 7) with benzoyl chloride (1.1 eq) in chloroform (0.1 M) in the presence of a catalytic amount of 4-DMAP gave after hydrolysis, as described in Example 1, Step 8, the title compound after purification by RP-HPLC and lyophilization. $\delta_H$ (300 MHz; DMSO-$d_6$) 3.32 (3H, s), 7.49 (2H, dd, *J* 7.2, 7.4), 7.57 (1H, t, *J* 7.2), 7.62 (1H, d, *J* 5.4), 7.79 (1H, d, *J* 5.4), 7.90 (2H, d, *J* 7.4), 10.22 (1H, bs); *m/z* ($ES^-$) 370 $(M-H)^-$, 20%.

**EXAMPLE 9**

5-Hydroxy-1-methyl-2-(3-{[(2-methyl-1*H*-indol-3-yl)acetyl]amino}thien-2-yl)-6-oxo-1,6-dihydropyrimidine-4-carboxylic acid

(2-Methyl-1*H*-indol-3-yl)acetic acid (1.1 eq) was dissolved in DCM (0.1 M) and cooled to 0˚C. Triethylamine (1.1 eq) was added, followed by BOP-Cl. After stirring for 30 min at 0˚C, solid methyl 5-[(2,2-dimethylpropanoyl)oxy]-1-methyl-2-(3-aminothien-2-yl)-6-oxo-1,6-dihydropyrimidine-4-carboxylate (1 eq) (obtained as described in Example 1, Step 7) was added, followed by triethylamine (2.2 eq). The reaction was allowed to warm to room temperature and stirred overnight. The crude product, obtained after work-up from ethyl acetate, was hydrolyzed, as described in Example 1, Step 8, to give 5-hydroxy-1-methyl-2-(3-{[(2-methyl-1*H*-indol-3-yl)acetyl]amino}thien-2-yl)-6-oxo-1,6-dihydropyrimidine-4-carboxylic acid after purification by RP-HPLC and lyophilization (23%). $\delta_H$ (300 MHz; DMSO-$d_6$) 2.29 (3H, s), 3.27 (3H, s), 3.63 (2H, s), 6.86 (1H, t, *J* 7.6), 6.94 (1H, t, *J* 7.6), 7.18 (1H, d, *J* 7.6), 7.35 (1H, d, *J* 7.6), 7.54 (1H, d, *J* 5.4), 7.70 (1H, d, *J* 5.4), 10.48 (1H, bs), 10.77 (1H, bs); *m/z* ($ES^-$) 437 $(M-H)^-$, 80%.

**EXAMPLE 10**

2-(3-{[3-(2-Chlorophenyl)propanoyl]amino}thien-2-yl)-5-hydroxy-1-methyl-6-oxo-1,6-dihydropyrimidine-4-carboxylic acid

Following generally the experimental procedures in Example 9, the title compound was prepared from 3-(2-chlorophenyl)propionic acid and methyl 5-[(2,2-dimethylpropanoyl)oxy]-1-methyl-2-(3-aminothien-2-yl)-6-oxo-1,6-dihydropyrimidine-4-carboxylate (1 eq) (obtained as described in Example 1, Step 7) (40%). $\delta_H$ (300 MHz; DMSO-$D_6$) 2.61 (2H, t, *J* 7.4), 2.95 (2H, t, *J* 7.4), 3.29 (3H, s), 7.28-7.33 (3H, m), 7.36-7.41 (1H, m), 7.54 (1H, d, *J* 5.3), 7.72 (1H, d, *J* 5.3), 10.24 (1H, bs); *m/z* ($ES^+$) 434, 436 $(M+H)^+$.

**EXAMPLE 11**

2-{3-[(Anilinocarbonyl)amino]thien-2-yl}-5-hydroxy-1-methyl-6-oxo-1,6-dihydropyrimidine-4-carboxylic acid

Following generally the procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) and phenyl isocyanate. $\delta_H$ (400 MHz; DMSO-$d_6$) 3.41 (3H, s), 6.97 (1H, t, *J* 7.6), 7.27 (2H, t, *J* 7.6), 7.42 (2H, d, *J* 7.6), 7.68 (1H, d, *J* 5.6), 7.72 (1H, d, *J* 5.6), 8.70 (1H, bs), 9.40 (1H, bs); *m/z* ($ES^-$) 385 $(M-H)^-$, 100%.

**EXAMPLE 12**

2-(3-{[(1,1'-Biphenyl-2-ylamino)carbonyl]amino}thien-2-yl)-5-hydroxy-1-methyl-6-oxo-1,6-dihydropyrimidine-4-carboxylic acid

Following generally the procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) and 2-biphenylyl isocyanate (14%). $\delta_H$ (400 MHz; DMSO-$d_6$) 3.30 (3H, s), 7.12-7.27 (2H, m), 7.27-7.40 (4H, m), 7.43 (2H, t, *J* 7.0), 7.54 (1H, d, *J* 5.6), 7.63-7.78 (3H, m), 8.92 (1H, s); *m/z* ($ES^+$) 463 $(M+H)^+$, 100%.

**EXAMPLE 13**

2-(3-{[(Benzhydrylamino)carbonyl]amino}thien-2-yl)-5-hydroxy-1-methyl-6-oxo-1,6-dihydropyrimidine-4-carboxylic acid

Following generally the procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) and diphenylmethyl isocyanate (52%). $\delta_H$ (400 MHz; DMSO-$d_6$) 3.35 (3H, s), 5.92 (1H, d, *J* 8.0), 7.17 (1H, d, *J* 8.0), 7.21-7.28 (6H, m), 7.30-7.36 (4H, m), 7.57 (1H, d, *J* 5.4), 7.66 (1H, d, *J* 5.4), 8.73 (1H, s); *m/z* ($ES^-$) 475 $(M-H)^-$, 100%.

**EXAMPLE 14**

5-Hydroxy-1-methyl-2-{3-[({[1-(1-naphthyl)ethyl]amino}-carbonyl)amino]thien-2-yl}-6-oxo-1,6-dihydropyrimidine-4-carboxylic acid

Following generally the procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) and (*R,S*)-1-(naphthyl)ethyl isocyanate (26%). $\delta_H$ (400 MHz; DMSO-$D_6$) 1.51 (3H, d, *J* 6.7), 3.34 (3H, s), 5.52-5.64 (1H, m), 6.81 (1H, d, *J* 7.7), 7.48-7.57 (4H, m), 7.60 (1H, d, *J* 5.4), 7.65 (1H, d, *J* 5.4), 7.84 (1H, d, *J* 7.7), 7.95 (1H, d, *J* 8.0), 8. 10 (1H, d, *J* 8.3), 8.86 (1H, bs); *m/z* ($ES^-$) 463 $(M-H)^-$, 80%.

**EXAMPLE 15**

5-Hydroxy-1-methyl-6-oxo-2-[3-({[(2-phenylcyclopropyl)amino]-carbonyl}amino)thien-2-yl]-1,6-dihydropyrimidine-4-carboxylic acid

Generally following the experimental procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) and *trans*-2-phenylcyclopropyl isocyanate (47%). $\delta_H$ (400 MHz; DMSO-$d_6$) 1.09-1.18 (2H, m), 1.90-1.96 (1H, m), 2.65-2.72 (1H, m), 3.38 (3H, s), 6.68 (1H, bs), 7.08 (2H, d, *J* 7.4), 7.13 (1H, t, *J* 7.3), 7.23 (2H, t, *J* 7.4), 7.61 (1H, d, *J* 5.4), 7.67 (1H, d, *J* 5.4), 8.82 (1H, bs); *m/z* ($ES^-$) 425 $(M-H)^-$, 100%.

**EXAMPLE 16**

5-Hydroxy-1-methyl-6-oxo-2-[3-({[(2-phenylethyl)amino]-carbonyl}amino)thien-2-yl]-1,6-dihydropyrimidine-4-carboxylic acid

Generally following the experimental procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7), and 2-phenylethyl isocyanate (28%). $\delta_H$ (400 MHz; DMSO-$d_6$) 2.72 (2H, t, *J* 7.1), 3.13-3.27 (2H, m), 3.30 (3H, s), 6.19 (1H, t, *J* 5.8), 7.18-7.23 (3H, m), 7.25-7.32 (2H, m), 7.56 (1H, d, *J* 5.4), 7.65 (1H, d, *J* 5.4), 8.68 (1H, bs); *m/z* ($ES^-$) 413 $(M-H)^-$, 100%.

**EXAMPLE 17**

5-Hydroxy-2-{3-[(isobutoxycarbonyl)amino]thien-2-yl}-1-methyl-6-oxo-1,6-dihydropyrimidine-4-carboxylic acid

Following generally the experimental procedures described in Example 1, Step 8, the title compound was obtained from methyl 5-[(2,2-dimethylpropanoyl)oxy]-1-methyl-2-(3-aminothien-2-yl)-6-oxo-1,6-dihydropyrimidine-4-carboxylate (obtained as described in Example 1, Step 7) and isobutyl chloroformate, using triethylamine as the base (18%). $\delta_H$ (300 MHz; DMSO-$d_6$) 0.88 (6H, d, *J* 6.6), 1.79-1.93 (1H, m), 3.30 (3H, s), 3.82 (2H, d, *J* 6.6), 7.42 (1H, d, *J* 5.3), 7.70 (1H, d, *J* 5.3), 9.3 (1H, bs); *m/z* ($ES^-$) 366 $(M-H)^-$, 100%.

**EXAMPLE 18**

*N*[1]-Benzyl-*N*[2]-[2-(4-carboxy-5-hydroxy-1-methyl-6-oxo-1,6-dihydropyrimidin-2-yl)thiophen-3-yl]glycinamide

*N*-Benzyl-2-bromoacetamide (1 eq), obtained by reacting bromoacetyl bromide and benzylamine in chloroform in the presence of solid sodium hydrogencarbonate, was dissolved in DMSO (0.3 M). DIPEA (excess) was added, followed by solid methyl 5-[(2,2-dimethylpropanoyl)oxy]-1-methyl-2-(3-aminothien-2-yl)-6-oxo-1,6-dihydropyrimidine-4-carboxylate (1 eq). The mixture was heated at 50˚C overnight. The crude product, obtained after work-up from ethyl acetate, was hydrolysed, as described in Example 1, Step 8. The title compound was obtained after purification by RP-HPLC and lyophilization (12%). $\delta_H$ (400 MHz; DMSO-$d_6$) 3.52 (3H, s), 3.82 (2H, s), 4.29 (2H, d, *J* 5.9), 6.72 (1H, d, *J* 5.5), 7.17-7.25 (4H, m), 7.25-7.33 (2H, m), 7.60 (1H, d, *J* 5.5), 8.39 (1H, t, *J* 5.9); *m*/*z* ($ES^-$) 413 $(M-H)^-$, 100%.

**EXAMPLE 19**

5-Hydroxy-1-methyl-6-oxo-2-(3-{[(2*E*)-3-phenylpro-2-enyl]amino}thien-2-yl)-1,6-dihydropyrimidine-4-carboxylic acid

To a mixture of cinnamaldehyde (1.5 eq), acetic acid (5 eq) and methyl 5-[(2,2-dimethylpropanoyl)oxy]-1-methyl-2-(3-aminothien-2-yl)-6-oxo-1,6-dihydropyrimidine-4-carboxylate (1 eq) in 1,2-dichloroethane (0.2 M) was added sodium triacetoxyborohydride (2.5 eq) at room temperature. The mixture was stirred for 30 min at room temeperature, whereupon sat. aqu. sodium hydrogencarbonate was added. The aqueous phase was extracted with ethyl acetate. The crude product was recrystallized from ethyl acetate and diethyl ether to give methyl 5-[(2,2-dimethylpropanoyl)oxy]-1-methyl-6-oxo-2-(3-{[(2*E*)-3-phenylprop-2-enyl]amino}thien-2-yl)-1,6-dihydropyrimidine-4-carboxylate (82%) as a yellow powder, which was hydrolyzed using 0.5 N sodium hydroxide (2.2 eq) in methanol as described in Example 1, Step 8. 5-Hydroxy-1-methyl-6-oxo-2-(3-{[(2*E*)-3-phenylprop-2-enyl]amino}thien-2-yl)-1,6-dihydropyrimidine-4-carboxylic acid was obtained after purification by RP-HPLC and lyophilization (61%). $\delta_H$ (400 MHz; DMSO-$d_6$) 3.58 (3H, s), 3.99 (2H, d, *J* 5.6), 6.34 (1H, dt, *J* 5.6, 16.0), 6.59 (1H, d, *J* 16.0), 6.90 (1H, d, *J* 5.5), 7.22 (1H, t, *J* 7.2), 7.30 (2H, app. t, *J* 7.2, 7.4), 7.40 (2H, d, *J* 7.4), 7.62 (1H, t, *J* 5.5); *m*/*z* ($ES^-$) 382 $(M-H)^-$, 100%.

**EXAMPLE 20**

2-(4-Carboxy-5-hydroxy-1-methyl-6-oxo-1,6-dihydropyrimidin-2-yl)-*N*-(3-phenylpropyl)thiophen-3-aminium trifluoroacetate

Methyl 5-[(2,2-dimethylpropanoyl)oxy]-1-methyl-6-oxo-2-(3-{[(2*E*)-3-phenylprop-2-enyl]amino}thien-2-yl)-1,6-dihydropyrimidine-4-carboxylate (see Example 19) was dissolved in methanol/ethyl acetate (0.1 M solution, 3:2 v/v). Pd/C (10% Pd, 15% weight) was added and the mixture stirred under an atmosphere of hydrogen overnight. After removal of the catalyst by filtration, the crude product, which was obtained after evaporation of the solvents, was hydrolyzed using 0.5 N sodium hydroxide (2.2 eq) in methanol as described in Example 1, Step 8. The title compound was obtained after purification by RP-HPLC and lyophilization (31%). $\delta_H$ (400 MHz; DMSO-$d_6$) 1.81 (2H, m), 2.67 (2H, t, *J* 7.6), 3.19 (2H, t, *J* 6.9), 3.62 (3H, s), 6.83 (1H, d, *J* 5.5), 7.13-7.22 (3H, m), 7.24-7.30 (2H, m), 7.64 (1H, d, *J* 5.5); *m*/*z* (ES-) 384 $(M-H)^-$, 75%.

**EXAMPLE 21**

2-{3-[2-(Benzyloxy)ethyl]thien-2-yl}1-5-hydroxy-1-methyl-6-oxo-1,6-dihydropyrimidine-4-carboxylic acid

Step 1: 3-[2-Benzyloxy)ethyl]thiophene-2-carbonitrile

To a stirred solution of 3-(2-ethoxy-2-oxoethyl)thiophene-2-carboxylic acid (prepared from 3-bromothiophene-2-carboxylic acid according to D.E. Ames & O. Ribairo, *J. Chem. Soc., Perkin Trans. I,* **1975,** 1390-1395) in dioxane (0.7 M) was added pyridine (0.6 eq), di-*tert*-butyl dicarbonate and ammonium bicarbonate (1.26 eq). The mixture was stirred at room temperature for 3 days. Dioxane was evaporated *in vacuo,* the residue dissolved in ethyl acetate and the organic phase washed with water (1x), hydrochloric acid (1 N, 1x), water (1x) and brine (1x), and dried. Evaporation of the solvent afforded a brown solid (95%) which was dissolved in dichloromethane (0.3 M solution). Triethylamine (2.5 eq) was added and the solution cooled at 0˚C with an ice-bath. Trifluoroacetic anhydride was added dropwise and the mixture was stirred at room temperature for 1 h. The solvent was evaporated, the residue dissolved in ethyl acetate and the organic phase was then washed with HCl (1x), water (1x), $NaHCO_3$ and brine. Purification by flash chromatography (PE/EtOAc 5:1) gave ethyl (2-cyanothien-3-yl)acetate as a pale yellow oil (75%). This compound was dissolved in anhydr.

THF (0.4 M), then EtOH (4 eq) and $NaBH_4$ (1.1 eq) were added and the mixture was stirred at 50°C for 48 h. Ethanol was added to quench the reaction, the solvents were evaporated and the residue purified by flash chromatography (PE/EtOAc 1/1 + 1% ethanol), affording 3-(2-hydroxyethyl)thiophene-2-carbonitrile as a pale yellow oil (60%).

To a solution of the foregoing nitrile in anhydr. THF (0.6 M solution), cooled to 0°C, was added portionwise sodium hydride (1.4 eq) and after 10 min benzyl bromide (1.4 eq). The resultant mixture was stirred at 40°C overnight. Some water was added and the aqueous phase was extracted with ethyl acetate (3x), the combined organic layers were washed with brine and dried. Purification by flash chromatography (PE/EtOAc 5:1) afforded 3-[2-(benzyloxy)ethyl]thiophene-2-carbonitrile as a colourless oil (78%). $\delta_H$ (400 MHz; DMSO-$d_6$) 3.03 (2H, t, *J* 6.3), 3.70 (2H, t, *J* 6.3), 4.48 (2H, s), 7.20-7.35 (6H, m), 7.97 (1H, d, *J* 5.0); *m/z* (ES-) 242 $(M-H)^-$, 100%.

Step 2: 2-{3-[2-Benzyloxy)ethyl]thien-2-yl}-5-hydroxy-1-methyl-6-oxo-1,6-dihydropyrimidine-4-carboxylic acid

The title compound was prepared from the foregoing nitrile as described in Example 1, Steps 2-6 and 8 and was obtained as a white powder (3% overall) after purification by RP-HPLC and lyophilization. $\delta_H$ (400 MHz; DMSO-$d_6$) 2.78 (2H, t, *J* 6.5), 3.23 (3H, s), 3.60 (2H, t, *J* 6.5), 4.40 (2H, s), 7.12 (1H, d, *J* 5.1), 7.20-7.35 (5H, m), 7.70 (1H, d, *J* 5.1); *m/z* $(ES^-)$ 385 $(M-H)^-$, 100%.

## EXAMPLE 22

2-{4-[({[(2-Chlorophenyl)sulfonyl]amino}carbonyl)amino]thien-3-yl}-5-hydroxy-1-methyl-6-oxo-1,6-dihydropyrimidine-4-carboxylic acid

Step 1: *tert*-Butyl (4-cyanothien-3-yl)carbamate

A solution (0.6 M) of 4-aminothiophene-3-carbonitrile (1 eq) in methylene chloride was treated with pyridine (1.15 eq) and di-*tert*-butyl dicarbonate (2.1 eq) then heated under reflux for 45 min. The cooled solution was concentrated *in vacuo* and the residue was diluted with aqueous hydrochloric acid (1 N). After extraction with ethyl acetate the organic layer was concentrated *in vacuo* to give the title compound (83%) as a brown solid, which was used without further purification. $\delta_H$ (400 MHz; DMSO-$d_6$) 1.47 (9H, s), 7.41 (1H, s), 8.42 (1H, s), 9.35 (1H, bs).

Step 2: *tert*-Butyl {4-[amino(hydroxyimino)methyl]thien-3-yl}carbamate

A solution (0.5 M) of *tert*-butyl (4-cyanothien-3-yl)carbamate (1 eq) and hydroxylamine hydrochloride (1.4 eq) in methanol was treated with triethylamine (1.7 eq) and heated at 50°C for 12 h. The solution was cooled and concentrated, and the residue was taken up with water and ethyl acetate. The organic layer was separated and dried, then concentrated to give the title compound (100%) as an orange solid. $\delta_H$ (400 MHz; DMSO-$d_6$) 1.47 (9H, s), 6.05 (2H, s), 7.51 (1H, s), 7.90 (1H, s), 9.85 (1H, s), 10.15 (1H, s); *m/z* (ES-) 256 $(M-H)^-$.

Step 3: Methyl 2-{4-[(*tert*-butoxycarbonyl)amino]thien-3-yl}-5,6-dihydroxypyrimidine-4-carboxylate

Generally following the procedure described in Example 2, Step 1, *tert*-butyl {4-[amino(hydroxyimino)methyl] thien-3-yl}carbamate was converted to the title compound. After cyclization, the precipitate was washed with methanol and diethyl ether and dried to give the desired product (28%) as a pale yellow solid. $\delta_H$ (400 MHz; DMSO-$d_6$) 1.48 (9H, s), 3.85 (3H, s), 7.70 (1H, s), 8.49 (1H, s), 10.78 (1H, bs), 10.90 (1H, s), 13.10 (1H, bs); *m/z* $(ES^+)$ 368 $(M+H)^+$.

Step 4: Methyl 2-{4-[(*tert*-butoxycarbonyl)amino]thien-3-yl}-5-[(2,2-dimethylpropanoyl)oxy]-6-hydroxypyrimidine-4-carboxylate

Generally following the procedure in Example 1, Step 5, the title compound was obtained from methyl 2-{4-[(*tert*-butoxycarbonyl)amino]thien-3-yl}-5,6-dihydroxypyrimidine-4-carboxylate (1.0 eq) in pyridine and pivaloyl chloride (1.0 eq) as a brown solid (100%) after workup and was used without further purification. $\delta_H$ (400 MHz; DMSO-$d_6$) 1.32 (9H, s), 1.50 (9H, s), 3.87 (3H, s), 7.72 (1H, s), 8.66 (1H, s), 10.70 (1H, s), 13.46 (1H, bs); *m/z* $(ES^+)$ 452 $(M+H)^+$.

Step 5: Methyl 2-{4-[(*tert*-butoxycarbonyl)amino]thien-3-yl}-5-[(2,2-dimethylpropanoyl)oxy]-1-methyl-6-oxo-1,6-dihydropyrimidine-4-carboxylate

Generally following the procedure in Example 2, Step 3, methyl 2-{4-[(*tert*-butoxycarbonyl)amino]thien-3-yl}-5-[(2,2-dimethylpropanoyl)oxy]-6-hydroxypyrimidine-4-carboxylate (1.0 eq) was methylated using cesium carbonate (1.1

eq) and iodomethane (3.0 eq) in THF (0.12 M). The mixture was stirred overnight at 40°C, then cooled to room temperature, diluted with aqueous hydrochloric acid (1 N) and extracted with ethyl acetate. The organic layer was separated, dried and concentrated to give a residue which was purified by flash chromatography (25% ethyl acetate in petrol ether) to afford methyl 2-{4-[(*tert*-butoxycarbonyl)amino]thien-3-yl}-5-[(2,2-dimethylpropanoyl)oxy]-6-methoxypyrimidine-4-carboxylate (48%, no data given) as a white solid and the title compound (16%) as a yellow solid. $\delta_H$ (400 MHz; DMSO-$d_6$, 340 K) 1.33 (9H, s), 1.43 (9H, s), 3.38 (3H, s), 3.83 (3H, s), 7.46 (1H, d, *J* 3.4), 7.93 (1H, d, *J* 3.4), 9.16 (1H, bs); *m/z* (ES$^+$) 466 (M+H)$^+$.

Step 6: 2-{4-[({[(2-Chlorophenyl)sulfonyl]amino}carbonyl)amino]thien-3-yl}-5-hydroxy-1-methyl-6-oxo-1,6-dihydropyrimidine-4-carboxylic acid

Methyl 2-{4-[(*tert*-butoxycarbonyl)amino]thien-3-yl}-5-[(2,2-dimethylpropanoyl)oxy]-1-methyl-6-oxo-1,6-dihydropyrimidine-4-carboxylate was treated with a 1:1 mixture of DCM and TFA (0.2 M). The solution was stirred for 1 h at room temperature, then concentrated to dryness. The residue was dissolved in pyridine and the resulting solution (0.2 M) was treated dropwise with 2-chlorobenzenesulfonyl isocyanate (0.9 eq). The mixture was stirred at room temperature overnight, then concentrated *in vacuo.* The residue was washed with hydrochloric acid (1 N), water and diethyl ether to give a solid which was taken up in a 4:1 mixture of THF and water. Lithium hydroxide (5.0 eq) was added and the mixture was heated at 40°C for 2 h then cooled to room temperature and acidified to pH 2. The precipitate was collected, washed with water and diethyl ether then dried to afford the title compound (37%). $\delta_H$ (300 MHz; DMSO-$d_6$) 3.31 (3H, s), 7.49-7.75 (4H, m), 7.87 (1H, d, *J* 3.2), 8.08 (1H, d, *J* 7.6), 8.64 (1H, s); *m/z* (ES$^+$) 485, 487 (M+H)$^+$.

**EXAMPLE 23**

2-{3-[({[(2-Chlorophenyl)sulfonyl]amino}carbonyl)amino]thien-2-yl}-5-hydroxy-1-methyl-6-oxo-1,6-dihydropyrimidine-4-carboxylic acid

Methyl 2-(3-aminothien-2-yl)-5-[(2,2-dimethylpropanoyl)oxy]-1-methyl-6-oxo-1,6-dihydropyrimidine-4-carboxylate (obtained as described in Example 1, Step 7) was dissolved in anhydr. acetonitrile and the resulting solution (0.03 M) was treated dropwise with 2-chlorobenzenesulfonyl isocyanate (1.1 eq). The mixture was stirred at room temperature for 30 minutes, then concentrated *in vacuo.* The residue was washed with diethyl ether to give a solid, which was taken up in a 1:1 mixture of MeOH and dioxane. Sodium hydroxide (1 N, 4.0 eq) was added and the mixture was heated at 80°C for 2 h, then cooled to room temperature and acidified to pH 2. The precipitate was collected and washed with diethyl ether. Final purification by RP-HPLC gave the title compound as a light grey powder (16% yield). $\delta_H$ (400 MHz; DMSO-$d_6$) 3.31 (3H, s), 7.32-7.42 (4H, m), 7.63 (1H, d, *J* 5.2), 8.06 (1H, d, *J* 7.6), 8.97 (1H, s); *m/z* (ES$^+$) 485, 487 (M+H)$^+$.

**EXAMPLE 24**

5-Hydroxy-2-(3-hydroxyphenyl)-1-methyl-6-oxo-1,6-dihydropyrimidine-4-carboxylic acid

Step 1: Methyl 5-(benzoyloxy)-6-hydroxy-2-{3-[(4-methoxybenzyl)oxy]phenyl}pyrimidine-4-carboxylate

Following generally the procedures outlined in Example 1, Step 2, and Example 2, Steps 1 and 2, the title compound was prepared from 3-[(4-methoxybenzyl)oxy]benzonitrile. $\delta_H$ (400 MHz, DMSO-$d_6$) 3.75 (3H, s), 3.78 (3H, s), 5.10 (2H, s), 6.95 (2H, d, *J* 8.6), 7.23 (1H, d, *J* 7.0), 7.39-7.42 (2H, m), 7.46 (1H, t, *J* 8.0), 7.63 (2H, t, J 7.8), 7.70 (1H, d, *J* 7.8), 7.76-7.80 (2H, m), 8.10 (2H, d, *J* 7.4); *m/z* (ES$^+$) 487 (M+H)$^+$, 100%.

Step 2: Methyl 5-(benzoyloxy)-2-{3-[(4-methoxybenzyl)oxy]phenyl}-1-methyl-6-oxo-1,6-dihydropyrimidine-4-carboxylate

Essentially following the procedure in Example 2, Step 3, methyl 5-(benzoyloxy)-6-hydroxy-2-{3-[(4-methoxybenzyl)oxy]phenyl}pyrimidine-4-carboxylate was treated with cesium carbonate and methyl iodide in THF. The reaction afforded the *N*- and *O*-methyl derivatives in approximately 60/40 ratio, which were separated by flash chromatography (DCM/EtOAc 98:2) to give the title compound. $\delta_H$ (400 MHz, $CDCl_3$) 3.47 (3H, s), 3.82 (3H, s), 3.84 (3H, s), 5.05 (2H, s), 6.92 (2H, d, *J* 8.6), 7.10-7.12 (1H, m), 7.13 (1H, s), 7.34-7.54 (6H, m), 7.65 (1H, t, *J* 7.5), 8.21 (2H, d, *J* 8.6); *m/z* (ES$^+$) 501 (M+H)$^+$, 100%.

Step 3: Methyl 5-(benzoyloxy)-2-(3-hydroxyphenyl)-1-methyl-6-oxo-1,6-dihydropyrimidine-4-carboxylate

Methyl 5-(benzoyloxy)-2-{3-[(4-methoxybenzyl)oxy]phenyl}-1-methyl-6-oxo-1,6-dihydropyrimidine-4-carboxylate was suspended in a 4:1 mixture of DCM and TFA (0.1 M). After 30 min the volatiles were evaporated and the remaining solid was triturated with diethyl ether to obtain the title product. $\delta_H$ (400 MHz, DMSO-$d_6$) 3.48 (3H, s), 3.77 (3H, s), 6.96 (1H, d, *J* 8.3), 7.02 (1H, s), 7.07 (1H, d, *J* 7.3), 7.33 (1H, dd, *J* 7.0, 8.3), 7.63 (2H, t, *J* 7.8), 7.78 (1H, t) *J* 7.8), 8.10 (2H, d, *J* 7.8), 9.84 (1H, s); *m/z* ($ES^+$) 381 $(M+H)^+$, 100%.

Step 4: 5-Hydroxy-2-(3-hydroxyphenyl)-1-methyl-6-oxo-1,6-dihydropyrimidine-4-carboxylic acid

Methyl 5-(benzoyloxy)-2-(3-hydroxyphenyl)-1-methyl-6-oxo-1,6-dihydropyrimidine-4-carboxylate was suspended in 0.5 N NaOH and stirred overnight at 80˚C. After cooling down to room temperature and addition of 1 N HCl, the title product precipitated as a white solid, and was filtered off and dried under vacuum. $\delta_H$ (400 MHz, DMSO-$d_6$) 3.30 (3H, s), 6.88-6.95 (3H, m), 7.28 (1H, t, *J* 7.9), 9.74 (1H, bs); *m/z* ($ES^+$) 263 $(M+H)^+$, 100%.

**EXAMPLE 25**

2-(3-{[(1,1'-Biphenyl-2-ylamino)carbonyl]amino}phenyl)-5-hydroxy-1-methyl-6-oxo-1,6-dihydropyrimidine-4-carboxylic acid

Step 1: Methyl 5-[(2,2-dimethylpropanoyl)oxy]-6-hydroxy-2-(3-nitrophenyl)pyrimidine-4-carboxylate

This compound was obtained from 3-nitrobenzonitrile following essentially the procedures described in Example 1, Steps 2-6. $\delta_H$ (400 MHz; DMSO-$d_6$) 1.31 (9H, s), 3.87 (3H, s), 7.85 (1H, t, *J* 8.0), 8.44 (1H, d, *J* 8.0), 8.52 (1H, d, *J* 8.0), 8.93 (1H, s); *m/z* ($ES^+$) 376 $(M+H)^+$.

Step 2: Methyl 5-[(2,2-dimethylpropanoyl)oxy]-1-methyl-2-(3-nitrophenyl)-6-oxo-1,6-dihydropyrimidine-4-carboxylate

To a solution (0.1 M) of methyl 5-[(2,2-dimethylpropanoyl)oxy]-6-hydroxy-2-(3-nitrophenyl)pyrimidine-4-carboxylate (1 eq) in dry dioxane was added solid lithium hydride (1.4 eq) at 38˚C and the mixture was stirred for 45 min. A solution (0.2 M, 1.3 eq) of dimethyl sulfate in dry dioxane was added over 10 h at 60˚C *via* syringe pump. The mixture was stirred for a further 8 h, then the reaction was quenched with brine and extracted with ethyl acetate. The organic phase was dried, filtered and concentrated. The crude product was purified by flash chromatography (30% ethyl acetate in petroleum ether) to give the title compound (75%) as a white solid. $\delta_H$ (400 MHz; DMSO-$d_6$) 1.32 (9H, s), 3.31 (3H, s), 3.82 (3H, s), 7.85 (1H, t, *J* 8.0), 8.13 (1H, d, *J* 8.0), 8.42 (1H, d, *J* 8.0), 8.54 (1H, s); *m/z* ($ES^+$) 390 $(M+H)^+$, 80%.

Step 3: Methyl 2-(3-aminophenyl)-5-[2,2-dimethylpropanoyl)oxy]-1-methyl-6-oxo-1,6-dihydropyrimidine-4-carboxylate

To a solution (0.1 M) of methyl 5-[(2,2-dimethylpropanoyl)oxy]-1-methyl-2-(3-nitrophenyl)-6-oxo-1,6-dihydropyrimidine-4-carboxylate in methanol was added Pd/C (10% Pd, 10% weight). The suspension was stirred 4 h under hydrogen, then filtered and concentrated to give the title compound (97%) as yellow solid. $\delta_H$ (400 MHz; DMSO-$d_6$) 1.31 (9H, s), 3.31 (3H, s), 3.81 (3H, s), 5.38 (2H, s), 6.70-6.75 (3H, m), 7.15 (1H, t, *J* 7.6); *m/z* ($ES^+$) 360 $(M+H)^+$, 100%.

Step 4: 2-(3-{[(1,1'-Biphenyl-2-ylamino)carbonyl]amino}phenyl)-5-hydroxy-1-methyl-6-oxo-1,6-dihydropyrimidine-4-carboxylic acid

A solution (0.1 M) of methyl 2-(3-aminophenyl)-5-[(2,2-dimethylpropanoyl)oxy]-1-methyl-6-oxo-1,6-dihydropyrimidine-4-carboxylate in anhyd. chloroform was treated with 2-biphenylyl isocyanate (1.2 eq). The mixture was heated at 50˚C for 3 h, and then the solvent was evaporated. The residue was dissolved in a 1:1 mixture of tetrahydrofuran and sodium hydroxide (1 N). The resulting solution (0.05 M) was stirred at 80˚C for 1 h, cooled in an ice-bath and treated with hydrochloric acid (1 N). The precipitate was collected, then washed with water and diethyl ether, to give the title compound (60%) as a white solid. $\delta_H$ (400 MHz; DMSO-$d_6$) 3.31 (3H, s), 7.12-7.17 (2H, m), 7.22 (1H, d, *J* 7.0), 7.30-7.44 (5H, m), 7.46-7.52 (3H, m), 7.63 (1H, s), 7.69 (1H, s), 7.91 (1H, d, *J* 8.4), 9.21 (1H, s); *m/z* (ES-) 455 $(M-H)^-$, 100%.

**EXAMPLE 26**

2-[3-({[(3-Carboxyphenyl)amino]carbonyl}amino)phenyl]-5-hydroxy-1-methyl-6-oxo-1,6-dihydropyimidine-4-carboxylic acid

Following essentially the procedures described in Example 25, Step 4, the title compound was obtained from methyl 2-(3-aminophenyl)-5-[(2,2-dimethylpropanoyl)oxy]-1-methyl-6-oxo-1,6-dihydropyrimidine-4-carboxylate and ethyl 3-isocyanatobenzoate. $\delta_H$ (400 MHz; DMSO-$d_6$) 3.37 (3H, s), 7.22 (1H, d, *J* 7.8), 7.40-7.50 (2H, m), 7.57-7.61 (2H, m), 7.68 (1H, d, *J* 7.8), 7.75 (1H, s), 8.17 (1H, s), 8.99 (1H, s), 9.06 (1H, s); *m/z* ($ES^-$) 423 $(M-H)^-$, 80%.

## EXAMPLE 27

2-{3-[(Benzylsulfonyl)amino]thien-2-yl}-5-hydroxy-1-methyl-6-oxo-1,6-dihydropyrimidine-4-carboxylic acid

Step 1: *N*-Hydroxy-*N*-methyl-3-nitrothiophene-2-carboximidamide

The nitrile (1 eq), prepared as described in Example 1, Step 1, was suspended in a 3:1 mixture of water and ethanol (0.4 M). Sodium carbonate (1.25 eq) and *N*-methylhydroxylamine hydrochloride (2.5 eq) were added and the mixture was heated to 70˚C for 20 min. After evaporation of the solvent the residue was dissolved in ethanol and filtered through a pad of celite. The ethanol was evaporated and the resulting solid was triturated with ethyl ether, filtered and dried. The amidoxime was obtained as a red-orange solid (96%). $\delta_H$ (400 MHz, DMSO-$d_6$) 3.25 (3H, s), 7.57 (2H, bs), 7.81 (1H, d, *J* 5.4), 8.06 (1H, d, *J* 5.4). *m/z* ($ES^+$) 202 $(M+H)^+$, 40%.

Step 2: Methyl 5-(2-methoxy-2-oxoethyl)-2-methyl-3-(3-nitrothien-2-yl)-2,5-dihydro-1,2,4-oxadiazole-5-carboxylate

The amidoxime (1 eq) prepared as described above, was dissolved in a 1:1 mixture of chloroform and methanol (0.2 M) at 0˚C. Dimethyl acetylenedicarboxylate (1.05 eq, filtered over basic alumina) was added dropwise at the same temperature. The mixture was stirred at 0˚C for 2 h. Evaporation of the dichloromethane left a yellow oil, which was dissolved in ethyl acetate and dichloromethane. After washing with ammonium chloride (1 N, 2x), water and brine, the organic phase was dried over sodium sulfate and the solution was concentrated *in vacuo.* The residual oil (98%) was used without further purification. $\delta_H$ (400 MHz, DMSO-$d_6$) 3.03 (3H, s), 3.08 (1H, d, *J* 16.8), 3.32 (1H, d, *J* 16.8), 3.63 (3H, s), 3.75 (3H, s), 7.79 (1H, d, *J* 5.6), 8.05 (1H, d, *J* 5.6). *m/z* ($ES^+$) 344 $(M+H)^+$.

Step 3: Methyl 5-hydroxy-1-methyl-2-(3-nitrothien-2-yl)-6-oxo-1,6-dihydropyrimidine-4-carboxylate

A solution of the foregoing compound in xylene (0.2 M) was heated at reflux until the disappearance of the starting material was evident by TLC. The reaction mixture was stored in a refrigerator at 4˚C overnight and the precipitate isolated by filtration. The solid was washed with petroleum ether and dried *in vacuo.* The product was obtained as a brown powder (57%). $\delta_H$ (400 MHz, DMSO-$D_6$) 3.27 (3H, s), 3.80 (3H, s), 7.78 (1H, d, *J* 5.6), 7.98 (1H, d, *J* 5.6), 10.90 (1H, bs). *m/z* (ES-) 310 $(M-H)^-$.

Step 4: Methyl 5-(acetyloxy)-1-methyl-2-(3-nitrothien-2-yl)-6-oxo-1,6-dihydropyrimidine-4-carboxylate

Triethylamine (2.2 eq) and acetic anhydride (2 eq) were added to a solution of the foregoing compound (1 eq) in dichloromethane (0.2 M) and the reaction mixture was stirred for 16 h at room temperature. The mixture was then diluted with dichloromethane and washed with hydrochloric acid (1 N), sat. aqu. $NaHCO_3$, and brine. After removal of the volatiles, the title compound was obtained as a pale yellow solid (97%), which was used without further purification. $\delta_H$ (400 MHz; DMSO-$d_6$) 2.35 (3H, s), 3.30 (3H, s), 3.85 (3H, s), 7.80 (1H, d, *J* 5.5), 8.02 (1H, d, *J* 5.5); *m/z* ($ES^+$) 354 $(M+H)^+$, 100%.

Step 5: Methyl 5-(acetyloxy)-2-(3-aminothien-2-yl)-1-methyl-6-oxo-1,6-dihydropyrimidine-4-carboxylate

Pd/C (10% Pd, 20% in weight) was added to a solution (0.1 M) of methyl 5-(acetyloxy)-1-methyl-2-(3-nitrothien-2-yl)-6-oxo-1,6-dihydropyrimidine-4-carboxylate in ethyl acetate. The reaction mixture was stirred under an atmosphere of hydrogen for 2 h at room temperature. The catalyst was removed by filtration and washed with warm ethyl acetate and THF. After removal of the solvent *in vacuo* the title compound was obtained as a pale yellow solid (80%). $\delta_H$ (400 MHz; DMSO-$D_6$) 2.30 (3H, s), 3.20 (3H, s), 3.85 (3H, s), 6.72 (1H, d, *J* 5.4), 7.08 (2H, bs), 7.70 (1H, d, *J* 5.4); *m/z* ($ES^+$) 324 $(M+H)^+$, 100%.

Step 6: 2-{(3-[(Benzylsulfonyl)amino]thien-2-yl}-5-hydroxy-1-methyl-6-oxo-1,6-dihydropyrimidine-4-carboxylic acid

Triethylamine (6 eq) and benzylsulfonyl chloride (4 eq) were added to a solution of methyl 5-(acetyloxy)-2-(3-aminothien-2-yl)-1-methyl-6-oxo-1,6-dihydropyrimidine-4-carboxylate in acetonitrile (0.1 M). The reaction mixture was stirred for 16 h at room temperature. After dilution with ethyl acetate, the organic layer was washed with hydrochloric acid (1 N), sat. aqu. $NaHCO_3$, and brine. Methyl 5-(acetyloxy)-2-{3-[(benzylsulfonyl)amino]thien-2-yl}-1-methyl-6-oxo-1,6-dihydropyrimidine-4-carboxylate was obtained as a pale yellow solid (60%), which was dissolved (0.05 M solution) at room temperature in a 2:1 mixture of 1,4-dioxane and sodium hydroxide (1N). The reaction mixture was stirred at 75°C for 2 h, and then cooled to room temperature and acidified to pH 2 with hydrochloric acid (1 N). The solvent was removed *in vacuo* and the crude purified by preparative RP-HPLC to give after lyophilization 2-{3-[(benzylsulfonyl)amino]thien-2-yl}-5-hydroxy-1-methyl-6-oxo-1,6-dihydropyrimidine-4-carboxylic acid (50%) as a colourless solid. $\delta_H$ (400 MHz; DMSO-$d_6$) 3.48 (3H, s), 4.52 (2H, s), 7.10 (1H, d, *J* 5.4), 7.14-7.32 (5H, m), 7.72 (1H, d, *J* 5.4), 10.1 (1H, s); *m*/*z* ($ES^+$) 422 $(M+H)^+$, 100%.

**EXAMPLE 28**

5-Hydroxy-1-methyl-2-{3-[(2-naphthylsulphonyl)amino]thien-2-yl}-6-oxo-1,6-dihydropyrimidine-4-carboxylic acid

Following essentially the procedures described in Example 27, Step 6, gave the title compound as a colourless solid (30%). $\delta_H$ (400 MHz; DMSO-$d_6$) 3.10 (3H, s), 7.08 (1H, d, *J* 5.8), 7.60-7.80 (4H, m), 8.20-7.95 (3H, m), 8.40 (1H, s), 10.70 (1H, s); *m*/*z* ($ES^+$) 458 $(M+H)^+$, 100%.

**EXAMPLE 29**

2-(3-Formylthien-2-yl)-5-hydroxy-1-methyl-6-oxo-1,6-dihydropyrimidine-4-carboxylic acid

Step 1: 3-(1,3-Dioxolan-2-yl)thiophene-2-carbonitrile

3-(1,3-Dioxolan-2-yl)thiophene-2-carbaldehyde (1 eq), prepared as described by Hibino (S. Hibino et al., Journal of Organic Chemistry, 1984, 49, 5006) was dissolved in ethanol (1.1 M). At room temperature a solution of hydroxylamine hydrochloride (2 eq) and sodium hydrogencarbonate (1 eq) in water (1 M) was added and the resultant mixture stirred at this temperature for 2 h. The ethanol was removed under reduced pressure and the aqueous phase extracted with ethyl acetate (3x). The combined organic layers were washed with brine and dried. Evaporation afforded an off-white solid (quantitative) which was used without further purification. $\delta_H$ ($CDCl_3$; 2 isomers, 1.1:1*) 4.00-4.19 (4H, m), 5.99*, 6.09 (1H, s), 7.12*, 7.21 (1H, d, *J* 5.2*, 5.3), 7.25*, 7.50 (1H, d, *J* 5.2*, 5.3), 8.45, 8.56* (1H, s).

The foregoing aldoxime (1 eq) was dissolved in acetonitrile (0.4 M) containing copper(II) acetate hydrate (0.1 eq). Triethylamine (1.5 eq) was added dropwise and the resulting mixture was heated to reflux. After 30 min TLC (PE/EtOAc 3:1) indicated complete conversion of starting material. The reaction mixture was cooled to room temperature, the solvents were evaporated under reduced pressure and the crude product was purified by flash chromatography (PE/EtOAc 3:1) to give the nitrile (87%) as an oily liquid. $\delta_H$ (400 MHz; $CDCl_3$) 4.21-4.06 (4H, m), 6.04 (1H, s), 7.20 (1H, d, *J* 5.2), 7.55 (1H, d, *J* 5.2); $\delta_C$ ($CDCl_3$) 65.5, 98.6, 108.2, 112.8, 126.8, 131.9, 149.7.

Step 2: 3-(1,3-Dioxolan-2-yl)-*N*-hydroxy-*N*-methylthiophen-2-carboximidamide

A solution of *N*-methylhydroxylamine hydrochloride (3.7 eq) in water (0.3 M) and sodium carbonate (1.7 eq) was added to 3-(1,3-dioxolan-2-yl)thiophene-2-carbonitrile (1 eq). The reaction mixture was stirred at room temperature for 2 h and then partitioned between ethyl acetate and water. The combined organic layers were washed with brine, dried and evaporated to afford the crude title compound, which was used without further purification. $\delta_H$ (400 MHz; DMSO-$d_6$) 3.18 (3H, s), 3.91-3.97 (2H, m), 4.01-4.04 (2H, m), 5.72 (1H, s), 7.20 (1H, d, *J* 5.2), 7.51 (1H, bs), 7.85 (1H, d, *J* 5.2); *m*/*z* ($ES^+$) 229 $(M+H)^+$.

Step 3: Methyl 2-(3-formylthien-2-yl)-5-hydroxy-1-methyl-6-oxo-1,6-dihydropyrimidine-4-carboxylate

To a mixture of 3-(1,3-dioxolan-2-yl)-*N*-hydroxy-*N*-methylthiophen-2-carboximidamide (1 eq) in dichloromethane (0.4 M) was added dimethyl acetylenedicarboxylate (1.2 eq) at 0°C. The reaction mixture was stirred at room temperature for 2.5 h at which time the solvent was evaporated *vacuo.* The crude product was dissolved in xylene (0.3 M solution) and heated at reflux for 5 h. The reaction was cooled to room temperature and the volatiles removed under reduced pressure. The orange oil thus obtained was suspended in formic acid (0.1 M) and heated at 50°C for 1 h, then

cooled to room temperature and evaporated to give the title compound, which was used without further purification. *m/z* ($ES^-$) 293 $(M-H)^-$; *m/z* ($ES^+$) 295 $(M+H)^+$.

Step 4: Methyl 5-[(2,2-dimethylpropanoyl)oxy]-2-(3-formylthien-2-yl)-1-methyl-6-oxo-1,6-dihydropyrimidine-4-carboxylate

To a solution of the foregoing compound (1 eq) in pyridine (0.2 M) was added 4-DMAP (catalytic amount) and pivaloyl chloride (1.05 eq). The reaction mixture was stirred at room temperature for 1 h, and was then partitioned between EtOAc and hydrochloric acid (1 N). The organic layer was dried. The residue obtained after evaporation was purified by flash chromatography (PE/EtOAc 1:1) to afford the title compound (16%) as a brown oil. $\delta_H$ (400 MHz; $CDCl_3$) 1.43 (9H, s), 3.44 (3H, s), 3.92 (3H, s), 7.59 (2H, s), 9.95 (1H, s).

Step 5: 2-(3-Formylthien-2-yl)-5-hydroxy-1-methyl-6-oxo-1,6-dihydropyrimidine-4-carboxylic acid

To a solution of methyl 5-[(2,2-dimethylpropanoyl)oxy]-2-(3-formylthien-2-yl)-1-methyl-6-oxo-1,6-dihydropyrimidine-4-carboxylate (1 eq) in methanol (1 M) was added sodium hydroxide (1 N). The reaction mixture was heated at 50˚C for 2 h. Hydrochloric acid was added to the cooled solution until pH 2 was reached, and the product purified by preparative RP-HPLC (Novapak (Waters) C18 Cartridge Column, 7 micron, 25 x 100 mm; flow: 10 ml/min). Lyophilization afforded 2-(3-formylthien-2-yl)-5-hydroxy-1-methyl-6-oxo-1,6-dihydropyrimidine-4-carboxylic acid (33%). $\delta_H$ (300 MHz; DMSO-$d_6$) 3.26 (3H, s), 7.58 (1H, d, *J* 5.1), 7.89 (1H, d, *J* 5.1), 9.83 (1H, s); *m/z* ($ES^+$) 281 $(M+H)^+$, 100%.

**EXAMPLE 30**

2-(3-Carboxythien-2-yl)-5-hydroxy-1-methyl-6-oxo-1,6-dihydropyrimidine-4-carboxylic acid

Step 1: Methyl 5-[(2,2-dimethylpropanoyl)oxy]-2-(3-carboxythien-2-yl)-1-methyl-6-oxo-1,6-dihydropyrimidine-4-carboxylate

To a solution of methyl 5-[(2,2-dimethylpropanoyl)oxy]-2-(3-formylthien-2-yl)-1-methyl-6-oxo-1,6-dihydropyrimidine-4-carboxylate (1 eq; obtained as described in Example 29, Step 4) in *tert*-butanol (0.05 M) and 2-methyl-2-butene (10 eq, 2 M in tetrahydrofuran) was added an aqueous (0.82 M) solution of sodium chlorite (7 eq) and sodium dihydrogenphosphate (7 eq). The reaction was stirred at room temperature for 2 h, at which time the solvent was evaporated *in vacuo.* The residue was partitioned between ethyl acetate and hydrochloric acid (1 N). The combined organic layers were washed with brine and dried. Evaporation gave the title compound (100%). $\delta_H$ (400 MHz; DMSO-$d_6$) 1.31 (9H, s), 3.24 (3H, s), 3.81 (3H, s), 7.49 (1H, d, *J* 5.2), 7.88 (1H, d, *J* 5.2), 13.3 (1H, bs); *m/z* ($ES^+$) 395 $(M+H)^+$, 100%.

Step 2: 2-(3-Carboxythien-2-yl)-5-hydroxy-1-methyl-6-oxo-1,6-dihydropyrimidine-4-carboxylic acid

The foregoing product was dissolved in MeOH (0.1 M) then sodium hydroxide (0.5 N, 4 eq) was added. The reaction mixture was heated at 80˚C for 1.5 h. Hydrochloric acid (1 N) was added to the cooled solution until pH 3 was reached and the product purified by preparative RP-HPLC (Novapak (Waters) C18 Cartridge Column, 7 micron, 25 x 100 mm; flow: 10 ml/min). Lyophilization gave 2-(3-carboxythien-2-yl)-5-hydroxy-1-methyl-6-oxo-1,6-dihydropyrimidine-4-carboxylic acid (39%). $\delta_H$ (400 MHz; DMSO-$d_6$) 3.21 (3H, s), 7.47 (1H, d, *J* 5.2), 7.82 (1H, d, *J* 5.2), 13.2 (1H, bs); *m/z* ($ES^+$) 297 $(M+H)^+$, 100%.

**EXAMPLE 31**

2[3-({[2-(2-Chlorophenyl)ethyl]amino}carbonyl)thien-2-yl]-5-hydroxy-1-methyl-6-oxo-1,6-dihydropyrimidine-4-carboxylic acid

To a solution of 2-[5-[(2,2-dimethylpropanoyl)oxy]-4-(methoxycarbonyl)-1-methyl-6-oxo-1,6-dihydropyrimidin-2-yl]thiophene-3-carboxylic acid (1 eq), obtained as described in Example 30, Step 1, and 2-(2-chlorophenyl)ethylamine (1.3 eq) in dichloromethane (0.05 M) were added HATU (1.3 eq) and DIPEA (1.3 eq). The reaction mixture was stirred overnight at room temperature, at which time the solvent was evaporated *in vacuo.* The resulting mixture was partitioned between EtOAc and hydrochloric acid (1 N). The combined organic layers were washed with brine. Evaporation afforded methyl 2-[3-({[2-(2-chlorophenyl)ethyl]amino}carbonyl)thien-2-yl]-5-[(2,2-dimethylpropanoyl)oxy]-1-methyl-6-oxo-1,6-dihydropyrimidine-4-carboxylate (90%), which was dissolved in MeOH (0.1 M). Sodium hydroxide (0.5 N, 4 eq) was added and the reaction mixture was heated at 80˚C for 1.5 h. After cooling it to room temperature, hydrochloric acid (1

N) was added until an acidic pH was reached. The product was purified by preparative RP-HPLC (Novapak (Waters) C18 Cartridge Column, 7 micron, 25 x 100 mm; flow: 10 ml/min). Lyophilization gave 2-[3-({[2-(2-chlorophenyl)ethyl] amino}carbonyl)thien-2-yl]-5-hydroxy-1-methyl-6-oxo-1,6-dihydropyrimidine-4-carboxylic acid (38%). $\delta_H$ (400 MHz; DMSO-$d_6$) 2.91 (2H, t, *J* 7.2), 3.13 (3H, s), 3.35 (2H, m), 7.41-7.22 (3H, m), 7.39-7.41 (1H, m), 7.57 (1H, d, *J* 5.6), 7.81 (1H, d, *J* 5.6), 8.60 (1H, bs); *m/z* ($ES^+$) 434, 436 $(M+H)^+$.

**EXAMPLE 32**

5-Hydroxy-1-methyl-6-oxo-2-{3-[(*E*)-2-phenylethenyl]thien-2-yl}-1,6-dihydropyrimidine-4-carboxylic acid

Step 1: Methyl 5-*tert*-butoxy-2-(3-formylthien-2-yl)-1-methyl-6-oxo-1,6-dihydropyrimidine-4-carboxylate

Crude methyl 2-(3-formylthien-2-yl)-5-hydroxy-1-methyl-6-oxo-1,6-dihydropyrimidine-4-carboxylate, obtained as described in Example 29, Step 3, was dissolved in THF (0.4 M solution), *O-tert*-butyl-*N,N'*-diisopropylisourea was added and the mixture was heated to reflux. After 20 min at reflux the solvent was evaporated *in vacuo* and the crude product purified by flash chromatography (PE/EtOAc 1:1) to afford methyl 5-*tert*-butoxy-2-(3-formylthien-2-yl)-1-methyl-6-oxo-1,6-dihydropyrimidine-4-carboxylate (10%). $\delta_H$ (400 MHz; DMSO-$d_6$) 1.37 (9H, s), 3.25 (3H, s), 3.81 (3H, s), 7.63 (1H, d, *J* 5.2), 7.94 (1H, d, *J* 5.2), 9.87 (1H, s); *m/z* ($ES^+$) 351 $(M+H)^+$, 100%.

Step 2: Methyl 5-*tert*-butoxy-1-methyl-6-oxo-2-[3-(2-phenylethenyl)thien-2-yl]-1,6-dihydropyrimidine-4-carboxylate

To a 0.25 M solution of benzyl(triphenyl)phosphonium bromide (1.1 eq) in anhydr. methanol was added sodium methoxide (1.1 eq), and the mixture was stirred 10 min at 50˚C. Then a 0.22 M solution of the foregoing aldehyde (1 eq) in anhydr. methanol was added dropwise. The brown mixture was stirred 1 h at 50˚C. Methanol was removed under reduced pressure, then water was added and the aqueous phase extracted with ethyl acetate. The combined organic layers were washed with water and brine. Purification by flash chromatography (PE/EtOAc 3: 1) gave a mixture of *Z*/*E* stereoisomers of methyl 5-*tert*-butoxy-1-methyl-6-oxo-2-[3-(2-phenylethenyl)thien-2-yl]-1,6-dihydropyrimidine-4-carboxylate (53%) as a pale yellow oil. $\delta_H$ (300 MHz; $CDCl_3$, 2 stereoisomers *E*/*Z* 1.3:1*) 1.44*, 1.49 (9H, s), 3.43, 3.45* (3H, s), 3.89*, 3.92 (3H, s), 6.31* (1H, d, *J* 12.1), 6.66* (1H, d, *J* 12.1), 6.48 (1H, d, *J* 16.2), 6.84* (1H, d, *J* 5.2), 6.98 (1H, d, *J* 16.2), 7.10-7.40 (6H, m), 7.45 (1H, d, *J* 5.2); *m/z* ($ES^+$) 425 $(M+H)^+$, 20%.

Step 3: Methyl 5-hydroxy-1-methyl-6-oxo-2-[3-(2-phenylethenyl)thien-2-yl]-1,6-dihydropyrimidine-4-carboxylate

The foregoing compound was dissolved in an excess of TFA and stirred at room temperature for 10 min. TFA was then removed under reduced pressure and co-evaporated with toluene affording methyl 5-hydroxy-1-methyl-6-oxo-2-[3-(2-phenylethenyl)thien-2-yl]-1,6-dihydropyrimidine-4-carboxylate (95%) as a yellow solid. $\delta_H$ (300 MHz; $CDCl_3$, 2 stereoisomers *E*/*Z* 1.3:1*) 3.47, 3.49* (3H, s), 4.01*, 4.03 (3H, s), 6.32* (1H, d, *J* 12.2), 6.66* (1H, d, *J* 12.2), 6.80 (1H, d, *J* 16.2), 6.88* (1H, d, *J* 5.2), 7.11 (1H, d, *J* 16.2), 7.10-7.40 (6H, m), 7.45 (1H, d, *J* 5.2), 10.80 (1H, bs); *m/z* ($ES^+$) 369 $(M+H)^+$, 100%.

Step 4: 5-Hydroxy-1-methyl-6-oxo-2{3-[(*E*)-2-phenylethenyl]thien-2-yl}-1,6-dihydropyirimidine-4-carboxylic acid

The methyl ester moiety of the foregoing compound was hydrolyzed as described in Example 1, Step 8. Purification by RP-HPLC and lyophilization afforded the title compound as a colourless solid (30%). $\delta_H$ (400 MHz; DMSO-$d_6$) 3.25 (3H, s), 7.04 (1H, d, *J* 16.3), 7.23 (1H, d, *J* 16.3), 7.26 (1H, t, *J* 7.1), 7.33 (2H, dd, *J* 7.1, 7.4), 7.57 (2H, d, *J* 7.4), 7.63 (1H, d, *J* 5.0), 7.79 (1H, d, *J* 5.0); *m/z* (ES-) 353 $(M-H)^-$, 60%.

**EXAMPLE 33**

5-Hydroxy-1-methyl-6-oxo-2-[3-(2-phenylethyl)thien-2-yl]-1,6-dihydropyrimidine-4-carboxylic acid

The compound obtained in Example 32, Step 2, was dissolved in methanol (0.1 M solution) and hydrogenated under balloon pressure in the presence of Pd/C (10% Pd, 10% in weight). After 3 h the catalyst was removed by filtration, the solvent evaporated and the crude product was deprotected and purified as described in Example 32, Steps 3 and 4, to give the title compound (71%). $\delta_H$ (400 MHz; DMSO-$d_6$) 2.70-2.90 (4H, m), 3.10 (3H, s), 7.09-7.16 (4H, m), 7.17-7.24 (2H, m), 7.70 (1H, d, *J* 5.1); *m/z* (ES-) 355 $(M-H)^-$, 80%.

**EXAMPLE 34**

2-{3-[(1*E*)-4-(2-Chlorophenyl)but-1-enyl]thien-2-yl}-5-hydroxy-1-methyl-6-oxo-1,6-dihydropyrimidine-4-carboxylic acid

Following essentially the procedures described in Example 32, the title compound was obtained from methyl 5-*tert*-butoxy-2-(3-formylthien-2-yl)-1-methyl-6-oxo-1,6-dihydropyrimidine-4-carboxylate and [3-(2-chlorophenyl)propyl](triphenyl)phosphonium bromide as a 2/1 mixture of *Z* and *E* stereoisomers (28%). The Wittig reaction was carried out in DCM at 50˚C, using potassium *tert*-butoxide as the base. The required phosphonium salt was synthesized in three steps from 3-(2-chlorophenyl)propionic acid by reduction to the alcohol, conversion to the bromide, and reaction with triphenylphosphine. After the Wittig reaction, the product was deprotected as described in Example 32, Step 3. It was then converted completely into the *E* isomer by dissolving it in benzene (0.1 M solution), containing a few crystals of iodine, and irradiating the solution for 9 h with a sun lamp (400 Watt). After hydrolysis, performed as described in Example 32, Step 4, 2-{3-[(1*E*)-4-(2-chlorophenyl)but-1-enyl]thien-2-yl}-5-hydroxy-1-methyl-6-oxo-1,6-dihydropyrimidine-4-carboxylic acid was obtained after purification by RP-HPLC (Novapak (Waters) C18 Cartridge Column, 7 micron, 25 x 100 mm; flow: 10 ml/min) and lyophilization as an off-white solid (13% overall). $\delta_H$ (400 MHz; $CD_3CN$) 2.50 (2H, m), 2.89 (2H, t, *J* 7.4), 3.20 (3H, s), 6.18 (1H, d, *J* 15.9), 6.28 (1H, dt, *J* 15.9, 6.6), 7.16-7.30 (3H, m), 7.31 (1H, d, *J* 5.1), 7.36 (1H, d, *J* 7.7), 7.56 (1H, d, *J* 5.1); *m*/*z* ($ES^+$) 417, 419 $(M+H)^+$.

**EXAMPLE 35**

5-Hydroxy-1-methyl-6-oxo-2-[3-(4-phenylbutyl)thien-2-yl]-1,6-dihydropyrimidine-4-carboxylic acid

Following generally the procedures of Example 32, Steps 1 and 2, and then the procedures of Example 33 the title compound was obtained as a white solid (21% overall) from methyl 5-*tert*-butoxy-2-(3-formylthien-2-yl)-1-methyl-6-oxo-1,6-dihydropyrimidine-4-carboxylate and 3-phenylpropyl(triphenyl)phosphonium bromide. The Wittig reaction was carried out in DCM at 50˚C, using potassium *tert*-butoxide as the base. $\delta_H$ (400 MHz; DMSO-$D_6$) 1.46-1.54 (4H, m), 2.50 (4H, under DMSO, m), 3.20 (3H, s), 7.05 (1H, d, *J* 4.7), 7.10-7.18 (3H, m), 7.19-7.24 (2H, m), 7.70 (1H, d, *J* 5.1); *m*/*z* ($ES^-$) 383 $(M-H)^-$, 60%.

**EXAMPLE 36**

5-Hydroxy-2-{3-[(4-methoxybenzyl)oxy]phenyl}-1-methyl-6-oxo-1,6-dihydropyrimidine-4-carboxylic acid

Following essentially the procedure outlined in Example 27, Steps 1-3, the title compound was prepared from 3-[(4-methoxybenzyl)oxy]benzonitrile. The amidoxime was synthesized using triethylamine (2.2 eq) in refluxing ethanol. After hydrolysis (see Example 24, Step 4), the mixture was cooled to room temperature. Upon addition of hydrochloric acid the title compound precipitated as a white solid, which was filtered off and dried *in vacuo.* $\delta_H$ (400 MHz; DMSO-$d_6$) 3.19 (3H, s), 3.74 (3H, s), 5.05 (2H, s), 6.92 (2H, d, *J* 8.2), 7.01-7.12 (3H, m), 7.38-7.48 (3H, m); *mlz* ($ES^+$) 383 $(M+H)^+$, 100%.

**EXAMPLE 37**

2-{2-[(3,4-Dichlorobenzyl)oxy]phenyl}-5-hydroxy-1-methyl-6-oxo-1,6-dihydropyrimidine-4-carboxylic acid

Following essentially the procedures outlined in Example 27 the title compound was prepared from 2-[(3,4-dichlorobenzyl)oxy]benzonitrile. After cyclization, the crude product was hydrolyzed with sodium hydroxide (0.5 N) for 6 h at 90˚C. The mixture was cooled to room temperature, hydrochloric acid (1 N) was added and the title compound precipitated as a white solid. After filtration, it was triturated with $Et_2O$ and dried. $\delta_H$ (400 MHz; DMSO-$d_6$) 3.12 (3H, s), 5.16 (2H, m), 7.08 (1H, t, *J* 7.5), 7.18 (1H, d, *J* 8.3), 7.28-7.34 (2H, m), 7.45-7.51 (2H, m), 7.57 (1H, d, *J* 8.3); *m*/*z* ($ES^+$) 422 $(M+H)^+$, 100%.

The starting nitrile was obtained by alkylation of 2-hydroxybenzonitrile (1 eq) in DMF with 3,4-dichlorobenzyl chloride (0.9 eq), using $K_2CO_3$ (2 eq) as the base. The mixture was kept at 60˚C overnight. After cooling to room temperature, the reaction mixture was partitioned between EtOAc and hydrochloric acid (1 N). The organic phase was washed with NaOH (1 N) and brine. $\delta_H$ (400 MHz; $CDCl_3$), 5.16 (2H, s), 6.96 (1H, d, *J* 8.5), 7.06 (1H, t, *J* 7.5), 7.33 (1H, d, *J* 8.2), 7.47-7.54 (3H, m), 7.60 (1H, d, *J* 7.7); *mlz* ($ES^+$) 279 $(M+H)^+$, 100%.

**EXAMPLE 38**

2-(Furan-2-yl)-5-hydroxy-1-methyl-6-oxo-1,6-dihydropyrimidine-4-carboxylic acid

Step 1: Ethyl 2-ethoxy-2-(1-methyl-2,5-dioxoimidazolidin-4-ylidene)-ethanoate

A mixture of diethyl 2-ethoxy-3-oxosuccinate (1 eq), prepared according to US patent 5,925,764, and *N*-methylurea (1 eq) was refluxed for 3 h in acetic acid containing 1 mol hydrogen chloride (0.2 M solution). The cooled reaction mixture was evaporated to dryness and the residue co-evaporated with toluene and dried *in vacuo* to afford ethyl 2-ethoxy-2-(1-methyl-2,5-dioxoimidazolidin-4-ylidene)ethanoate (99%) as a white solid. $\delta_H$ (400 MHz; DMSO-$D_6$, 3:1 *E*/*Z** ratio) 1.23-1.29 (6H, m), 2.86*, 2.91 (3H, s), 3.87, 3.94* (2H, q, *J* 6.8, 7.2*), 4.28 (2H, q, *J* 7.2), 10.03, 10.46* (1H, s); *m*/*z* ($ES^+$) 243 $(M+H)^+$, 80%.

Step 2: 5-Ethoxy-2-hydroxy-1-methyl-6-oxo-1,6-dihydropyrimidine-4-carboxylic acid

Ethyl 2-ethoxy-2-(1-methyl-2,5-dioxoimidazolidin-4-ylidene)-ethanoate (1 eq) was suspended in potassium hydroxide (1 N, 4 eq) and refluxed for 3 h. The reaction mixture was cooled to 0˚C and carefully acidified with concentrated HCl. After standing at 4˚C overnight, a white precipitate had formed, which was isolated by filtration and dried *in vacuo* to give 5-ethoxy-2-hydroxy-1-methyl-6-oxo-1,6-dihydropyrimidine-4-carboxylic acid (63%) as a white solid. $\delta_H$ (400 MHz; DMSO-$d_6$) 1.20 (3H, t, *J* 6.8), 3.13 (3H, s), 3.93 (2H, q, *J* 6.8), 10.89 (1H, s), 14.2 (1H, bs); $\delta_C$ (100 MHz; DMSO) 14.9, 27.2, 68.8, 131.6, 132.0, 149.3, 160.8, 161.7; *m*/*z* (ES-) 213 $(M-H)^-$, 100%.

Step 3: Ethyl 5-ethoxy-2-hydroxy-1-methyl-6-oxo-1,6-dihydropyrimidine-4-carboxylate

Acetyl chloride (20 eq) was added dropwise at 0˚C to absolute ethanol (0.06 M solution). After stirring the resulting solution for 20 minutes at room temperature 5-ethoxy-2-hydroxy-1-methyl-6-oxo-1,6-dihydropyrimidine-4-carboxylic acid (1 eq) was added in one portion and the reaction mixture was refluxed overnight. Volatiles were evaporated *in vacuo* and the residue co-evaporated with dichloromethane and dried to give ethyl 5-ethoxy-2-hydroxy-1-methyl-6-oxo-1,6-dihydropyrimidine-4-carboxylate (98%) as an off-white solid. $\delta_H$ (400 MHz; DMSO-$D_6$) 1.22 (3H, t, *J* 7.1), 1.30 (3H, t, *J* 7.1), 3.14 (3H, s), 3.95 (2H, q, *J* 7.1), 4.30 (2H, q, *J* 7.1), 11.03 (1H, bs); *m*/*z* ($ES^+$) 243 $(M+H)^+$.

Step 4: Ethyl 2-chloro-5-ethoxy-1-methyl-6-oxo-1,6-dihydropyrimidine-4-carboxylate

*N,N*-Dimethylaniline (1.4 eq) was added to a stirred solution of ethyl 5-ethoxy-2-hydroxy-1-methyl-6-oxo-1,6-dihydropyrimidine-4-carboxylate (1 eq) in phosphorus oxychloride (0.14 M solution) and the mixture refluxed overnight. The volatiles were evaporated *in vacuo* and the residue was poured into ice water and extracted into diethyl ether. The combined ethereal layers were washed with brine and dried. Purification by flash chromatography (PE/EtOAc 2:1) gave ethyl 2-chloro-5-ethoxy-1-methyl-6-oxo-1,6-dihydropyrimidine-4-carboxylate (85%) as a light yellow oil, which solidified upon standing. $\delta_H$ (400 MHz; DMSO-$d_6$) 1.24 (3H, t, *J* 7.1), 1.29 (3H, t, *J* 7.1), 3.53 (3H, s), 4.14 (2H, q, *J* 7.1), 4.30 (2H, q, *J* 7.1).

Step 5: Ethyl 5-ethoxy-2-(furan-2-yl)-1-methyl-6-oxo-1,6-dihydropyrimidine-4-carboxylate

Ethyl 2-chloro-5-ethoxy-1-methyl-6-oxo-1,6-dihydropyrimidine-4-carboxylate (1 eq), 2-furanboronic acid (1.1 eq), bis(tri-*tert*-butylphosphine)palladium(0) (0.015 eq) and spray-dried potassium fluoride (3.3 eq) were placed in an oven-dried Schlenk tube and purged with argon. Anhydrous 1,4-dioxane (0.3 M solution) was added and the reaction mixture was purged with three vacuum/argon cycles before being heated at 85˚C under an inert atmosphere for 3 h. The cooled reaction mixture was diluted with EtOAc, filtered over a plug of Celite. After evaporation, the residue was purified by flash chromatography (PE/FtOAc 3:1) to give the title compound as an off-white solid. $\delta_H$ (400 MHz; DMSO-$d_6$) 1.25 (3H, t, *J* 7.1), 1.30 (3H, t, *J* 7.1), 3.63 (3H, s), 4.18 (2H, q, *J* 7.1), 4.32 (2H, q, *J* 7.1), 6.75 (1H, dd, *J* 3.5, 1.7), 7.25 (1H, d, *J* 3.5), 7.99 (1H, bs); *m*/*z* ($ES^+$) 293 $(M+H)^+$, 100%.

Step 6: 2-(Furan-2-yl)-5-hydroxy-1-methyl-6-oxo-1,6-dihydropyrimidine-4-carboxylic acid

A solution of ethyl 5-ethoxy-2-(furan-2-yl)-1-methyl-6-oxo-1,6-dihydropyrimidine-4-carboxylate (1 eq) in anhydrous methylene chloride (0.2 M solution) was treated dropwise with a 1 M solution of boron tribromide (5 eq). The reaction mixture was stirred for 1 hour at room temperature. Dichloromethane was evaporated *in vacuo* and the residue stirred in hydrochloric acid (1 N) for 15 min, then diluted with acetonitrile/water and purified by preparative RP-HPLC

(Novapak (Waters) C18 Cartridge Column, 7 micron, 25 x 100 mm; flow: 10 ml/min) to give after lyophilization 2-(furan-2-yl)-5-hydroxy-1-methyl-6-oxo-1,6-dihydropyrimidine-4-carboxylic acid as an off-white solid (37%). $\delta_H$ (400 MHz; DMSO-$d_6$) 3.57 (3H, s), 6.70 (1H, bs), 7.09 (1H, d, *J* 3.2), 7.91 (1H, bs); *m*/*z* (ES-) 235 $(M-H)^-$, 100%.

**EXAMPLE 39**

5-Hydroxy-1-methyl-6-oxo-2-{3-[(*E*)-2-phenylethenyl]furan-2-yl}-1,6-dihydropyrimidine-4-carboxylic acid

Step 1: Ethyl 5-ethoxy-2-(3-formylfuran-2-yl)-1-methyl-6-oxo-1,6-dihydropyrimidine-4-carboxylate

Generally following the procedure described in Example 38, Step 5, the title compound was obtained from 3-formyl-2-furylboronic acid and ethyl 2-chloro-5-ethoxy-1-methyl-6-oxo-1,6-dihydropyrimidine-4-carboxylate (64% after flash chromatography). $\delta_H$ (400 MHz; DMSO-$d_6$) 1.29 (6H, t, *J* 7.2), 3.52 (3H, s), 4.26 (2H, q, *J* 7.2), 4.32 (2H, q, *J* 7.2), 7.03 (1H, d, *J* 2.0), 8.08 (1H, d, *J* 2.0), 10.12 (1H, s); *mlz* ($ES^+$) 321 $(M+H)^+$, 100%.

Step 2: Ethyl 5-ethoxy-1-methyl-6-oxo-2-{3-[(*E)*-2-phenylethenyl]furan-2-yl}-1,6-dihydropyrimidine-4-carboxylate

Sodium hydride (2 eq) was added in one portion to a stirred solution of diethyl benzylphosphonate (2 eq) and ethyl 5-ethoxy-2-(3-formylfuran-2-yl)-1-methyl-6-oxo-1,6-dihydropyrimidine-4-carboxylate (1 eq) in anhydrous 1,2-dimethoxyethane (0.28 M solution). The resulting reaction mixture was refluxed for 24 h, and then poured into water and extracted with ethyl acetate. The combined organic layers were washed with brine, dried over sodium sulfate and evaporated *in vacuo.* Purification by flash chromatography (PE/EtOAc 3:1) gave the title compound as a light yellow solid (20%). $\delta_H$ (400 MHz; DMSO-$d_6$) 1.27-1.31 (6H, m), 3.52 (3H, s), 4.22 (2H, q, *J* 7.2), 4.35 (2H, q, *J* 7.2), 7.21 (1H, d, *J* 1.6), 7.27 (1H, d, *J* 16.8), 7.29 (1H, t, *J* 7.2), 7.38 (2H, t, *J* 7.2), 7.52 (2H, d, *J* 7.2), 7.61 (1H, d, *J* 16.8), 7.95 (1H, d, *J* 1.6); *m*/*z* ($ES^+$) 395 $(M+H)^+$, 100%.

Step 3: 5-Hydroxy-1-methyl-6-oxo-2-{3-[(*E*)-2-phenylethenyl]furan-2-yl}-1,6-dihydropyrimidine-4-carboxylic acid

Generally following the procedure described in Example 38, Step 6, the title compound was obtained from ethyl 5-ethoxy-1-methyl-6-oxo-2-{3-[(*E)*-2-phenylethenyl]furan-2-yl}-1,6-dihydropyrimidine-4-carboxylate, after purification by preparative RP-HPLC (Novapak (Waters) C18 Cartridge Column, 7 micron, 25 x 100 mm; flow: 10 ml/min) and lyophilization, as a colourless solid (37%). $\delta_H$ (400 MHz; DMSO-$d_6$) 3.51 (3H, s), 7.16-7.20 (2H, m), 7.25-7.28 (1H, m), 7.34-7.37 (2H, m), 7.55 (2H, d, *J* 7.6), 7.68 (1H, d, *J* 16.8), 7.89 (1H, s); *m*/*z* ($ES^-$) 337 $(M-H)^-$, 40%.

**EXAMPLE 40**

5-Hydroxy-1-methyl-6-oxo-2-(thien-3-yl)-1,6-dihydropimidine-4-carboxylic acid

The title compound was obtained by reaction of ethyl 2-chloro-5-ethoxy-1-methyl-6-oxo-1,6-dihydropyrimidine-4-carboxylate with 3-thienylboronic acid, as described in Example 38, Step 5 [64%; tetrakis(triphenylphosphine)palladium (0) was used for the Suzuki coupling instead of bis(tri-*tert*-butylphosphine)palladium(0)], followed by deprotection of ethyl 5-ethoxy-1-methyl-6-oxo-2-(thien-3-yl)-1,6-dihydropyrimidine-4-carboxylate as described in Example 38, Step 6. Purification by preparative RP-HPLC (Novapak (Waters) C18 Cartridge Column, 7 micron, 25 x 100 mm; flow: 10 ml/min) gave 5-hydroxy-1-methyl-6-oxo-2-(thien-3-yl)-1,6-dihydropyrimidine-4-carboxylic acid as a colourless powder (60%). $\delta_H$ (400 MHz; DMSO-$d_6$) 3.42 (3H, s), 7.40 (1H, d, *J* 4.8), 7.69 (1H, dd, *J* 4.8, 2.4), 7.97 (1H, d, *J* 2.4); *m*/*z* ($ES^-$) 251 $(M-H)^-$, 100%.

**EXAMPLE 41**

5-Hydroxy-1-methyl-6-oxo-2-[(trimethylsilyl)ethynyl]-1,6-dihydropyrimidine-4-carboxylate

Step 1: Ethyl 5-ethoxy-1-methyl-6-oxo-2-[(trimethylsilyl)ethynyl]-1,6-dihydropyrimidine-4-carboxylate

A solution of ethyl 2-chloro-5-ethoxy-1-methyl-6-oxo-1,6-dihydropyrimidine-4-carboxylate (1 eq), obtained as described in Example 38, trimethylsilylacetylene (1.5 eq), bis(triphenylphosphine)palladium(II) chloride (0.1 eq), triphenylphosphine (0.05 eq) and triethylamine (1.5 eq) in anhydrous THF (0.4 M solution) was stirred for 20 minutes at room temperature under an atmosphere of argon. Copper(I) iodide (0.012 eq) was added, the tube was purged with three vacuum/argon cycles, and the reaction mixture was stirred at room temperature for 3 h. The reaction mixture was diluted

with EtOAc, filtered over a plug of Celite and the filtrate was evaporated *in vacuo.* The residue was purified by flash chromatography (PE/EtOAc 6:1), and ethyl 5-ethoxy-1-methyl-6-oxo-2-[(trimethylsilyl)ethynyl]-1,6-dihydropyrimidine-4-carboxylate (68%) was obtained as an off-white solid. $\delta_H$ (400 MHz; DMSO-$d_6$) 0.29 (9H, s), 1.23 (3H, t, *J* 7.2), 1.28 (3H, t, *J* 7.2), 3.55 (3H, s), 4.19 (2H, q, *J* 7.2), 4.29 (2H, q, *J* 7.2); *m*/*z* ($ES^+$) 323 $(M+H)^+$, 100%.

Step 2: 5-Hydroxy-1-methyl-6-oxo-2-[(trimethylsilyl)ethynyl]-1,6-dihydropyrimidine-4-carboxylate

A solution of ethyl 5-ethoxy-1-methyl-6-oxo-2-[(trimethylsilyl)-ethynyl]-1,6-dihydropyrimidine-4-carboxylate (1 eq) in anhydrous dichloromethane (0.2 M solution) was treated dropwise with a solution of boron tribromide (3.3 eq) in the same solvent (1 M). The reaction mixture was stirred for 30 min at room temperature. Dichloromethane was evaporated *in vacuo* and the residue stirred in hydrochloric acid (1 N) for 15 min, whereupon a precipitate formed. The solid was filtered, washed with hydrochloric acid (1 N), water, pentane and dried *in vacuo* to give the title compound as an off-white solid. $\delta_H$ (400 MHz; DMSO-$d_6$) 0.28 (9H, s), 3.55 (3H, s); *m*/*z* ($ES^-$) 265 $(M-H)^-$, 100%.

## EXAMPLE 42

1-[2-(2-Chlorophenyl)ethyl]-5-hydroxy-6-oxo-2-(thien-2-yl)-1,6-dihydropyrimidine-4-carboxylic acid

Step 1: *N*-Hydroxythiophene-2-carboximidoyl chloride

A DMF solution of thiophene-2-carboxaldehyde oxime (1 eq) was treated dropwise with a solution of NCS (1.05 eq) in DMF. The resulting mixture (0.35 M) was stirred at room temperature for 14 h and then diluted with hydrochloric acid (1 N). After extraction with ethyl acetate the organic layer was dried and concentrated *in vacuo* to give the title compound (95%) as a yellow solid. $\delta_H$ (400 MHz; DMSO-$d_6$) 7.13 (1H, dd, *J* 3.8, 5.1), 7.50 (1H, dd, *J* 1.1, 3.8), 7.68 (1H, dd, *J* 1.1, 5.1), 12.30 (1H, s); *m*/*z* ($ES^+$) 162, 164 $(M+H)^+$.

Step 2: Dimethyl 2-({[(1*Z*)-{[2-(2-chlorophenyl)ethyl)amino}(thien-2-yl)methylidene]amino}oxy)but-2-enedioate

A solution (0.2 M) of *N*-hydroxythiophene-2-carboximidoyl chloride (1 eq) in tetrahydrofuran was treated at 0˚C with a solution (0.5 M) of 2-(2-chlorophenyl)ethylamine (1.05 eq) and $Et_3N$ (1.05 eq). The mixture was stirred at room temperature for 1 h then the solvent was removed *in vacuo.* The residue was taken up with ethyl acetate and aqueous hydrochloric acid (1 N). The organic layer was separated and the aqueous phase was neutralized by addition of a saturated aqueous solution of sodium hydrogencarbonate. Following extraction with ethyl acetate the dried organic layer was concentrated to give a residue that was dissolved in chloroform (0.15 M). Dimethyl acetylenedicarboxylate (1.0 eq) was then added dropwise and the mixture was heated at reflux for 1 h. After cooling the mixture was concentrated *in vacuo* to give the title compound as a mixture of *E*/*Z* isomers on the newly-formed double bond. These were separated by flash chromatography (10% ethyl acetate in petroleum ether):

*Z*-Isomer (7%, yellow oil) $R_f$ = 0.55 (30% ethyl acetate in petroleum ether). $\delta_H$ (400 MHz; DMSO-$d_6$) 2.92 (2H, t, *J* 7.2), 3.50-3.60 (2H, m), 3.59 (3H, s), 3.77 (3H, s), 5.79 (1H, s), 6.88 (1H, t, *J* 5.4), 7.12 (1H, dd, *J* 3.8, 4.9), 7.17-7.31 (4H, m), 7.38 (1H, m), 7.66 (1H, d, *J* 4.9); $\delta_C$ (100 MHz; DMSO-$d_6$) 34.5, 43.3, 51.3, 52.6, 102.2, 127.1, 127.3, 128.2, 128.8, 129.0, 129.1, 130.7, 131.3, 133.0, 136.1, 151.9, 153.5, 162.7, 164.6; *m*/*z* ($ES^+$) 423, 425 $(M+H)^+$.

*E*-Isomer (26%, yellow oil) $R_f$ = 0.45 (30% ethyl acetate in petroleum ether). $\delta_H$ (400 MHz; DMSO-$d_6$) 2.87 (2H, t, *J* 7.0), 3.44 (2H, m), 3.61 (3H, s), 3.81 (3H, s), 5.66 (1H, s), 7.15 (1H, dd, *J* 5.0, 3.7), 7.16-7.39 (6H, m), 7.72 (1H, dd, *J* 5.0, 1.0); $\delta_C$(100 MHz; DMSO-$d_6$) 34.4, 43.3, 51.2, 52.7, 93.4, 127.2, 127.4, 128.3, 129.1, 129.1, 129.7, 129.8, 131.2, 133.0, 135.9, 153.2, 160.9, 162.5, 165.8; *m*/*z* ($ES^+$) 423, 425 $(M+H)^+$.

An additional 12% of an *E*/*Z* mixture was obtained.

Step 3: Methyl 1-[2-(2-chlorophenyl)ethyl]-5-hydroxy-6-oxo-2-(thien-2-yl)-1,6-dihydropyrimidine-4-carboxylate

For the cyclization the mixture of *E*/*Z* isomers can be used with similar results.

Dimethyl (2*E*)-2-({[(1*Z*)-{[2-(2-chlorophenyl)ethyl]amino}(thien-2-yl)methylidene]amino}oxy)but-2-enedioate was dissolved in *p*-xylene (0.12 M) and heated at 150˚C for 8 h. The solvent was removed *in vacuo* and the residue was purified by RP-HPLC (stationary phase: Symmetry $C_{18}$ 19 x 100 mm 5 $\mu$m) to afford the title compound as a white solid (27%). $\delta_H$ (300 MHz; DMSO-$d_6$) 3.00 (2H, t, *J* 7.2), 3.80 (3H, s), 4.33 (2H, t, *J* 7.2), 7.06 (1H, m), 7.14 (1H, t, *J* 4.3), 7.17-7.27 (2H, m), 7.32 (1H, m), 7.39 (1H, d, *J* 3.5), 7.74 (1H, d, *J* 5.2), 10.62 (1H, bs); δc (100 MHz; DMSO-$d_6$) 31.0, 45.6, 52.1, 126.8, 127.1, 127.4, 128.7, 129.1, 129.3, 129.3, 130.9, 133.1, 134.8, 135.1, 143.0, 144.5, 158.9, 165.1; *m*/*z* ($ES^+$) 391, 393 $(M+H)^+$.

Step 4: 1-[2-(2-Chlorophenyl)ethyl]-5-hydroxy-6-oxo-2-(thien-2-yl)-1,6-dihydropyrimidine-4-carboxylic acid

Methyl 1-[2-(2-chlorophenyl)ethyl]-5-hydroxy-6-oxo-2-(thien-2-yl)-1,6-dihydropyrimidine-4-carboxylate was dissolved in a 4:1 mixture of THF/water (0.02 M). Lithium hydroxide (5.0 eq) was added and the mixture was heated at 40°C for 4 h. The cooled mixture was acidified to pH 2 and the white precipitate purified by RP-HPLC (column: Symmetry $C_{18}$ 19 x 100 mm 5 μm) to afford the title compound (80%). δH (300 MHz; DMSO-$d_6$) 2.98 (2H, t, *J* 7.3), 4.30 (2H, t, *J* 7.3), 7.01-7.28 (4H, m), 7.29-7.42 (2H, m), 7.73 (1H, d, *J* 4.7), 10.62 (1H, bs); *m*/*z* ($ES^+$) 377, 379 $(M+H)^+$.

**EXAMPLE 43**

1-Ethyl-5-hydroxy-6-oxo-2-(thien-2-yl)-1,6-dihydropyrimidine-4-carboxylic acid

Following generally the procedures outlined in Example 42, the title compound was obtained from *N*-hydroxythiophene-2-carboximidoyl chloride and ethylamine hydrochloride as a colourless solid. δH (400 MHz; DMSO-$d_6$) 1.24 (3H, t, *J* 7.0), 4.09 (2H, q, *J* 7.0), 7.18 (1H, dd, *J* 3.5, 5.0), 7.50 (1H, d, *J* 3.5), 7.77 (1H, d, *J* 5.0); *m*/*z* ($ES^+$) 267 $(M+H)^+$.

**EXAMPLE 44**

1-Benzyl-5-hydroxy-6-oxo-2-(thien-2-yl)-1,6-dihydropyrimidine-4-carboxylic acid

Following-generally the procedures outlined in Example 42, the title compound was obtained from *N*-hydroxythiophene-2-carboximidoyl chloride and benzylamine as a colourless solid. δH (300 MHz; DMSO-$d_6$) 5.32 (2H, s), 6.97-7-09 (3H, m), 7.14 (1H, d, *J* 3.0), 7.19-7.37 (3H, m), 7.68 (1H, d, *J* 4.8); *m*/*z* ($ES^+$) 329 $(M+H)^+$, 100%.

**EXAMPLE 45**

5-Hydroxy-6-oxo-2-(thien-2-yl)-1-(2,2,2-trifluoroethyl)-1,6-dihydropyrimidine-4-carboxylic acid

Following generally the procedures outlined in Example 42, the title compound was obtained from *N*-hydroxythiophene-2-carboximidoyl chloride and 2,2,2-trifluoroethylamine as a colourless solid. δH (400 MHz; DMSO-$d_6$) 5.02 (2H, q, *J* 9.0), 7.18 (1H, dd, *J* 4.9, 3.6), 7.50 (1H, d, *J* 3.6), 7.80 (1H, d, *J* 4.9); $\delta_F$ (376 MHz; DMSO-$d_6$) -69.04 (t, *J* 9.0); $\delta_c$ (75 MHz; DMSO-$d_6$) 45.3 (q, *J* 35), 123.3 (q, *J* 282), 127.0, 127.3, 129.4, 129.8, 134.7, 142.1, 146.2, 158.6, 167.9; *m*/*z* ($ES^+$) 321 $(M+H)^+$, 100%.

**EXAMPLE 46**

1-(4-Carboxybenzyl)-5-hydroxy-6-oxo-2-(thien-2-yl)-1,6-dihydropyridine-4-carboxylic acid

Following generally the procedures outlined in Example 42, the title compound was obtained from *N*-hydroxythiophene-2-carboximidoyl chloride and methyl 4-(aminomethyl)benzoate hydrochloride as a colourless solid. $\delta_H$ (400 MHz; DMSO-$d_6$) 5.37 (2H, s), 7.03 (1H, dd, *J* 3.5, 5.0), 7.11 (1H, d, *J* 3.5), 7.18 (2H, d, *J* 8.2), 7.68 (1H, d, *J* 5.0), 7.88 (2H, d, *J* 8.2); *mlz* (ES-) 371 $(M-H)^-$, 100%.

**EXAMPLE 47**

5-Hydroxy-1-methyl-2-[3-([2-(1-naphthyl)ethyl]sulfonylamino)thien-2-yl]-6-oxo-1,6-dihydropyrimidine-4-carboxylic acid

Step 1: Methyl 2-(3-aminothien-2-yl)-5-hydroxy-1-methyl-6-oxo-1,6-dihydropyrimidine-4-carboxylate

To a solution (0.02 M) of methyl 5-hydroxy-1-methyl-2-(3-nitrothien-2-yl)-6-oxo-1,6-dihydropyrimidine-4-carboxylate (obtained as described in Example 27, step 3) in MeOH/EtOAc (4:1) was added Pd/C (10% Pd, 10% weight). The suspension was stirred for 4 h under hydrogen, then filtered and concentrated to give the title compound (90%) as a yellow solid. $\delta_H$ (400 MHz; DMSO-$d_6$) 3.58 (3H, s), 3.83 (3H, s), 6.28 (2H, bs), 6.65 (1H, d, *J* 5.6), 7.49 (1H, d, *J* 5.6), 10.18 (1H, s); *m*/*z* ($ES^-$) 280 (M-H); *m*/*z* ($ES^+$) 282 $(M^++H)$.

Step 2: 5-Hydroxy-1-methyl-2-[3-([2-(1-naphthyl)ethyl]sulfonylamino)-thien-2-yl]-6-oxo-1,6-dihydropyrimidine-4-carboxylic acid

2-(1-Naphthyl)ethanesulphonyl chloride (2 eq) was added to a stirred solution of methyl 2-(3-aminothien-2-yl)-5-hydroxy-1-methyl-6-oxo-1,6-dihydropyrimidine-4-carboxylate and NaOH (1 N) (2 eq) in THF (0.16 N). The reaction mixture was stirred at room temperature. After 6 h more sulphonyl chloride (2 eq) and NaOH (1 N) (2 eq) were added. After 24 h a solution of NaOH (1 N) (8 eq) in THF (0.5 N) was added. The reaction mixture was heated to 60˚C for 1 h, and then cooled to room temperature and acidified to pH 2 with hydrochloric acid (1 N). After extraction with EtOAc, the solvent was removed *in vacuo* and the crude purified by preparative RP-HPLC to give after lyophilization the title compound (38%) as a colourless solid. $\delta_H$ (300 MHz; DMSO-$d_6$) 3.39 (3H, s), 3.43 (2H, m), 3.54 (2H, m), 7.29 (1H, d, *J* 5.5), 7.42 (2H, d, *J* 4.8), 7.51 (2H, m), 7.82 (1H, d, *J* 5.5), 7.83 (2H, m), 7.93 (1H, m), 10.34 (1H, s); *m/z* (ES-) 484 (M-H)-, 100%; *m/z* ($ES^+$) 486 $(M+H)^+$, 100%.

**EXAMPLE 48**

5-Hydroxy-1-methyl-6-oxo-2-{3-[({[(2-phenyl-1,3-thiazol-4-yl)methyl]amino}carbonyl)amino]thien-2-yl}-1,6-dihydropyrimidine-4-carboxylic acid

Step 1: Methyl 5-*tert*-butoxy-1-methyl-2-(3-nitrothien-2-yl)-6-oxo-1,6-dihydropyrimidine-4-carboxylate

O-*tert*-Butyl-*N,N'*-diisopropylisourea (5 eq) was added to a solution of methyl 5-hydroxy-1-methyl-2-(3-nitrothien-2-yl)-6-oxo-1,6-dihydropyrimidine-4-carboxylate (obtained as described in Example 27, step 3) in THF (0.4 M). After heating for 10 min at reflux, solvent was evaporated *in vacuo* and the crude purified by flash chromatography (PE/EtOAc 2:3) affording the title compound (71%) as a pale orange solid. $\delta_H$ (400 MHz; DMSO-$d_6$) 1.37 (9H, s), 3.24 (3H, s), 3.81 (3H, s), 7.77 (1H, d, *J* 5.6), 7.99 (1H, d, *J* 5.6); *m/z* ($ES^+$) 368 $(M+H)^+$.

Step 2: Methyl 5-*tert*-butoxy-1-methyl-2-(3-aminothien-2-yl)-6-oxo-1,6-dihydropyrimidine-4-carboxylate

A solution of the foregoing compound in MeOH/EtOAc (3:2) (0.02 M) was cooled at 0˚C under a nitrogen atmosphere and the same amount of 10% wt Pd/C was added. The reaction mixture was left for 3 h at room temperature under $H_2$ atmosphere. The catalyst was filtered off and washed exhaustively with MeOH/EtOAc (3:5). Evaporation of the solvent afforded the title compound (86%) as an orange-yellow solid. $\delta_H$ (400 MHz; DMSO-$d_6$) 1.32 (9H, s), 3.57 (3H, s), 3.82 (3H, s), 6.40 (2H, bs), 6.66 (1H, d, *J* 5.4), 7.57 (1H, d, *J* 5.4); *m/z* ($ES^+$) 338 $(M+H)^+$.

Step 3: Methyl 5-*tert*-butoxy-1-methyl-6-oxo-2-{3-[({[(2-phenyl-1,3-thiazol-4-yl)methyl]amino}carbonyl)amino]thien-2-yl}-1,6-dihydropyrimidine-4-carboxylate

Triphosgene (1.5 eq) was added to a solution of the foregoing compound in dry 1,4-dioxane. The flask was continuously purged with nitrogen and the effluent was bubbled through aqueous KOH solution to destroy excess phosgene. After 15 min (2-phenyl-1,3-thiazol-4-yl)methylamine (3 eq) was added and the mixture was stirred 3 h at room temperature. After dilution with water it was extracted with EtOAc. The collected organic layers were treated with brine and dried on $Na_2SO_4$.

Removal of the solvent afforded the title compound (60%) as a yellow solid. $\delta_H$ (400 MHz; DMSO-$d_6$) 1.34 (9H, s), 3.35 (3H, s), 3.81 (3H, s), 4.39 (2H, d, *J* 5.7), 6.62 (1H, t, *J* 5.7), 7.32 (1H, d, *J* 5.5), 7.40 (1H, s), 7.48 (3H, m), 7.71 (1H, d, *J* 5.5), 7.92 (2H, d, *J* 6.6), 8.99 (1H, s); *m/z* ($ES^+$) 554 $(M+H)^+$.

Step 4: 5-Hydroxy-1-methyl-6-oxo-2-{3-[({[(2-phenyl-1,3-thiazol-4-yl)methyl]amino}carbonyl)amino]thien-2-yl}-1,6-dihydropyrimidine-4-carboxylic acid,

KOH (1 M) (10 eq) was added to a solution of the foregoing compound in MeOH (0.1 M). The resulting yellow solution was stirred at room temperature for 2 h. An excess of hydrochloric acid (3 N) was then added and the mixture was stirred at the same temperature for 2 h more. Purification by RP-HPLC (Conditions: Waters X-TERRA MS C18, 7 micron, 19 x 150 mm; flow: 17 ml/min; gradient: A, $H_2O$ + 0.05% TFA; B, MeCN + 0.05% TFA; 85% A isocratic for 3 min then linear to 35% A in 11 min) gave the title compound (20%) after lyophilization as a white solid. $\delta_H$ (400 MHz; DMSO-$d_6$) 3.35 (3H, s), 4.41 (2H, d, *J* 5.6), 6.78 (1H, t, *J* 5.6), 7.41 (1H, s), 7.47 (3H, m), 7.60 (1H, d, *J* 5.4), 7.66 (1H, d, *J* 5.4), 7.91 (2H, d, *J* 7.5); *m/z* (ES-) 482 (M-H)-.

**EXAMPLE 49**

2-[3-({[(2-Chlorobenzyl)oxy]carbonyl}amino)thien-2-yl]-5-hydroxy-1-methyl-6-oxo-1,6-dihydropyrimidine-4-carboxylic acid

Step 1: *tert*-Butyl 5-*tert*-butoxy-2-[3-({[(2-chlorobenzyl)oxy]carbonyl}-amino)thien-2-yl]-1-methyl-6-oxo-1,6-dihydropyrimidine-4-carboxylate

A solution of triphosgene in dioxane (0.1 M) (1.5 eq) was added to a solution of methyl 5-*tert*-butoxy-1-methyl-2-(3-aminothien-2-yl)-6-oxo-1,6-dihydropyrimidine-4-carboxylate (obtained as described in Example 48, step 2) in dry 1,4-dioxane (0.1 M). After stirring for 15 min at room temperature 2-chlorobenzyl alcohol (2 eq) and triethylamine (2 eq) were added. After stirring the white suspension at 40˚C overnight, the solvent was removed under reduced pressure and the crude purified by flash column chromatography on silica gel (PE/ethyl acetate 3: 1) to give the title compound (42%). $\delta_H$ (400 MHz; DMSO-$d_6$) 1.45 (18H, s), 3.82 (3H, s), 5.33 (2H, s), 7.25 (3H, m), 7.50 (2H, m), 8.01 (1H, d, *J* 5.2), 10.66 (1H, s); *m*/*z* ($ES^+$) 548, 550 $(M+H)^+$.

Step 2: 2-[3-({[(2-Chlorobenzyl)oxy]carbonyl}amino)thien-2-yl]-5-hydroxy-1-methyl-6-oxo-1,6-dihydropyrimidine-4-carboxylic acid

A solution of the foregoing compound in TFA (0.05 M) was stirred at 0˚C for 30 min, and then the solvent was removed under reduced pressure. The oily residue was treated with DCM, and precipitate was isolated by filtration and dried to give the title compound as a pale yellow solid (63%). $\delta_H$ (400 MHz; DMSO-$d_6$) 3.29 (3H, s), 5.19 (2H, s), 7.38 (2H, m), 7.45 (3H, m), 7.73 (1H, d, *J* 5.5), 10.04 (1H, s); *m*/*z* ($ES^+$) 436, 438 $(M+H)^+$.

## Claims

**1.** A compound of formula (I) below, or a pharmaceutically acceptable salt thereof:

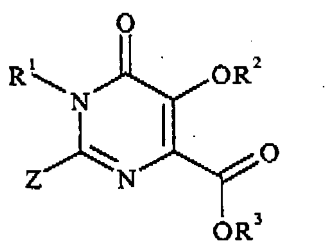

**(I)**

wherein

Z represents $C_{2-6}$ alkynyl, aryl or heteroaryl, any of which groups may be optionally substituted;
$R^1$ represents $C_{1-6}$ alkyl or aryl$(C_{1-6})$alkyl, either of which groups may be optionally substituted;
$R^2$ represents hydrogen; or $C_{1-6}$ alkyl, $C_{2-6}$ alkylearbanyl, aryl, arylcarbonyl, heteroa-ryl, aryl$(C_{1-6})$alkyl or heteroaryl$(C_{1-6})$alkyl, any of which groups may be optionally substituted; and
$R^3$ represents hydrogen, $C_{1-6}$ alkyl, $C_{3-7}$ heterocycloalkyl$(C_{1-6})$alkyl, di$(C_{1-6})$alkylamino$(C_{1-6})$alkyl, $C_{2-6}$ alkylcarbonyloxy$(C_{1-6})$alkyl or $C_{3-7}$ cycloalkoxycarbonyloxy$(C_{1-6})$alkyl;

for use in therapy.

**2.** A compound of formula (I) as defined in claim 1, or a pharmaceutically acceptable salt thereof; provided that, when Z is unsubstituted phenyl, then $R^1$, $R^2$ and $R^3$ do not each simultaneously represent methyl.

**3.** A compound as claimed in claim 2 represented by formula (III) below:

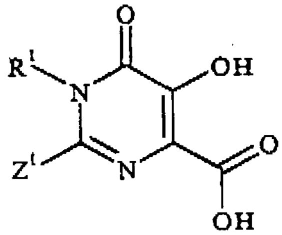

(III)

wherein

$Z^1$ represents optionally substituted aryl; and
$R^1$ is as defined in claim 1.

**4.** A compound as claimed in claim 3 represented by formula (IV):

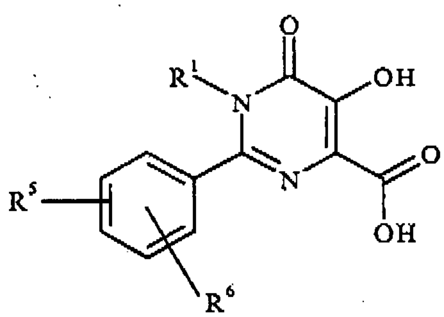

(IV)

wherein

$R^1$ is as defined in claim 1; and
$R^5$ and $R^6$ are each independently selected from hydrogen and a substituent group of formula (II):

$-X-R^4$ (II)

in which
X is selected from $-NH-SO_2-$, $-NH-SO_2-NH-$, $-CH_2-SO_2-$, $-SO_2-NH--NH-CO-NH-$, -NH-CS-NH-, -NH-CO-O-, -NH-CO-, -CO-NH-, $-NH-CO-NH-SO_2-$ -NH-CO-NH-CO-, -O-, -S-, -SO-, $-SO_2-$ -NH-, $-CH_2-$, $-CH_2O-$ and $-CH_2S-$; and
$R^4$ represents aryl, aryl($C_{1-6}$)alkyl, $C_{3-7}$ cycloalkyl, $C_{1-6}$ alkyl, heteroaryl($C_{1-6}$)alkyl, $C_{3-7}$ heterocycloalkyl or $C_{2-6}$ alkenyl, any of which groups may be optionally substituted.

**5.** A compound as claimed in claim 2 represented by formula (X) below:

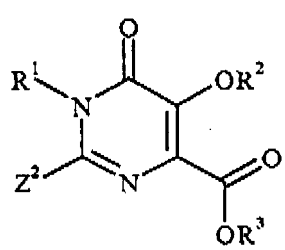

(X)

wherein

$Z^2$ represents optionally substituted heteroaryl; and

$R^1$, $R^2$ and $R^3$ are as defined in claim 1.

**6.** A compound as claimed in claim 5 represented by formula (XI) below:

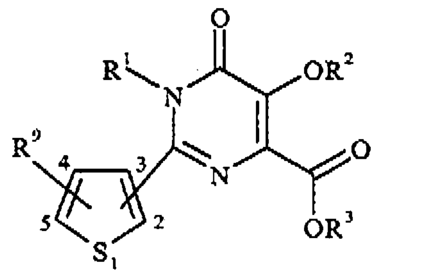

(XI)

wherein

$R^9$ represents hydrogen or a group of formula (II) as defined in claim 4.

**7.** A compound selected from Example Nos. 1 to 46,
2-[3-({[(2-Chlorobenzyl)amino]carbonyl} amino)thien-2-yl]-5-hydroxy-1-methyl-6-oxo-1,6-dihydropyrimidine-4-carboxylic acid;
5-Hydroxy-1-methyl-6-oxo-2-(thien-2-yl)-1,6-dihydropyrimidine-4-carboxylic acid;
5-Hydroxy-1-methyl-6-oxo-2-(thiazol-2-yl)-1,6-dihydropyrimidine-4-carboxylic acid;
5-Hydroxy-1-methyl-2-(3-nitrothien-2-yl)-6-oxo-1,6-dihydropyrimidine-4-carboxylic acid;
5-Hydroxy-1-methyl-2-(3-aminothien-2-yl)-6-oxo-1,6-dihydropyrimidine-4-carboxylic acid hydrochloride salt;
5-Hydroxy-1-methyl-2-(3-bromothien-2-yl)-6-oxo-1,6-dihydropyrimidine-4-carboxylic acid;
2-[3-(Acctylamino)thien-2-yl]-5-hydroxy-1-methyl-6-oxo-1,6-dihydropyrimidine-4-carboxylic acid;
2-[3-(Benzoylamino)thien-2-yl]-5-hydroxy-1-methyl-6-oxo-1,6-dihydropyrimidine-4-carboxylic acid;
5-Hydroxy-1-methyl-2-(3-{[(2-methyl-1*H*-indol-3-yl)acetyl]amino}thien-2-yl)-6-oxo-1,6-dihydropyrimidine-4-carboxylic acid;
2-(3-{[3-(2-Chlorophenyl)propanoyl]amino}thien-2-yl)-5-hydroxy-1-methyl-6-oxo-1,6-dihydropyrimidine-4-carboxylic acid;
2- {3-[(Anilinocarbonyl)amino]thien-2-yl}-5-hydroxy-1-methyl-6-oxo-1,6-dihydropyrimidine-4-carboxylic acid;
2-(3- {[(1,1'-Biphenyl-2-ylamino)carbonyl]amino}thien-2-yl)-5-hydroxy-1-methyl-6-oxo-1,6-dihydropyrimidine-4-carboxylic acid;
2-(3-{[(Benzhydrylamino)carbonyl]amino}thien-2-yl)-5-hydroxy-1-methyl-6-oxo-1,6-dihydropyrimidine-4-carboxylic acid;
5-Hydroxy-1-methyl-2-{3-[({[1-(1-naphthyl)ethyl]amino}-carbonyl)amino]thien-2-yl}-6-oxo-1,6-dihydropyrimidine-4-carboxylic acid;
5-Hydroxy-1-methyl-6-oxo-2-[3-({[(2-phenylcyclopropyl)amino]-carbonyl}amino)thien-2-yl]-1,6-dihydropyrimidine-4-carboxylic acid;
5-Hydroxy-1-methyl-6-oxo-2-[3-({(([(2-phenylethyl)amino]-carbonyl}amino)thien-2-yl]-1,6-dihydropyrimidine-4-carboxylic acid;
5-Hydroxy-2-{3-[(isobutoxycarbonyl)amino]thien-2-yl}-1-methyl-6-oxo-1,6-dihydropyrimidine-4-carboxylic acid;
*N*[1]-Benzyl-*N*[2]-[2-(4-carboxy-5-hydroxy-1-methyl-6-oxo-1,6-dihydropyrimidin-2-yl)thiophen-3-yl]glycinamide;
5-Hydroxy-1-methyl-6-oxo-2-(3- {[(2*E*)-3-phenylprop-2-enyl] amino}thien-2-yl)-1,6-dihydropyrimidine-4-carboxylic acid;
2-(4-Carboxy-5-hydroxy-1-methyl-6-oxo-1,6-dihydropyrimidin-2-yl)-*N*-(3-phenylpropyl)thiophen-3-aminium trifluoroacetate;
2-{3-[2-(Benzyloxy)ethyl]thien-2-yl}-5-hydroxy-1-methyl-6-oxo-1,6-dihydropyrimidine-4-carboxylic acid;
2- {4-[({[(2-Chlorophenyl)sulfonyl]amino}carbonyl)amino]thien-3-yl}-5-hydroxy-1-methyl-6-oxo-1,6-dihydropyrimidine-4-carboxylic acid;
2- {3-[({[(2-Chlorophenyl)sulfonyl] amino} carbonyl)amino]thien-2-yl}-5-hydroxy-1-methyl-6-oxo-1,6-dihydropyrimidine-4-carboxylic acid;
5-Hydroxy-2-(3-hydroxyphenyl)-1-methyl-6-oxo-1,6-dihydropyrimidine-4-carboxylic acid;
2-(3-{[(1,1'-Biphenyl-2-ylamino)carbonyl]amino}phenyl)-5-hydroxy-1-methyl-6-oxo-1,6-dihydropyrimidine-4-carboxylic acid;

2-[3-({[(3-Carboxyphenyl)amino]carbonyl} amino)phenyl]-5-hydroxy-1-methyl-6-oxo-1,6-dihydropyrimidine-4-carboxylic acid;
2-{3-[(Benzylsulfonyl)amino] thien-2-yl}-5-hydroxy-1-methyl-6-oxo-1,6-dihydropyrimidine-4, carboxylic acid;
5-Hydroxy-1-methyl-2-{3-[(2-naphthylsulphonyl)amino]thien-2-yl} -6-oxy-1,6-dihydropyrimidine-4-carboxylic acid;
2-(3-Formylthien-2-yl)-5-hydroxy-1-methyl-6-oxo-1,6-dihydropyrimidine-4-carboxylic acid;
2-(3-Carboxythien-2-yl)-5-hydroxy-1-methyl-6-oxo-1,6-dihydropyrimidine-4-carboxylic acid;
2-[3-({[2-(2-Chlorophenyl)ethyl]amino}carbonyl)thien-2-yl]-5-hydroxy-1-methyl-6-oxo-1,6-dihydropyrimidine-4-carboxylic acid;
5-Hydroxy-1-methyl-6-oxo-2-{3-[(E)-2-phenylethenyl]thien-2-yl}-1,6-dihydropyrimidine-4-carboxylic acid;
5-Hydroxy-1-methyl-6-oxo-2-[3-(2-phenylethyl)thien-2-yl]-1,6-dihydropyrimidine-4-carboxylic acid;
2-{3-[(1E)-4-(2-Chlorophenyl)but-1-enyl]thien-2-yl}-5-hydroxy-1-methyl-6-oxo-1,6-dihydropyrimidine-4-carboxylic acid;
5-Hydroxy-1-methyl-6-oxo-2-[3-(4-phenylbutyl)thien-2-yl]-1,6-dihydropyrimidine-4-carboxylic acid;
5-Hydroxy-2-{3-[(4-methoxybenzyl)oxy]phenyl}-1-methyl-6-oxo-1,6-dihydropyrimidine-4-carboxylic acid;
2- {2-[(3,4-Dichlorobenzyl)oxy]phenyl} -5-hydroxy-1-methyl-6-oxo-1,6-dihydropyrimidine-4-carboxylic acid;
2-(Furan-2-yl)-5-hydroxy-1-methyl-6-oxo-1,6-dihydropyrimidine-4-carboxylic acid;
5-Hydroxy-1-methyl-6-oxo-2-{3-[(E)-2-phenylethenyl]furan-2-yl}-1,6-dihydropyrimidine-4-carboxylic acid;
5-Hydroxy-1-methyl-6-oxo-2-(thien-3-yl)-1,6-dihydropyrimidine-4-carboxylic acid;
5-Hydroxy-1-methyl-6-oxo-2-[(trimethylsilyl)ethynyl] 1,6-dihydropyrimidine-4-carboxylate;
1-[2-(2-Chlorophenyl)ethyl]-5-hydroxy-6-oxo-2-(thien-2-yl)-1,6-dihydropyrimidine-4-carboxylic acid;
1-Ethyl-5-hydroxy-6-oxo-2-(thien-2-yl)-1,6-dihydropyrimidine-4-carboxylic acid;
1-Benzyl-5-hydroxy-6-oxo-2-(thien-2-yl)-1,6-dihydropyrimidine-4-carboxylic acid;
5-Hydroxy-6-oxo-2-(thien-2-yl)-1-(2,2,2-trifluoroethyl)-1,6-dihydropyrinidine-4-carboxylic acid;
1-(4-Carboxybenzyl)-5-hydroxy-6-oxo-2-(thien-2-yl)-1;6-dihydropyridine-4-carboxylic acid;
or a pharmaceutically acceptable salt thereof.

**8.** A compound selected from Example Nos. 47 to 49,
5-Hydroxy-1-methyl-2-[3-([2-(1-naphthyl)ethyl]sulfonylamino)thien-2-yl]-6-oxo-1,6-dihydropyrimidine-4-carboxylic acid;
5-Hydroxy-1-methyl-6-oxo-2-{3-[({[(2-phenyl-1,3-thiazol-4-yl)methyl]amino}carbonyl)amino]thien-2-yl}-1,6-dihydropyrimidine-4-carboxylic acid;
2-[3-({[(2-Chlorobenzyl)oxy]carbonyl}amino)thien-2-yl]-5-hydroxy-1-methyl-6-oxo-1,6-dihydropyrimidine-4-carboxylic acid;
or a pharmaceutically acceptable salt thereof.

**9.** A pharmaceutical composition comprising a compound of formula (I) as defined in claim 1, or a pharmaceutically acceptable salt thereof, in association with a pharmaceutically acceptable carrier.

**10.** The use of a compound of formula (I) as defined in claim 1, or a pharmaceutically acceptable salt thereof, for the manufacture of a medicament for treatment or prevention of infection by hepatitis C virus.

**11.** A process for the preparation of a compound of formula (I) as defined in claim 1, which comprises:

(A) reacting a compound of formula (XIV) with a compound of formula (XV):

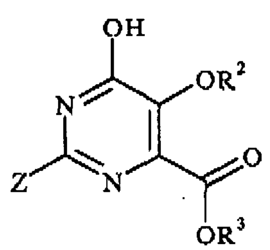

(XIV)

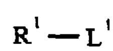

(XV)

wherein Z, $R^1$, $R^2$ and $R^3$ are as defined in claim 1, and $L^1$ represents a suitable leaving group; or (B) reacting a compound of formula (XVIII) with a compound of formula (XIX):

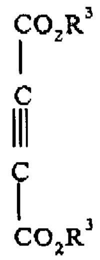

(XVIII)

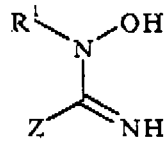

(XIX)

wherein Z, $R^1$ and $R^3$ are as defined in claim 1; followed by cyclisation of the intermediate thereby obtained; or (C) reacting a compound of formula (XVIII) as defined above with a compound of formula (XX):

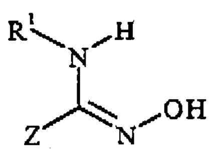

(XX)

wherein Z and $R^1$ are as defined in claim 1; followed by cyclisation of the intermediate thereby obtained; or (D) reacting a compound of formula Z-$B(OH)_2$ with a compound of formula (XXII):

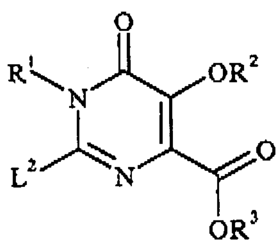

(XXII)

wherein Z, $R^1$, $R^2$ and $R^3$ are as defined in claim 1, and $L^2$ represents a suitable leaving group; in the presence of a transition metal catalyst; and

(E) subsequently, if required, converting a compound of formula (I) initially obtained into a further compound of formula (I) by standard methods.

**12.** A compound as claimed in any one of claims 1 to 8 for use in therapy.

**Patentansprüche**

**1.** Eine Verbindung der nachstehenden Formel (I) oder ein pharmazeutisch annehmbares Salz davon:

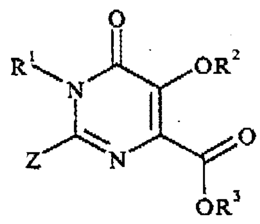

(I)

,

wobei

Z $C_{2-6}$-Alkinyl, Aryl oder Heteroaryl bedeutet, wobei jede dieser Gruppen gegebenenfalls substituiert sein kann,
$R^1$ $C_{1-6}$-Alkyl oder Aryl($C_{1-6}$)alkyl bedeutet, wobei jede dieser Gruppen gegebenenfalls substituiert sein kann,
$R^2$ Wasserstoff bedeutet, oder $C_{1-6}$-Alkyl, $C_{2-6}$-Alkylcarbonyl, Aryl, Arylcarbonyl, Heteroaryl, Aryl($C_{1-6}$)alkyl oder Heteroaryl($C_{1-6}$)alkyl, wobei jede dieser Gruppen gegebenenfalls substituiert sein kann, und
$R^3$ Wasserstoff, $C_{1-6}$-Alkyl, $C_{3-7}$-Heterocycloalkyl($C_{1-6}$)alkyl, Di($C_{1-6}$)alkylamino($C_{1-6}$)alkyl, $C_{2-6}$-Alkylcarbonyloxy($C_{1-6}$)alkyl oder $C_{3-7}$-Cycloalkoxycarbonyloxy($C_{1-6}$)alkyl bedeutet,

zur Verwendung in der Therapie.

**2.** Eine wie in Anspruch 1 definierte Verbindung der Formel (I) oder ein pharmazeutisch annehmbares Salz davon, mit der Maßgabe, dass, wenn Z unsubstituiertes Phenyl ist, $R^1$, $R^2$ und $R^3$ dann nicht gleichzeitig Methyl bedeuten.

**3.** Eine wie in Anspruch 2 beanspruchte Verbindung, dargestellt durch die nachstehende Formel (III):

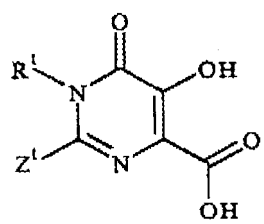

(III)

,

wobei

$Z^1$ gegebenenfalls substituiertes Aryl bedeutet und
$R^1$ wie in Anspruch 1 definiert ist.

**4.** Eine wie in Anspruch 3 beanspruchte Verbindung, dargestellt durch Formel (IV):

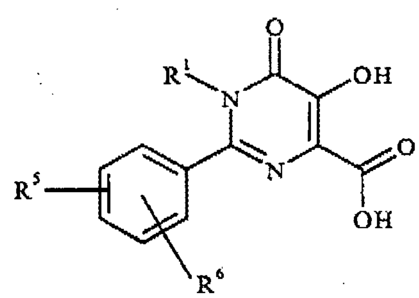

(IV)

,

wobei

$R^1$ wie in Anspruch 1 definiert ist und

$R^5$ und $R^6$ jeweils unabhängig ausgewählt sind aus Wasserstoff und einer Substituentengruppe der Formel (II):

$$-X-R^4 \qquad (II),$$

wobei
X ausgewählt ist aus $-NH-SO_2-$, $-NH-SO_2-NH-$, $-CH_2-SO_2-$, $-SO_2-NH-$, -NH-CO-NH-, -NH-CS-NH-, -NH-CO-O-, NH-CO-, -CO-NH-, $-NH-CO-NH-SO_2-$, -NH-CO-NH-CO-, -O-, -S-, -SO-, $-SO_2-$, -NH-, $-CH_2-$, $-CH_2O-$ und $-CH_2S-$, und
$R^4$ Aryl, Aryl($C_{1-6}$)alkyl, $C_{3-7}$-Cycloalkyl, $C_{1-6}$-Alkyl, Heteroaryl($C_{1-6}$)alkyl, $C_{3-7}$-Heterocycloalkyl oder $C_{2-6}$-Alkenyl bedeutet, wobei jede dieser Gruppen gegebenenfalls substituiert sein kann.

**5.** Eine wie in Anspruch 2 beanspruchte Verbindung, dargestellt durch die nachstehende Formel (X):

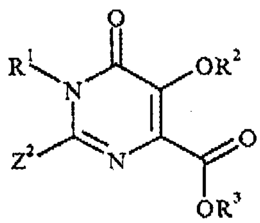

(X)

,

wobei

$Z^2$ gegebenenfalls substituiertes Heteroaryl bedeutet und
$R^1$, $R^2$ und $R^3$ wie in Anspruch 1 definiert sind.

**6.** Eine wie in Anspruch 5 beanspruchte Verbindung, dargestellt durch die nachstehende Formel (XI):

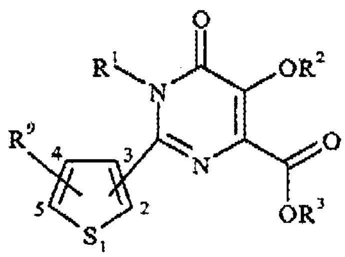

(XI)

,

wobei

$R^9$ Wasserstoff oder eine wie in Anspruch 4 definierte Gruppe der Formel (II) bedeutet.

**7.** Eine Verbindung, ausgewählt aus den Beispielen Nr. 1 bis 46,
2-[3-({[(2-Chlorbenzyl)amino]carbonyl} amino)thien-2-yl]-5-hydroxy-1-methyl-6-oxo-1,6-dihydropyrimidin-4-carbonsäure,
5-Hydroxy-1-methyl-6-oxo-2-(thien-2-yl)-1,6-dihydropyrimidin-4-carbonsäure,
5-Hydroxy-1-methyl-6-oxo-2-(thiazol-2-yl)-1,6-dihydropyrimidin-4-carbonsäure,
5-Hydroxy-1-methyl-2-(3-nitrothien-2-yl)-6-oxo-1,6-dihydropyrimidin-4-carbonsäure,
5-Hydroxy-1-methyl-2-(3-aminothien-2-yl)-6-oxo-1,6-dihydropyrimidin-4-carbonsäure-Hydrochloridsalz,
5-Hydroxy-1 -methyl-2-(3-bromthien-2-yl)-6-oxo-1,6-dihydropyrimidin-4-carbonsäure,
2-[3-(Acetylamino)thien-2-yl]-5-hydroxy-1-methyl-6-oxo-1,6-dihydropyrimidin-4-carbonsäure,
2-[3-(Benzoylamino)thien-2-yl]-5-hydroxy-1-methyl-6-oxo-1,6-dihydropyrimidin-4-carbonsäure,
5-Hydroxy-1-methyl-2-(3-{[(2-methyl-1*H*-indol-3-yl)acetyl]amino}thien-2-yl)-6-oxo-1,6-dihydropyrimidin-4-carbonsäure,
2-(3-{[3-(2-Chlorphenyl)propanoyl]amino}thien-2-yl)-5-hydroxy-1-methyl-6-oxo-1,6-dihydropyrimidin-4-carbonsäure,
2-{3-[(Anilinocarbonyl)amino]thien-2-yl}-5-hydroxy-1-methyl-6-oxo-1,6-dihydropyrimidin-4-carbonsäure,
2-(3-{[(1,1'-Biphenyl-2-ylamino)carbonyl]amino}thien-2-yl)-5-hydroxy-1-methyl-6-oxo-1,6-dihydropyrimidin-4-carbonsäure,
2-(3-{[(Benzhydrylamino)carbonyl]amino}thien-2-yl)-5-hydroxy-1-methyl-6-oxo-1,6-dihydropyrimidin-4-carbonsäure,
5-Hydroxy-1-methyl-2-{3-[({[1-(1-naphthyl)ethyl]amino}-carbonyl)amino]thien-2-yl}-6-oxo-1,6-dihydropyrimidin-4-carbonsäure,
5-Hydroxy-1-methyl-6-oxo-2-[3-({[(2-phenylcyclopropyl)amino]-carbonyl}amino)thien-2-yl]-1,6-dihydropyrimidin-4-carbonsäure,
5-Hydroxy-1-methyl-6-oxo-2-[3-({[(2-phenylethyl)amino]carbonyl} amino)thien-2-yl]-1,6-dihydropyrimidin-4-carbonsäure,
5-Hydroxy-2-{3-[(isobutoxycarbonyl)amino]thien-2-yl}-1-methyl-6-oxo-1,6-dihydropyrimidin-4-carbonsäure,
*N*$^1$-Benzyl-*N*$^2$-[2-(4-carboxy-5-hydroxy-1-methyl-6-oxo-1,6-dihydropyrimidin-2-yl)thiophen-3-yl]glycinamid,
5-Hydroxy-1-methyl-6-oxo-2-(3-{[(2*E*)-3-phenylprop-2-enyl]amino}thien-2-yl)-1,6-dihydropyrimidin-4-carbonsäure,
2-(4- Carboxy- 5- hydroxy- 1- methyl- 6- oxo- 1,6- dihydropyrimidin- 2- yl)-*N*-(3- phenylpropyl) thiophen- 3- aminiumtrifluoracetat,
2-{3-[2-(Benzyloxy)ethyl]thien-2-yl}-5-hydroxy-1-methyl-6-oxo-1,6-dihydropyrimidin-4-carbonsäure,
2-{4-[({[(2-Chlorphenyl)sulfonyl]amino}carbonyl)amino]thien-3-yl}-5-hydroxy-1-methyl-6-oxo-1,6-dihydropyrimidin-4-carbonsäure,
2-{3-[({[(2-Chlorphenyl)sulfonyl]amino}carbonyl)amino]thien-2-yl}-5-hydroxy-1-methyl-6-oxo-1,6-dihydropyrimidin-4-carbonsäure,
5-Hydroxy-2-(3-hydroxyphenyl)-1-methyl-6-oxo-1,6-dihydropyrimidin-4-carbonsäure,
2-(3-{[(1,1'-Biphenyl-2-ylamino)carbonyl]amino}phenyl)-5-hydroxy-1-methyl-6-oxo-1,6-dihydropyrimidin-4-carbonsäure,
2-[3-({[(3-Carboxyphenyl)amino]carbonyl} amino)phenyl]-5-hydroxy-1-methyl-6-oxo-1,6-dihydropyrimidin-4-carbonsäure,
2-{3-[(Benzylsulfonyl)amino]thien-2-yl}-5-hydroxy-1-methyl-6-oxo-1,6-dihydropyrimidin-4-carbonsäure,
5-Hydroxy-1-methyl-2-{3-[(2-naphthylsulfonyl)amino]thien-2-yl}-6-oxo-1,6-dihydropyrimidin-4-carbonsäure,
2-(3-Formylthien-2-yl)-5-hydroxy-1-methyl-6-oxo-1,6-dihydropyrimidin-4-carbonsäure,
2-(3-Carboxythien-2-yl)-5-hydroxy-1-methyl-6-oxo-1,6-dihydropyrimidin-4-carbonsäure,
2-[3-({2-([2-Chlorphenyl)ethyl]amino}carbonyl)thien-2-yl]-5-hydroxy-1-methyl-6-oxo-1,6-dihydropyrimidin-4-carbonsäure,
5-Hydroxy-1-methyl-6-oxo-2-{3-[(*E*)-2-phenylethenyl]thien-2-yl}-1,6-dihydropyrimidin-4-carbonsäure,
5-Hydroxy-1-methyl-6-oxo-2-[3-(2-phenylethyl)thien-2-yl]-1,6-dihydropyrimidin-4-carbonsäure,
2-{3-[(1*E*)-4-(2-Chlorphenyl)but-1-enyl]thien-2-yl}-5-hydroxy-1-methyl-6-oxo-1,6-dihydropyrimidin-4-carbonsäure,
5-Hydroxy-1 -methyl-6-oxo-2-[3-(4-phenylbutyl)thien-2-yl]-1,6-dihydropyrimidin-4-carbonsäure,
5-Hydroxy-2-{3-[(4-methoxybenzyl)oxy]phenyl}-1-methyl-6-oxo-1,6-dihydropyrimidin-4-carbonsäure,
2-{2-[(3,4-Dichlorbenzyl)oxy]phenyl}-5-hydroxy-1-methyl-6-oxo-1,6-dihydropyrimidin-4-carbonsäure,
2-(Furan-2-yl)-5-hydroxy-1-methyl-6-oxo-1,6-dihydropyrimidin-4-carbonsäure,
5-Hydroxy-1-methyl-6-oxo-2-{3-[(*E*)-2-phenylethenyl]furan-2-yl}-1,6-dihydropyrimidin-4-carbonsäure,
5-Hydroxy-1-methyl-6-oxo-2-(thien-3-yl)-1,6-dihydropyrimidin-4-carbonsäure,
5-Hydroxy-1-methyl-6-oxo-2-[(trimethylsilyl)ethinyl]-1,6-dihydropyrimidin-4-carboxylat,
1-[2-(2-Chlorphenyl)ethyl]-5-hydroxy-6-oxo-2-(thien-2-yl)-1,6-dihydropyrimidin-4-carbonsäure,
1-Ethyl-5-hydroxy-6-oxo-2-(thien-2-yl)-1,6-dihydropyrimidin-4-carbonsäure,
1-Benzyl-5-hydroxy-6-oxo-2-(thien-2-yl)-1,6-dihydropyrimidin-4-carbonsäure,
5-Hydroxy-6-oxo-2-(thien-2-yl)-1-(2,2,2-trifluorethyl)-1,6-dihydropyrimidin-4-carbonsäure,
1-(4-Carboxybenzyl)-5-hydroxy-6-oxo-2-(thien-2-yl)-1,6-dihydropyridin-4-carbonsäure,
oder ein pharmazeutisch annehmbares Salz davon.

**8.** Eine Verbindung, ausgewählt aus den Beispielen Nr. 47 bis 49,
5-Hydroxy-1-methyl-2-[3-([2-(1-naphthyl)ethyl]sulfonylamino)thien-2-yl]-6-oxo-1,6-dihydropyrimidin-4-carbonsäure,
5-Hydroxy-1-methyl-6-oxo-2-{3-[({[(2-phenyl-1,3-thiazol-4-yl)methyl]amino}carbonyl)amino]thien-2-yl}-1,6-dihydropyrimidin-4-carbonsäure,
2-[3-({[(2-Chlorbenzyl)oxy]carbonyl}amino)thien-2-yl]-5-hydroxy-1-methyl-6-oxo-1,6-dihydropyrimidin-4-carbonsäure,
oder ein pharmazeutisch annehmbares Salz davon.

**9.** Eine pharmazeutische Zusammensetzung, die eine wie in Anspruch 1 definierte Verbindung der Formel (I) oder ein pharmazeutisch annehmbares Salz davon in Verbindung mit einem pharmazeutisch annehmbaren Träger enthält.

**10.** Die Verwendung einer wie in Anspruch 1 definierten Verbindung der Formel (I) oder eines pharmazeutisch annehmbaren Salzes davon zur Herstellung eines Medikaments zur Behandlung oder Prävention einer Infektion durch das Hepatitis-C-Virus.

**11.** Ein Verfahren zur Herstellung einer wie in Anspruch 1 definierten Verbindung der Formel (I), umfassend:

(A) Umsetzung einer Verbindung der Formel (XIV) mit einer Verbindung der Formel (XV):

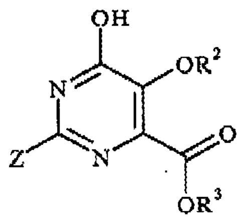

(XIV)

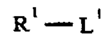

(XV)

,

wobei Z, $R^1$, $R^2$ und $R^3$ wie in Anspruch 1 definiert sind und $L^1$ eine geeignete Abgangsgruppe bedeutet, oder
(B) Umsetzung einer Verbindung der Formel (XVIII) mit einer Verbindung der Formel (XIX):

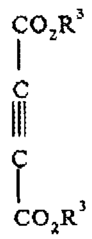

(XVIII)

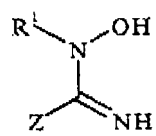

(XIX)

,

wobei Z, $R^1$ und $R^3$ wie in Anspruch 1 definiert sind, gefolgt von der Cyclisierung des dabei erhaltenen Zwischenprodukts, oder
(C) Umsetzung einer wie oben definierten Verbindung der Formel (XVIII) mit einer Verbindung der Formel (XX):

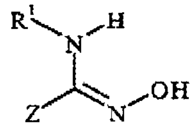

(XX)

,

wobei Z und $R^1$ wie in Anspruch 1 definiert sind, gefolgt von der Cyclisierung des dabei erhaltenen Zwischenprodukts, oder
(D) Umsetzung einer Verbindung der Formel Z-$B(OH)_2$ mit einer Verbindung der Formel (XXII):

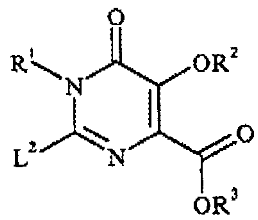

(XXII)

,

wobei Z, $R^1$, $R^2$ und $R^3$ wie in Anspruch 1 definiert sind und $L^2$ eine geeignete Abgangsgruppe bedeutet, in Gegenwart eines Übergangsmetallkatalysators, und

(E) anschließend, falls erforderlich, Umwandlung einer ursprünglich erhaltenen Verbindung der Formel (I) in eine weitere Verbindung der Formel (I) durch Standardverfahren.

**12.** Eine wie in irgendeinem der Ansprüche 1 bis 8 beanspruchte Verbindung zur Verwendung in der Therapie.

**Revendications**

**1.** Composé de la formule (1) ci-dessous ou un sel pharmaceutiquement acceptable de celui-ci:

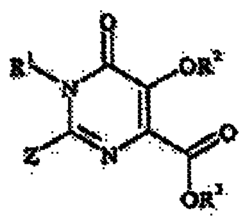

(I)

où

Z représente un alcynyle $C_{2-6}$ un aryle ou un hétéroaryle, dont l'un quelconque de ces groupes peut être optionnellement substitué;

$R^1$ représente un alkyle $C_{1-6}$ ou un arylalkyle ($C_{1-6}$), dont l'un ou l'autre de ces groupes peut être optionnellement substitué;

$R^2$ représente un hydrogène; ou un alkyle $C_{1-6}$, un alkyle-$C_{2-6}$-carbonyle, un aryle, un arylcarbonyle, un hétéroaryle, un arylalkyle($C_{1-6)}$ ou un hétéroarylalkyle($C_{1-6}$), dont l'un quelconque de ces groupes peut être optionnellement substitué; et

$R^3$ représente un hydrogène un alkyle $C_{1-6}$ un hétérocycloalkyle-$C_{3-7}$-alkyle($C_{1-6}$), un dialkyle($C_1$-$_6$)aminoalkyle ($C_{1-6}$), un alkyle-$C_{2-6}$-carbonyloxyalkyle ($C_{1-6}$) ou un cycloalcoxy-$C_{3-7}$carbonyloxyalkyle($C_1$-6);

à utiliser en thérapie.

**2.** Composé de la formule (I) tel que défini dans la revendication 1 ou un sel pharmaceutiquement acceptable de celui-ci; à condition que, lorsque Z est un phényle non substitué, alors $R^1$, $R^2$ et $R^3$ ne représentent pas simultanément chacun un méthyle.

**3.** Composé tel que revendiqué dans la revendication 2 représenté par la formule (III) ci-dessous:

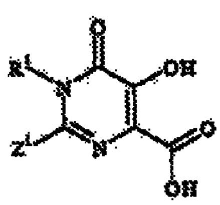

(III)

où

$Z^1$ représente un aryle optionnellement substitué; et
$R^1$ est tel que défini dans la revendication 1.

**4.** Composé tel que revendiqué dans la revendication 3 représenté par la formule (IV).

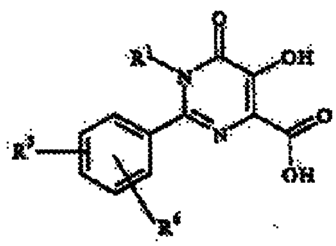

(IV)

où

$R^1$ est tel que défini dans la revendication 1 ; et.
$R^5$ et $R^6$ sont sélectionnés indépendamment chacun parmi un hydrogène et un groupe substitué de la formule (II);

-X-$R^4$ (II)

où
X est sélectionné parmi -NH-$SO_2$-, -NH-$SO_2$-NH-,-$CH_2$-$SO_2$-, -$SO_2$-NH-,-NH-CO-NH-, -NH-CS NH-, -NH-CO-O-,NH-CO-, -CO-NH-, -NH-CO-NH-$SO_2$-, -NH-CO-NH-CO, -O-, -S-, -SO-, -$SO_2$-, NH, -$CH_2$-, $CH_2$O- et -$CH_2$S-, et
$R^4$ représente un style, un arylalkyle($C_{1-6}$), un cycloalkyle, $C_{3-7}$ un alkyle $C_{1-6}$ un hétéroalkyle($C_{1-6}$), un hétérocycloalkyle $C_{3-7}$ ou un alcényle $C_{2-6}$ dont l'un quelconque de ces groupes peut être optionnellement substitué:

**5.** Composé tel que revendiqué dans la revendication 2 représenté par la formule (X) ci-dessous;

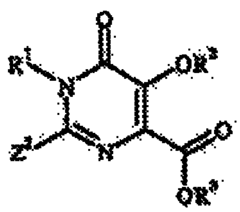

(X)

où

$Z^2$ représente un hétéroaryle optionnellement substitué; et
$R^1$, $R^2$ et $R^3$ sont tels que définis dans la revendication 1,

**6.** Composé tel que revendiqué dans la revendication 5 représenté par la formule (XI) ci-dessous:

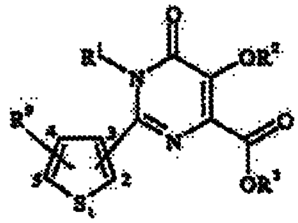

(XI)

où

$R^9$ représente un hydrogène ou un groupe de la formule (II) tel que défini dans la revendication 4,

**7.** Composé sélectionné parmi les Examples n° 1 à 46,
Acide 2-[3-({[2-chlorobenzyl)amino]carbonyl}amino)thién-2-yl]-5-hydroxy-1-méthyl-6-oxo-1,6-dihydropyrimidine-4-carboxylique;
Acide 5-hydroxy-1-méthyl-6-oxo-2-(thién-2-yl)-1,6-dihydropyrimidine-4-carboxylique;
Acide 5-hydroxy-1-méthyl-6-oxo-2-(thiazol-2-yl)-1,6-dihydropyrimidine-4-carboxylique;
Acide 5-hydroxy-1-méthyl-2-(3-nitrothién-2-yl)-6-oxo-1,6-dihydropyrimidine-4-carboxylique;
Sel chlorhydrate d'acide 5-hydroxy-1-méthyl-2-(3-aminothién-2-yl)-6-oxo-1,6-dihydropyrimidine, 4-carboxylique;
Acide 5-hydroxy-1-méthyl-2-(3-bromothién-2-yl)-6-oxo-1,6-dihydropyrimidine-4-carboxylique;
Acide 2-[3-(acétylamino)thién-2-yl-5-hydroxy-1-méthyl-6-oxo-1,6-dihydropyrimidine-4-carboxylique;
Acide 2-[3-(benzoylamino)thién-2-yl]-5-hydroxy-1-méthyl-6-oxo-1,6-dihydropyrimidine-4-carboxylique;
Acide 5-hydroxy-1-méthyl-2-(3-([(2-méthyl-1*H*-indol-3-yl)acétyl]amino}thién-2-yl)-6-oxo-1,6-dihydropyrimidine-4-carboxylique;
Acide 2(3-{[3-(2-chlorophényl)propanoyl]amino}thién-2-yl)-5-hydroxy-1-méthyl-6-oxo-1,6-dibydropyrimidine-4-carboxylique;
Acide 2-{3-[(anilinocarbonyl]amino}thién-2-yl}-5-hydroxy-1-méthyl-6-oxo-1,6-dihydropyrimidine-4-carboxylique;
Acide 2-(3-{[(1,1'-biphényl-2-ylamino)carbonyl]amino}thién-2-yl)-5-hydroxy-1-méthyl-6-oxo-1,6-dihydropyrimidine-4-carboxylique;
Acide 2-(3-{[(benzhydrylamino)carbonyl]amino}thién-2-yl)-5-hydroxy-1-méthyl-6-oxo-1,6-dihydropyrimidine-4-carboxylique;
Acide 5-hydroxy-1-methyl-2-{3-[({[1-(1-naphtyl)éthyl]amino}-carbonyl)amino]thién-2-yl]-6-oxo-1,6-dihydropyrimidine-4-carboxylique;
Acide 5-hydroxy-1-méthyl-6-oxo-2-[3-([(2-phénylcyclopropyl)amino]-carbonyl}amino]thién-2-yl]-1,6-dihydropyrimidine-4-carboxylique;
Acide 5-hydroxy-1-méthyl-6-oxo-2-[3-({[(2-phényléthyl)amino]-carbonyl}amino]thién-2-yl]-1,6-dihydropyrimidine-4-carboxylique;
Acide 5-hydroxy-2-{3-[(isobutoxycarbonyl)amino]thién-2-yl}1-méthyl-6-oxo-1,6-dihydropyrimidine-4-carboxylique;
*N*[1]-benzyl-*N*[2]-[2-(4-carboxy-5-hydroxy-1-méthyl-6-oxo-1,6-dihydropyrimidine-2-yl)thiophén-3-yl]glycinamide;
Acide 5-hydroxy-1-méthyl-6-oxo-2-(3-{[(2*E*)-3-phénylprop-2-ényl]amino}thién-2-yl)-1,6-dihydropyrimidine-4-carboxylique;
Trifluoroacétate de 2-(4-carboxy-5-hydroxy-1-méthyl-6-oxo-1,6-dihydropyrimidin-2-yl)-*N*-(3-phénylpropyl)thiophén-3-aminium;
Acide 2-{3-[2-(benzyloxy)éthyl]thién-2-yl}-5-hydroxy-1-méthyl-6-oxo-1,6-dihydropyrimidine-4-carboxylique;
Acide 2-{4-[({[(2-chlorophényl)sulphonyl]amino}carbonyl)amino]thién-3-yl}-5-hydroxy-1-méthyl-6-oxo-1,6-dihydropyrimidine-4-carboxylique;
Acide 2-{3-[({[(2-chlorophényl)sulfonyl]amino}carbonyl)amino]thién-2-yl}-5-hydroxy-1-méthyl-6-oxo-1,6-dihydropyrimidine-4-carboxylique;
Acide 5-hydroxy-2-(3-hydroxyphényl)-1-méthyl-6-oxo-1,6-dihydropyrimidine-4-carboxylique;
Acide 2-(3-{[(1,1'-biphényl-2-ylamino)carbonyl]amino}phényl)-5-hydroxy-1-méthyl-6-oxo-1,6-dihydropyrimidine-4-carboxylique;
Acide 2-[3-[{[(3-carboxyphényl)amino]carbonyl)amino)phényl]-5-hydroxy-1-méthyl-6-oxo-1,6-dihydropyrimidine-4-carboxylique;
Acide 2-[3-[(benzylsulfonyl)amino]thién-2-yl}-5-hydroxy-1-méthyl-6-oxo-1,6-dihydropyrimidine-4-carboxylique;
Acide 5-hydroxy-1-méthyl-2-{3-[(2-naphtylsulfonyl)amino]thién-2-yl}-6-oxo-1,6-dihydropyrimidine-4-carboxylique;
Acide 2-(3-formylthién-2-yl)-5-hydroxy-1-méthyl-6-oxo-1,6-dihydropyrimidine-4-carboxylique;
Acide2-(3-carboxythién-2-yl)-5-hydroxy-1-méthyl-6-oxo-1,6-dihydropyrimidine-4-carboxylique;
Acide 2-[3-({[2-(2-chlorophényl)éthyl]amino}carbonyl)amino]thién-2-yl]-5-hydroxy-1-méthyl-6-oxo-1,6-dihydropyrimidine-4-carboxylique;
Acide 5-hydroxy-1-méthyl-6-oxo-2-{3-[(*E*)-2-phényléthényl]thién-2-yl]-1,6-dihydropyrimidine-4-carboxylique;
Acide 5-hydroxy-1-méthyl-6-oxo-2-[3-(2-phényléthyl]thién-2-yl]-1,6-dihydropyrimidine-4-carboxylique;
Acide 2-[3-[(1*E*)-4-(2-chlorophényl)but-1-ényl]thién-2-yl]-5-hydroxy-1-méthyl-6-oxo-1,6-dihydropyrimidine-4-carboxilique;
Acide 5-hydroxy-1-méthyl-6-oxo-2-[3-(4-phénylbutyl]thién-2-yl}-1,6-dihydropyrimidine-4-carboxylique;
Acide 5-hydroxy-2-{3-[(4-méthoxybenzyl)oxy]phényl}-1-méthyl-6-oxo-1,6-dihydropyrimidine-4-carboxylique;
Acide 2-{2-[(3,4-dichlorobenzyl)oxy]phényl}-5-hydroxy-1-méthyl-6-oxo-1,6-dihydropyrimidine-4-carboxylique;

Acide 2-(furan-2-yl)-5-hydroxy-1-méthyl-6-oxo-1,6-dihydropyrimidine-4-carboxilique;
Acide 5-hydroxy-1-méthyl-6-oxo-2-{3-[(-*E*)-2-phényléthényl]furan-2-yl]-1,6-dihydropyrimidine-4-carboxylique;
Acide5-hydroxy-1-méthyl-6-oxo-2-(thién-3-yl)-1,6-dihydropyrimidine-4-carboxylique;
5-hydroxy-1-méthyl-6-oxo-2-[(triméthylsilyl)éthynyl]-1,6-dihydropyrimidine-4-carboxylate;
Acide 1-[2-(2-chlorophényl)éthyl]-5hydroxy-6-oxo-2-(thién-2-yl)-1,6-dihydropyrimidine-4-carboxylique;
Acide 1-éthyl-5-hydroxy-6-oxo-2-(thién-2-yl)-1,6-dihydropyrimidine-4-carboxilique;
Acide 1-benzyl-5-hydroxy-6-oxo-(thién-2-yl)-1,6-dihydropyrimidine-4-carboxylique;
Acide 5-hydroxy-6-oxo-2-(thién-2-yl)-1-(2,2,2-trifluoréthyl)-1,6-dihydropyrimidine-4-carboxilique;
Acide 1-(4-carboxybenzyl)-5-hydroxy-6-oxo-(thién-2-yl)-1,6-dihydropyridine-4-carboxylique;
ou un sel pharmaceutiquement acceptable de ceux-ci.

**8.** Composé sélectionné parmi les Examples n° 47 à 49,
Acide 5-hydroxy-1-méthyl-2-[3-[(2-(1naphtyl)éthyl]sulfonylamino)thién-2-yl]-6-oxo-1,6-dihydropyrimidine-4-carboxylique;
Acide 5-hydroxy-1-méthyl-6-oxo-2-[3-[({[(2-phényl-1,3-thiazol-4-yl)méthyl]amino}carbonyl)amino]thién-2-yl]-1,6-dihydropyrimidine-4-carboxylique;
Acide 2-[3-({[(2-chlorobenzyl)oxy]carbonyl}amino)thién-2-yl]-5-hydroxy-1-méthyl-6-oxo-1,6-1,6-dihydropyrimidine-4-carboxylique;
ou un sel pharmaceutiquement acceptable de ceux-ci.

**9.** Composition pharmaceutique comprenant un composé de la formule (I) tel que défini dans la revendication 1 ou un sel pharmaceutiquement acceptable de celui-ci, en association avec un véhicule pharmaceutiquement acceptable.

**10.** utilisation d'un composé de la formule (I) tel que défini dans la revendication 1 ou d'un pharmaceutiquement acceptable de celui-ci dans la fabrication d'un médicament pour le traitement ou la prévention d'une infection par le virus de l'hépatite C.

**11.** Procédé de préparation d'un composé de la formule (I) tel que défini dans la revendication 1, lequel comprend:

(A) mettre un composé de la formule (XIV) à réagir avec un composé de la formule (XV).

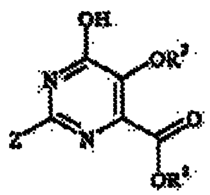

(XIV)

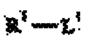

(XV)

où Z, $R^1$, $R^2$ et $R^3$ sont tels que définis dans la revendication 1 et $L^1$ représente un groupe labile approprié ou bien (B) mettre un composé de la formule (XVIII) à réagir avec un composé de la formule (XIX);

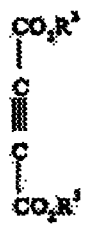

(XVIII)

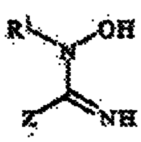

(XIX)

où Z, $R^1$ et $R^3$ sont tels que définis dans la revendication 1; ce qui est suivi par la cyclisation de l'intermédiaire

ainsi obtenu; ou bien

(C) mettre un composé de la formule (XVIII) tel que défini ci-dessus, à réagir avec un composé de la formule (XX):

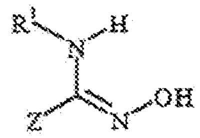

(XX)

où Z et $R^1$ sont tels que définis dans la revendication 1; ce qui est suivi par la cyclisation de l'intermédiaire ainsi obtenu; ou bien

(D) mettre un composé de la formule $Z\text{-}B(OH)_2$ à réagir avec un composé de la formule (XXII):

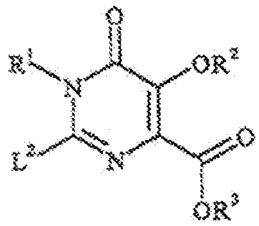

(XXII)

où Z, $R^1$, $R^2$ et $R^3$ sont tels que définis dans la revendication 1 et $L^2$ représente un groupe labile approprié; en présence d'un catalyseur à base de métal de transition; et

(E) convertir ensuite, si requis, un composé de la formule (I) obtenu initialement en un autre composé de la formule (I) par des méthods standard.

**12.** Composé tel que revendiqué dans l'une quelconque des revendications 1 à 8 à utiliser en thérapie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO U013708 A
- WO 0206246 A
- WO 9637619 A
- US 5925764 A


### Non-patent literature cited in the description


- *J. Heterocyclic Chemistry,* November 1979, vol. 16 (7), 1423-4
- Protective Groups in Organic Chemistry. Plenum Press, 1973
- **T.W. GREENE ; P.G.M. WUTS.** Protective Groups in Organic Synthesis. John Wiley & Sons, 1999
- **W.C. STILL et al.** *J. Org. Chem.,* 1978, vol. 43, 2923
- **P.R. HUDDLESTON et al.** *Synthetic Communications,* 1995, vol. 25, 3729
- **S. HIBINO et al.** *Journal of Organic Chemistry,* 1984, vol. 49, 5006